# EUROPEAN PATENT APPLICATION

(11) **EP 4 737 557 A1**
(43) Date of publication of application: **06.05.2026**
(21) Application number: 24830697.9
(22) Date of filing: 24.06.2024
(51) Int. Cl.: C12N 1/21, C12N 15/53

(54) **MODIFIED ESCHERICHIA COLI AND USE THEREOF IN PRODUCTION OF L-AMINO ACID BY MEANS OF FERMENTATION**

(30) Priority: 27.06.2023 CN 202310765899; 29.06.2023 CN 202310784984; 29.06.2023 CN 202310784863; 04.07.2023 CN 202310809284
(71) Applicant: Ningxia Eppen Biotech Co. Ltd, Ningxia 750100 (CN)
(72) Inventor: MENG, Gang, Yinchuan, Ningxia 750100 (CN); WEI, Aiying, Yinchuan, Ningxia 750100 (CN); ZHAO, Chunguang, Yinchuan, Ningxia 750100 (CN); SU, Houbo, Yinchuan, Ningxia 750100 (CN); ZHANG, Ying, Yinchuan, Ningxia 750100 (CN); BI, Guodong, Yinchuan, Ningxia 750100 (CN); ZHANG, Xiaoqin, Yinchuan, Ningxia 750100 (CN); WANG, Pan, Yinchuan, Ningxia 750100 (CN); FU, Lixia, Yinchuan, Ningxia 750100 (CN)
(74) Representative: Winter, Brandl - Partnerschaft mbB
(86) International application number: PCT/CN2024/100995
(87) International publication number: WO 2025/002055

(57) **Abstract**

A modified *Escherichia coli* and the use thereof in the production of L-amino acid by means of fermentation. Specifically, disclosed is the use of pyruvate formate lyase, alcohol dehydrogenase, branched-chain amino acid transaminase derived from *Escherichia coli,* branched-chain amino acid transaminase derived from *Bacillus subtilis,* thiamine phosphate synthase and malate dehydrogenase in the construction of a genetically engineered bacterium for producing L-amino acid. Experiments prove that the recombinant bacterium, which is conducive to the accumulation of L-amino acid, is obtained by means of knocking out the encoding genes of the pyruvate formate lyase, the alcohol dehydrogenase, the branched-chain amino acid transaminase derived from *Escherichia coli,* the thiamine phosphate synthase and the malate dehydrogenase of *Escherichia coli* capable of producing L-amino acid, and introducing the encoding gene of the branched-chain amino acid transaminase derived from *Bacillus subtilis.* The recombinant bacteria can greatly increase the yield of L-amino acid and reduce the costs, and is of great significance to accelerate the industrial progress of L-amino acid.

## Description

### Cross-reference to Related Application

This application claims priority to the following four patent applications: Chinese patent application 202310765899.5 filed to the China Patent Office on June 27, 2023 and titled "Recombinant *Escherichia Coli* for fermentation and production of L-valine"; Chinese patent application 202310784984.6 filed to the China Patent Office on June 29, 2023 and titled "Modified *Escherichia coli* and use thereof in production of L-valine by means of fermentation"; Chinese patent application 202310784863.1 filed to the Chinese Patent Office on June 29, 2023 and titled "Recombinant bacterium and use thereof in production of L-valine"; and Chinese patent application 202310809284.8 filed to the Chinese Patent Office on July 04, 2023 and titled "Method for Increasing Yield of L-valine and Recombinant Bacterium Used in Method", all of which are incorporated herein by reference in their entireties.

### Technical Field

The present invention belongs to the field of biotechnology, and relates to modified *Escherichia coli* and use thereof in the production of L-amino acid by means of fermentation.

### Background Art

L-valine has important applications in food, medicine, health products, feed and other fields. In the field of food, L-valine can supplement nutrients, promote body growth, and provide energy. In the field of medicine, L-valine may be used in infusions and injections to promote surgical wound healing. The export volume of feed-grade L-valine in China reaches more than 30,000 tons, with an average annual growth rate of 20%.

At present, many domestic enterprises have carried out the production of L-valine through a fermentation method, and many laboratories are also conducting research projects on the fermentation process of L-valine. The researchers have found that the process of intracellular L-valine secretion through a cell membrane to the outside of a cell requires the participation of specific transporters, but in this process, the activity of specific transporters cannot meet the timely secretion of intracellular valine through the cell membrane to the outside of the cell, resulting in accumulation of intracellular L-valine; at the same time, acetohydroxyacid synthase is contained in bacterial cells, has a catalytic effect on the biosynthesis of L-valine and can promote acid production, but when L-valine synthesized in the bacterial cells cannot be excreted and accumulated in time, acetohydroxyacid synthase is strongly inhibited by L-valine to reduce its catalytic effect, and the synthesis of L-valine cannot be further catalyzed, so the continued synthesis of L-valine is limited, which ultimately leads to low yield. In addition, studies have found that *Escherichia coli* is very sensitive to L-valine, and when L-valine in L-valine producing bacteria cannot be excreted in time and accumulates, the growth of *Escherichia coli* cannot be seriously restricted, which in turn leads to the inhibition of L-valine synthesis in bacterial cells, resulting in low yield.

### Summary of the Invention

The main purpose of the present invention is how to increase the yield of L-amino acid. The purpose of the present invention is not limited to the described subject. Other purposes which are not mentioned herein can be clearly understood by those skilled in the art through the following description.

The present invention protects use of a protein combination first, which may be at least one of A1)-A5):
A1) the construction of genetically engineered bacteria that produces L-amino acid;
A2) the production of L-amino acid;
A3) the regulation of the yield of L-amino acid;
A4) the preparation of a product for producing L-amino acid; and
A5) the preparation of food, feed or drug containing L-amino acid.

In the above-mentioned use, the protein combination may include at least one of pyruvate formate lyase, alcohol dehydrogenase, branched-chain amino acid transaminase derived from *Escherichia coli,* branched-chain amino acid transaminase derived from *Bacillus subtilis,* thiamine phosphate synthase and malate dehydrogenase.

In the above-mentioned use, the protein combination may specifically be composed of at least one of pyruvate formate lyase, alcohol dehydrogenase, branched-chain amino acid transaminase derived from *Escherichia coli,* branched-chain amino acid transaminase derived from *Bacillus subtilis,* thiamine phosphate synthase and malate dehydrogenase.

Any of the above-mentioned pyruvate formate lyase is B1) or B2) or B3):
B1) a protein having an amino acid sequence as shown in SEQ ID No. 13;
B2) a protein which is obtained by substitution and/or deletion and/or addition of an amino acid residue in the amino acid sequence as shown in SEQ ID No. 13, has the identity of 90% or more with the protein shown in B1) and has the same function as the protein shown in B1); and
B3) a fusion protein which is obtained by linking a tag to an N-terminal or/and a C-terminal of B1) or B2) and has the same function as that of B1) or B2).

Any of the above-mentioned alcohol dehydrogenase may be C1) or C2) or C3):
C1) a protein having an amino acid sequence as shown in SEQ ID No. 14;
C2) a protein which is obtained by substitution and/or deletion and/or addition of an amino acid residue in the amino acid sequence as shown in SEQ ID No. 14, has the identity of 90% or more with the protein shown in C1) and has the same function as the protein shown in C1); and
C3) a fusion protein which is obtained by linking a tag to an N-terminal or/and a C-terminal of C1) or C2) and has the same function as that of C1) or C2).

Any of the above-mentioned branched-chain amino acid transaminase derived from *Escherichia coli* may be D1) or D2) or D3):
D1) a protein having an amino acid sequence as shown in SEQ ID No. 15;
D2) a protein which is obtained by substitution and/or deletion and/or addition of an amino acid residue in the amino acid sequence as shown in SEQ ID No. 15, has the identity of 90% or more with the protein shown in D1) and has the same function as the protein shown in D1); and
D3) a fusion protein which is obtained by linking a tag to an N-terminal or/and a C-terminal of D1) or D2) and has the same function as that of D1) or D2).

Any of the above-mentioned branched-chain amino acid transaminase derived from *Bacillus subtilis* may be E1) or E2) or E3):
E1) a protein having an amino acid sequence as shown in SEQ ID No. 16;
E2) a protein which is obtained by substitution and/or deletion and/or addition of an amino acid residue in the amino acid sequence as shown in SEQ ID No. 16, has the identity of 90% or more with the protein shown in E1) and has the same function as the protein shown in E1); and
E3) a fusion protein which is obtained by linking a tag to an N-terminal or/and a C-terminal of E1) or E2) and has the same function as that of E1) or E2).

Any of the above-mentioned thiamine phosphate synthase may be F1) or F2) or F3):
F1) a protein having an amino acid sequence as shown in SEQ ID No. 17;
F2) a protein which is obtained by substitution and/or deletion and/or addition of an amino acid residue in the amino acid sequence as shown in SEQ ID No. 17, has the identity of 90% or more with the protein shown in F1) and has the same function as the protein shown in F1); and
F3) a fusion protein which is obtained by linking a tag to an N-terminal or/and a C-terminal of F1) or F2) and has the same function as that of F1) or F2).

Any of the above-mentioned malate dehydrogenase may be G1) or G2) or G3):
G1) a protein having an amino acid sequence as shown in SEQ ID No. 18;
G2) a protein which is obtained by substitution and/or deletion and/or addition of an amino acid residue in the amino acid sequence as shown in SEQ ID No. 18, has the identity of 90% or more with the protein shown in G1) and has the same function as the protein shown in G1); and
G3) a fusion protein which is obtained by linking a tag to an N-terminal or/and a C-terminal of G1) or G2) and has the same function as that of G1) or G2).

The present invention further protects use a nucleic acid molecule that encodes any of the above protein combinations, which may be at least one of A1)-A5):
A1) the construction of genetically engineered bacteria that produces L-amino acid;
A2) the production of L-amino acid;
A3) the regulation of the yield of L-amino acid;
A4) the preparation of a product for producing L-amino acid; and
A5) the preparation of food, feed or drug containing L-amino acid.

The present invention further protects use of a substance that inhibits or down-regulates the expression amount and/or activity of any of the above-mentioned pyruvate formate lyase; and/or a substance that inhibits or down-regulates the expression amount and/or activity of any of the above-mentioned alcohol dehydrogenase; and/or a substance that inhibits or down-regulates the expression amount and/or activity of the above-mentioned branched-chain amino acid transaminase derived from *Escherichia coli;* and/or a substance that inhibits or down-regulates the expression amount and/or activity of any of the above-mentioned thiamine phosphate synthase; and/or a substance that inhibits or down-regulates the expression amount and/or activity of any of the above-mentioned malate dehydrogenase; and/or a substance that increases or up-regulates the expression amount and/or activity of any of the above-mentioned branched-chain amino acid transaminase derived from *Bacillus subtilis,* which may be at least one of A1)-A5):
A1) the construction of genetically engineered bacteria that produces L-amino acid;
A2) the production of L-amino acid;
A3) the regulation of the yield of L-amino acid;
A4) the preparation of a product for producing L-amino acid; and
A5) the preparation of food, feed or drug containing L-amino acid.

In the above-mentioned use, the substance that inhibits or down-regulates the expression amount and/or activity of any of the above-mentioned pyruvate formate lyase may be sgRNAa as shown in SEQ ID No. 1.

In the above-mentioned use, the substance that inhibits or down-regulates the expression amount and/or activity of any of the above-mentioned alcohol dehydrogenase may be sgRNAb as shown in SEQ ID No. 4.

In the above-mentioned use, the substance that inhibits or down-regulates the expression amount and/or activity of any of the above-mentioned branched-chain amino acid transaminase derived from *Escherichia coli* may be sgRNAc as shown in SEQ ID No. 7.

In the above-mentioned use, the substance that inhibits or down-regulates the expression amount and/or activity of any of the above-mentioned thiamine phosphate synthase may be sgRNAd as shown in SEQ ID No. 9.

In the above-mentioned use, the substance that inhibits or down-regulates the expression amount and/or activity of any of the above-mentioned malate dehydrogenase may be sgRNAe as shown in SEQ ID No. 11.

In the above-mentioned use, the substance that increases or up-regulates the expression amount and/or activity of any of the above-mentioned branched-chain amino acid transaminase derived from *Bacillus subtilis* may be a nucleic acid molecule. Further, the nucleic acid molecule may initiate an encoding gene of the branched-chain amino acid transaminase derived from *Bacillus subtilis* for ptrc. Furthermore, a nucleotide sequence of the nucleic acid may be as shown in SEQ ID: No. 8.

The present invention further protects use of an expression cassette, a recombinant vector, a recombinant bacterium or a recombinant host cell, which contains any of the above-mentioned nucleic acid molecules; a substance that inhibits or down-regulates the expression amount and/or activity of any of the above-mentioned pyruvate formate lyase, a substance that inhibits or down-regulates the expression amount and/or activity of any of the above-mentioned alcohol dehydrogenase, a substance that inhibits or down-regulates the expression amount and/or activity of any of the above-mentioned branched-chain amino acid transaminase derived from *Escherichia coli,* a substance that inhibits or down-regulates the expression amount and/or activity of any of the above-mentioned thiamine phosphate synthase, a substance that inhibits or down-regulates the expression amount and/or activity of any of the above-mentioned malate dehydrogenase, and/or a substance that increases or up-regulates the expression amount and/or activity of any of the above-mentioned branched-chain amino acid transaminase derived from *Bacillus subtilis,* which may be at least one of A1)-A5):
A1) the construction of genetically engineered bacteria that produces L-amino acid;
A2) the production of L-amino acid;
A3) the regulation of the yield of L-amino acid;
A4) the preparation of a product for producing L-amino acid; and
A5) the preparation of food, feed or drug containing L-amino acid.

The present invention further protects a recombinant bacterium, which is obtained by means of: weak expression or no expression of at least one of any of the above-mentioned pyruvate formate lyase, any of the above-mentioned alcohol dehydrogenase, any of the above-mentioned branched-chain amino acid transaminase derived from *Escherichia coli,* any of the above-mentioned thiamine phosphate synthase and any of the above-mentioned malate dehydrogenase in originating bacteria; and/or the expression or over-expression of any of the above-mentioned branched-chain amino acid transaminase derived from *Bacillus subtilis.*

In the recombinant bacterium, the weak expression or no expression is implemented by reducing the expression amount and/or activity of any of the above-mentioned pyruvate formate lyase, any of the above-mentioned alcohol dehydrogenase, any of the above-mentioned branched-chain amino acid transaminase derived from *Escherichia coli,* any of the above-mentioned thiamine phosphate synthase and any of the above-mentioned malate dehydrogenase in the originating bacterium.

In the recombinant bacterium, the expression or over-expression is implemented by introducing an encoding gene of any of the above-mentioned branched-chain amino acid transaminase derived from *Bacillus subtilis* into the originating bacterium.

In the recombinant bacterium, the reduction in the expression amount and/or activity of any of the above-mentioned pyruvate formate lyase, any of the above-mentioned alcohol dehydrogenase, any of the above-mentioned branched-chain amino acid transaminase derived from *Escherichia coli,* any of the above-mentioned thiamine phosphate synthase and any of the above-mentioned malate dehydrogenase in the originating bacteria can be achieved by a gene editing, gene knockout, gene mutation or gene attenuation technology, causing a decrease in the expression amount, activity reduction or inactivation of any of the above-mentioned pyruvate formate lyase, any of the above-mentioned alcohol dehydrogenase, any of the above-mentioned branched-chain amino acid transaminase derived from *Escherichia coli,* any of the above-mentioned thiamine phosphate synthase and any of the above-mentioned malate dehydrogenase in the originating bacteria.

In the recombinant bacterium, the gene editing is carried out using a CRISPR/Cas9 system. The CRISPR/Cas9 system may include a vector expressing sgRNA targeting an encoding gene of any of the above-mentioned pyruvate formate lyase; a vector expressing sgRNA targeting an encoding gene of any of the above-mentioned alcohol dehydrogenase; a vector expressing sgRNA targeting an encoding gene of any of the above-mentioned branched-chain amino acid transaminase derived from *Escherichia coli;* a vector expressing sgRNA targeting an encoding gene of any of the above-mentioned thiamine phosphate synthase; and/or a vector expressing sgRNA targeting an encoding gene of any of the above-mentioned malate dehydrogenase.

In the recombinant bacterium, sgRNA targeting the encoding gene of the pyruvate formate lyase may be as shown in SEQ ID No. 1. In the recombinant bacterium, sgRNA targeting the encoding gene of the alcohol dehydrogenase may be as shown in SEQ ID No. 4. In the recombinant bacterium, sgRNA targeting the encoding gene of the branched-chain amino acid transaminase derived from *Escherichia coli* may be as shown in SEQ ID No. 7. In the recombinant bacterium, sgRNA targeting the encoding gene of the thiamine phosphate synthase may be as shown in SEQ ID No. 9. In the recombinant bacterium, sgRNA targeting the encoding gene of the malate dehydrogenase may be as shown in SEQ ID No. 11.

The recombinant bacterium protected by the present invention may specifically be an engineered bacterium A, which may be a microorganism that inhibits or down-regulates the expression amount and/or activity of any of the above-mentioned pyruvate formate lyase in vivo; and the microorganism may produce valine.

In the engineered bacterium A, the inhibition or down-regulation of the expression amount and/or activity of the pyruvate formate lyase is implemented by knocking out or knocking down the encoding gene (i.e., a pflB gene) of the pyruvate formate lyase in the microorganism.

The inhibition or down-regulation of the expression amount and/or activity of the pyruvate formate lyase is implemented by introducing a pGRB-pflB sgRNA plasmid and a ΔpflB-Up-Down fragment mentioned in the examples into the microorganism.

The above-mentioned engineered bacterium A also inhibits or down-regulates the expression and/or activity of alcohol dehydrogenase in vivo.

In the engineered bacterium A, the inhibition or down-regulation of the expression amount and/or activity of the alcohol dehydrogenase is implemented by knocking out or knocking down the encoding gene (i.e., an adhE gene) of the alcohol dehydrogenase in the microorganism.

The inhibition or down-regulation of the expression amount and/or activity of the alcohol dehydrogenase is implemented by introducing a pGRB-adhE sgRNA plasmid and a ΔadhE-Up-Down fragment mentioned in the examples into the microorganism.

The above-mentioned engineered bacterium A also inhibits or down-regulates the expression amount and/or activity of the branched-chain amino acid transaminase derived from *Escherichia coli* in vivo, and contains or expresses the branched-chain amino acid transaminase derived from *Bacillus subtilis.*

In the engineered bacterium A, the inhibition or down-regulation of the expression amount and/or activity of the branched-chain amino acid transaminase derived from *Escherichia coli* is implemented by knocking out or knocking down the encoding gene (i.e., an ilvE(E) gene) of the branched-chain amino acid transaminase in the microorganism.

In the engineered bacterium A, the inclusion or expression of the branched-chain amino acid transaminase derived from *Bacillus subtilis* is implemented by knocking in or introducing the encoding gene (i.e., an ilvE(B) gene) of the branched-chain amino acid transaminase derived from *Bacillus subtilis* in the microorganism.

In the engineered bacterium A, the knock-in or introduction of the encoding gene of the branched-chain amino acid transaminase derived from *Bacillus subtilis* in the microorganism is implemented by introducing an expression cassette into the microorganism; and the expression cassette includes a promoter and the encoding gene of the branched-chain amino acid transaminase derived from *Bacillus subtilis.* The promoter may be an inducible promoter. The inducible promoter may specifically be a ptrc promoter.

The inhibition or down-regulation of the expression amount and/or activity of the branched-chain amino acid transaminase derived from *Escherichia coli* and the inclusion or expression of the branched-chain amino acid transaminase derived from *Bacillus subtilis* may be implemented by introducing a pGRB-ilvE sgRNA plasmid and a ptrc-ilvE(B)-Up-Down fragment mentioned in the examples into the microorganism.

The above-mentioned engineered bacterium A also inhibits or down-regulates the expression amount and/or activity of the thiamine phosphate synthase.

In the engineered bacterium A, the inhibition or down-regulation of the expression amount and/or activity of the thiamine phosphate synthase is implemented by knocking out or knocking down the encoding gene (i.e., a thiE gene) of the thiamine phosphate synthase in the microorganism.

The inhibition or down-regulation of the expression amount and/or activity of the thiamine phosphate synthase is implemented by introducing a pGRB-thiE sgRNA plasmid and a ΔthiE-Up-Down fragment mentioned in the examples into the microorganism.

The above-mentioned engineered bacterium A also inhibits or down-regulates the expression amount and/or activity of malate dehydrogenase in vivo.

In the engineered bacterium A, the inhibition or down-regulation of the expression amount and/or activity of the malate dehydrogenase is implemented by knocking out or knocking down the encoding gene (i.e., an mdh gene) of the malate dehydrogenase in the microorganism.

The inhibition or down-regulation of the expression amount and/or activity of the malate dehydrogenase is implemented by introducing a pGRB-mdh sgRNA plasmid and a Δmdh-Up-Down fragment mentioned in the examples into the microorganism.

The recombinant bacterium protected by the present invention may specifically be an engineered bacterium B, which may be a microorganism that inhibits or down-regulates the expression amount and/or activity of the alcohol dehydrogenase; and the microorganism may produce valine. In the engineered bacterium B, the inhibition or down-regulation of the expression amount and/or activity of the alcohol dehydrogenase is implemented by knocking out or knocking down the encoding gene (i.e., an adhE gene) of the alcohol dehydrogenase in the microorganism.

The inhibition or down-regulation of the expression amount and/or activity of the alcohol dehydrogenase is implemented by introducing a pGRB-adhE sgRNA plasmid and a ΔadhE-Up-Down fragment mentioned in the examples into the microorganism.

The above-mentioned engineered bacterium B also inhibits or down-regulates the expression amount and/or activity of the microorganism-derived branched-chain amino acid transaminase in vivo, and contains or expresses the branched-chain amino acid transaminase derived from *Bacillus subtilis.*

In the engineered bacterium B, the inhibition or down-regulation of the expression amount and/or activity of the microorganism-derived branched-chain amino acid transaminase is implemented by knocking out or knocking down the encoding gene (i.e., an ilvE(E) gene) of the branched-chain amino acid transaminase in the microorganism.

In the engineered bacterium B, the inclusion or expression of the branched-chain amino acid transaminase derived from *Bacillus subtilis* is implemented by knocking in or introducing the encoding gene (i.e., an ilvE(B) gene) of the branched-chain amino acid transaminase derived from *Bacillus subtilis* in the microorganism.

In the engineered bacterium B, the knock-in or introduction of the encoding gene of the branched-chain amino acid transaminase derived from *Bacillus subtilis* in the microorganism is implemented by introducing an expression cassette into the microorganism; and the expression cassette includes a promoter and the encoding gene of the branched-chain amino acid transaminase derived from *Bacillus subtilis.* The promoter may be an inducible promoter. The inducible promoter may specifically be a ptrc promoter.

The inhibition or down-regulation of the expression amount and/or activity of the microorganism-derived branched-chain amino acid transaminase and the inclusion or expression of the branched-chain amino acid transaminase derived from *Bacillus subtilis* may be implemented by introducing a pGRB-ilvE sgRNA plasmid and a ptrc-ilvE(B)-Up-Down fragment mentioned in the examples into the microorganism.

The above-mentioned engineered bacterium B also inhibits or down-regulates the expression amount and/or activity of the thiamine phosphate synthase.

In the engineered bacterium B, the inhibition or down-regulation of the expression amount and/or activity of the thiamine phosphate synthase is implemented by knocking out or knocking down the encoding gene (i.e., a thiE gene) of the thiamine phosphate synthase in the microorganism.

The inhibition or down-regulation of the expression amount and/or activity of the thiamine phosphate synthase is implemented by introducing a pGRB-thiE sgRNA plasmid and a ΔthiE-Up-Down fragment mentioned in the examples into the microorganism.

The above-mentioned engineered bacterium B also inhibits or down-regulates the expression amount and/or activity of malate dehydrogenase in vivo.

In the engineered bacterium B, the inhibition or down-regulation of the expression amount and/or activity of the malate dehydrogenase is implemented by knocking out or knocking down the encoding gene (i.e., an mdh gene) of the malate dehydrogenase in the microorganism.

The inhibition or down-regulation of the expression amount and/or activity of the malate dehydrogenase is implemented by introducing a pGRB-mdh sgRNA plasmid and a Δmdh-Up-Down fragment mentioned in the examples into the microorganism.

The recombinant bacterium protected by the present invention may specifically be an engineered bacterium C, which may be a microorganism that inhibits or down-regulates the expression amount and/or activity of the microorganism-derived branched-chain amino acid transaminase in vivo, and contains or expresses the branched-chain amino acid transaminase derived from *Bacillus subtilis;* and the microorganism may produce valine.

In the engineered bacterium C, the inhibition or down-regulation of the expression amount and/or activity of the microorganism-derived branched-chain amino acid transaminase is implemented by knocking out or knocking down the encoding gene (i.e., an ilvE(E) gene) of the branched-chain amino acid transaminase in the microorganism.

In the engineered bacterium C, the inclusion or expression of the branched-chain amino acid transaminase derived from *Bacillus subtilis* is implemented by knocking in or introducing the encoding gene (i.e., an ilvE(B) gene) of the branched-chain amino acid transaminase derived from *Bacillus subtilis* in the microorganism.

In the engineered bacterium C, the knock-in or introduction of the encoding gene of the branched-chain amino acid transaminase derived from *Bacillus subtilis* in the microorganism is implemented by introducing an expression cassette into the microorganism; and the expression cassette includes a promoter and the encoding gene of the branched-chain amino acid transaminase derived from *Bacillus subtilis.* The promoter may be an inducible promoter. The inducible promoter may specifically be a ptrc promoter.

The inhibition or down-regulation of the expression amount and/or activity of the microorganism-derived branched-chain amino acid transaminase and the inclusion or expression of the branched-chain amino acid transaminase derived from *Bacillus subtilis* may be implemented by introducing a pGRB-ilvE sgRNA plasmid and a ptrc-ilvE(B)-Up-Down fragment mentioned in the examples into the microorganism.

The above-mentioned engineered bacterium C also inhibits or down-regulates the expression amount and/or activity of the thiamine phosphate synthase in vivo.

In the engineered bacterium C, the inhibition or down-regulation of the expression amount and/or activity of the thiamine phosphate synthase is implemented by knocking out or knocking down the encoding gene (i.e., a thiE gene) of the thiamine phosphate synthase in the microorganism.

The inhibition or down-regulation of the expression amount and/or activity of the thiamine phosphate synthase is implemented by introducing a pGRB-thiE sgRNA plasmid and a ΔthiE-Up-Down fragment mentioned in the examples into the microorganism.

The above-mentioned engineered bacterium C also inhibits or down-regulates the expression amount and/or activity of malate dehydrogenase in vivo.

In the engineered bacterium C, the inhibition or down-regulation of the expression amount and/or activity of the malate dehydrogenase is implemented by knocking out or knocking down the encoding gene (i.e., an mdh gene) of the malate dehydrogenase in the microorganism.

The inhibition or down-regulation of the expression amount and/or activity of the malate dehydrogenase is implemented by introducing a pGRB-mdh sgRNA plasmid and a Δmdh-Up-Down fragment mentioned in the examples into the microorganism.

The recombinant bacterium protected by the present invention may specifically be an engineered bacterium D, which may be a microorganism that inhibits or down-regulates the expression amount and/or activity of the thiamine phosphate synthase; and the microorganism may produce valine.

In the engineered bacterium D, the inhibition or down-regulation of the expression amount and/or activity of the thiamine phosphate synthase is implemented by knocking out or knocking down the encoding gene (i.e., a thiE gene) of the thiamine phosphate synthase in the microorganism.

The inhibition or down-regulation of the expression amount and/or activity of the thiamine phosphate synthase is implemented by introducing a pGRB-thiE sgRNA plasmid and a ΔthiE-Up-Down fragment mentioned in the examples into the microorganism.

The above-mentioned engineered bacterium D also inhibits or down-regulates the expression amount and/or activity of malate dehydrogenase in vivo.

In the engineered bacterium D, the inhibition or down-regulation of the expression amount and/or activity of the malate dehydrogenase is implemented by knocking out or knocking down the encoding gene (i.e., an mdh gene) of the malate dehydrogenase in the microorganism.

The inhibition or down-regulation of the expression amount and/or activity of the malate dehydrogenase is implemented by introducing a pGRB-mdh sgRNA plasmid and a Δmdh-Up-Down fragment mentioned in the examples into the microorganism.

The recombinant bacterium protected by the present invention may specifically be an engineered bacterium E, which may be a microorganism that inhibits or down-regulates the expression amount and/or activity of the malate dehydrogenase; and the microorganism may produce valine.

In the engineered bacterium E, the inhibition or down-regulation of the expression amount and/or activity of the malate dehydrogenase is implemented by knocking out or knocking down the encoding gene (i.e., an mdh gene) of the malate dehydrogenase in the microorganism.

The inhibition or down-regulation of the expression amount and/or activity of the malate dehydrogenase is implemented by introducing a pGRB-mdh sgRNA plasmid and a Δmdh-Up-Down fragment mentioned in the examples into the microorganism.

The present invention further protects a method for increasing the yield of L-amino acid. The method includes the following steps: reducing the expression amount and/or activity of at least one of any of the above-mentioned pyruvate formate lyase, any of the above-mentioned alcohol dehydrogenase, any of the above-mentioned branched-chain amino acid transaminase derived from *Escherichia coli,* any of the above-mentioned thiamine phosphate synthase and any of the above-mentioned malate dehydrogenase in originating bacteria; and/or increasing the expression amount and/or activity of any of the above-mentioned branched-chain amino acid transaminase derived from *Bacillus subtilis* to obtain a recombinant bacterium, wherein the yield of L-amino acid in the recombinant bacterium is higher than that of an originating bacteria.

In the method, the reduction in the expression amount and/or activity of any of the above-mentioned pyruvate formate lyase, any of the above-mentioned alcohol dehydrogenase, any of the above-mentioned branched-chain amino acid transaminase derived from *Escherichia coli,* any of the above-mentioned thiamine phosphate synthase and any of the above-mentioned malate dehydrogenase in the originating bacteria can be implemented by a gene editing, gene knockout, gene mutation or gene attenuation technology, causing a decrease in the expression amount, activity reduction or inactivation of any of the above-mentioned pyruvate formate lyase, any of the above-mentioned alcohol dehydrogenase, any of the above-mentioned branched-chain amino acid transaminase derived from *Escherichia coli,* any of the above-mentioned thiamine phosphate synthase and any of the above-mentioned malate dehydrogenase in the originating bacteria.

In the above-mentioned method, the increase in the expression amount and/or activity of any of the above-mentioned branched-chain amino acid transaminase derived from *Bacillus subtilis* may be implemented by introducing an encoding gene of the branched-chain amino acid transaminase derived from *Bacillus subtilis* into the originating bacteria.

In the method, the gene editing is carried out using a CRISPR/Cas9 system. The CRISPR/Cas9 system may include a vector expressing sgRNA targeting an encoding gene of any of the above-mentioned pyruvate formate lyase; a vector expressing sgRNA targeting an encoding gene of any of the above-mentioned alcohol dehydrogenase; a vector expressing sgRNA targeting an encoding gene of any of the above-mentioned branched-chain amino acid transaminase derived from *Escherichia coli;* a vector expressing sgRNA targeting an encoding gene of any of the above-mentioned thiamine phosphate synthase; and/or or a vector expressing sgRNA targeting an encoding gene of any of the above-mentioned malate dehydrogenase.

In the above-mentioned method, sgRNA targeting the encoding gene of the pyruvate formate lyase may be as shown in SEQ ID No. 1. sgRNA targeting the encoding gene of any of the above-mentioned the alcohol dehydrogenase may be as shown in SEQ ID No. 4. sgRNA targeting the encoding gene of any of the above-mentioned the branched-chain amino acid transaminase derived from *Escherichia coli* may be as shown in SEQ ID No. 7. sgRNA targeting the encoding gene of any of the above-mentioned thiamine phosphate synthase may be as shown in SEQ ID No. 9. sgRNA targeting the encoding gene of any of the above-mentioned malate dehydrogenase may be as shown in SEQ ID No. 11.

A preparation method for the recombinant bacterium protected by the present invention may specifically be a method for preparing an engineered bacterium A. The method may include a step (a1): reducing the expression amount and/or activity of any of the above-mentioned pyruvate formate lyase in a microorganism, wherein the microorganism may produce valine.

In the preparation method, the reduction of the expression amount and/or activity of the pyruvate formate lyase in the microorganism is implemented by knocking out or knocking down the encoding gene (i.e., a pflB gene) of the pyruvate formate lyase in the microorganism.

The reduction of the expression amount and/or activity of the pyruvate formate lyase in the microorganism is implemented by introducing a pGRB-pflB sgRNA plasmid and a ΔpflB-Up-Down fragment mentioned in the examples into the microorganism.

The preparation method further includes a step (a2): after completing the step (a1), reducing the expression and/or activity of any of the above-mentioned alcohol dehydrogenase in the microorganism.

In the preparation method, the reduction of the expression amount and/or activity of the alcohol dehydrogenase in the microorganism is implemented by knocking out or knocking down the encoding gene (i.e., an adhE gene) of the alcohol dehydrogenase in the microorganism.

The reduction of the expression amount and/or activity of the alcohol dehydrogenase in the microorganism is implemented by introducing a pGRB-adhE sgRNA plasmid and a ΔadhE-Up-Down fragment mentioned in the examples into the microorganism.

The preparation method further includes a step (a3): after completing the step (a2), reducing the expression amount and/or activity of the branched-chain amino acid transaminase derived from *Escherichia coli,* and expressing the branched-chain amino acid transaminase derived from *Bacillus subtilis* in the microorganism.

In the preparation method, the reduction of the expression amount and/or activity of the branched-chain amino acid transaminase in the microorganism is implemented by knocking out or knocking down the encoding gene (i.e., an ilvE(E) gene) of the branched-chain amino acid transaminase in the microorganism. The expression of the branched-chain amino acid transaminase derived from *Bacillus subtilis* in the microorganism is implemented by knocking in or introducing the encoding gene (i.e., an ilvE(B) gene) of the branched-chain amino acid transaminase derived from *Bacillus subtilis* in the microorganism. The knock-in or introduction of the encoding gene of the branched-chain amino acid transaminase derived from *Bacillus subtilis* in the microorganism is implemented by introducing an expression cassette into the microorganism; and the expression cassette includes a promoter and the encoding gene of the branched-chain amino acid transaminase derived from *Bacillus subtilis.* The promoter may be an inducible promoter. The inducible promoter may specifically be a ptrc promoter.

The reduction of the expression amount and/or activity of the branched-chain amino acid transaminase derived from *Escherichia coli* and the expression of the branched-chain amino acid transaminase derived from *Bacillus subtilis* in the microorganism may be implemented by introducing a pGRB-ilvE sgRNA plasmid and a ptrc-ilvE(B)-Up-Down fragment mentioned in the examples into the microorganism.

The preparation method further includes a step (a4): after completing the step (a3), reducing the expression amount and/or activity of any of the above-mentioned thiamine phosphate synthase in the microorganism.

In the preparation method, the reduction of the expression amount and/or activity of the thiamine phosphate synthase in the microorganism is implemented by knocking out or knocking down the encoding gene (i.e., a thiE gene) of the thiamine phosphate synthase in the microorganism.

The reduction of the expression amount and/or activity of the thiamine phosphate synthase in the microorganism may be implemented by introducing a pGRB-thiE sgRNA plasmid and a ΔthiE-Up-Down fragment mentioned in the examples into the microorganism.

The preparation method further includes a step (a5): after completing the step (a4), reducing the expression amount and/or activity of any of the above-mentioned malate dehydrogenase in the microorganism.

In the preparation method, the reduction of the expression amount and/or activity of the malate dehydrogenase in the microorganism is implemented by knocking out or knocking down the encoding gene (i.e., a mdh gene) of the malate dehydrogenase in the microorganism.

The reduction of the expression amount and/or activity of the malate dehydrogenase in the microorganism may be implemented by introducing a pGRB-mdh sgRNA plasmid and a Δmdh-Up-Down fragment mentioned in the examples into the microorganism.

The preparation method for the recombinant bacterium protected by the present invention may specifically be a method for preparing an engineered bacterium B. The method may include a step (b1): reducing the expression amount and/or activity of any of the above-mentioned alcohol dehydrogenase in a microorganism, wherein the microorganism may produce valine.

In the method, the reduction of the expression amount and/or activity of the alcohol dehydrogenase in the microorganism is implemented by knocking out or knocking down the encoding gene (i.e., an adhE gene) of the alcohol dehydrogenase in the microorganism.

The reduction of the expression amount and/or activity of the alcohol dehydrogenase in the microorganism is implemented by introducing a pGRB-adhE sgRNA plasmid and a ΔadhE-Up-Down fragment mentioned in the examples into the microorganism.

The method further includes a step (b2): after completing the step (b1), reducing the expression amount and/or activity of the microorganism-derived branched-chain amino acid transaminase, and expressing the branched-chain amino acid transaminase derived from *Bacillus subtilis* in the microorganism.

In the method, the reduction of the expression amount and/or activity of the branched-chain amino acid transaminase in the microorganism is implemented by knocking out or knocking down the encoding gene (i.e., an ilvE(E) gene) of the branched-chain amino acid transaminase in the microorganism. The expression of the branched-chain amino acid transaminase derived from *Bacillus subtilis* in the microorganism is implemented by knocking in or introducing the encoding gene (i.e., an ilvE(B) gene) of the branched-chain amino acid transaminase derived from *Bacillus subtilis* in the microorganism. The knock-in or introduction of the encoding gene of the branched-chain amino acid transaminase derived from *Bacillus subtilis* in the microorganism is implemented by introducing an expression cassette into the microorganism; and the expression cassette includes a promoter and the encoding gene of the branched-chain amino acid transaminase derived from *Bacillus subtilis.* The promoter may be an inducible promoter. The inducible promoter may specifically be a ptrc promoter.

The reduction of the expression amount and/or activity of the microorganism-derived branched-chain amino acid transaminase and the expression of the branched-chain amino acid transaminase derived from *Bacillus subtilis* in the microorganism may be implemented by introducing a pGRB-ilvE sgRNA plasmid and a ptrc-ilvE(B)-Up-Down fragment mentioned in the examples into the microorganism.

The preparation method further includes a step (b3): after completing the step (b2), reducing the expression amount and/or activity of any of the above-mentioned thiamine phosphate synthase in the microorganism.

In the method, the reduction of the expression amount and/or activity of the thiamine phosphate synthase in the microorganism is implemented by knocking out or knocking down the encoding gene (i.e., a thiE gene) of the thiamine phosphate synthase in the microorganism.

The reduction of the expression amount and/or activity of any of the above-mentioned thiamine phosphate synthase in the microorganism may be implemented by introducing a pGRB-thiE sgRNA plasmid and a ΔthiE-Up-Down fragment mentioned in the examples into the microorganism.

The method further includes a step (b4): after completing the step (b3), reducing the expression amount and/or activity of any of the above-mentioned malate dehydrogenase in the microorganism.

In the method, the reduction of the expression amount and/or activity of the malate dehydrogenase in the microorganism is implemented by knocking out or knocking down the encoding gene (i.e., a mdh gene) of the malate dehydrogenase in the microorganism.

The reduction of the expression amount and/or activity of the malate dehydrogenase in the microorganism may be implemented by introducing a pGRB-mdh sgRNA plasmid and a Δmdh-Up-Down fragment mentioned in the examples into the microorganism.

The preparation method for the recombinant bacterium protected by the present invention may specifically be a method for preparing an engineered bacterium C. The method may include a step (c1): reducing the expression amount and/or activity of the microorganism-derived branched-chain amino acid transaminase in the microorganism, and expressing the branched-chain amino acid transaminase derived from *Bacillus subtilis* in the microorganism, wherein the microorganism may produce valine.

In the preparation method, the reduction of the expression amount and/or activity of the branched-chain amino acid transaminase in the microorganism is implemented by knocking out or knocking down the encoding gene (i.e., an ilvE(E) gene) of the branched-chain amino acid transaminase in the microorganism. The expression of the branched-chain amino acid transaminase derived from *Bacillus subtilis* in the microorganism is implemented by knocking in or introducing the encoding gene (i.e., an ilvE(B) gene) of the branched-chain amino acid transaminase derived from *Bacillus subtilis* in the microorganism. The knock-in or introduction of the encoding gene of the branched-chain amino acid transaminase derived from *Bacillus subtilis* in the microorganism is implemented by introducing an expression cassette into the microorganism; and the expression cassette includes a promoter and the encoding gene of the branched-chain amino acid transaminase derived from *Bacillus subtilis.* The promoter may be an inducible promoter. The inducible promoter may specifically be a ptrc promoter.

The reduction of the expression amount and/or activity of the microorganism-derived branched-chain amino acid transaminase in the microorganism and the expression of the branched-chain amino acid transaminase derived from *Bacillus subtilis* in the microorganism may be implemented by introducing a pGRB-ilvE sgRNA plasmid and a ptrc-ilvE(B)-Up-Down fragment mentioned in the examples into the microorganism.

The preparation method further includes a step (c2): after completing the step (c1), reducing the expression amount and/or activity of any of the above-mentioned thiamine phosphate synthase in the microorganism.

In the preparation method, the reduction of the expression amount and/or activity of the thiamine phosphate synthase in the microorganism is implemented by knocking out or knocking down the encoding gene (i.e., a thiE gene) of the thiamine phosphate synthase in the microorganism.

The reduction of the expression amount and/or activity of any of the above-mentioned thiamine phosphate synthase in the microorganism may be implemented by introducing a pGRB-thiE sgRNA plasmid and a ΔthiE-Up-Down fragment mentioned in the examples into the microorganism.

The preparation method further includes a step (c3): after completing the step (c2), reducing the expression amount and/or activity of any of the above-mentioned malate dehydrogenase in the microorganism.

In the preparation method, the reduction of the expression amount and/or activity of the malate dehydrogenase in the microorganism is implemented by knocking out or knocking down the encoding gene (i.e., a mdh gene) of the malate dehydrogenase in the microorganism.

The reduction of the expression amount and/or activity of the malate dehydrogenase in the microorganism may be implemented by introducing a pGRB-mdh sgRNA plasmid and a Δmdh-Up-Down fragment mentioned in the examples into the microorganism.

The preparation method for the recombinant bacterium protected by the present invention may specifically be a method for preparing an engineered bacterium D. The method may include a step (d1): reducing the expression amount and/or activity of any of the above-mentioned alcohol dehydrogenase in a microorganism, wherein the microorganism may produce valine.

In the preparation method, the reduction of the expression amount and/or activity of the thiamine phosphate synthase in the microorganism is implemented by knocking out or knocking down the encoding gene (i.e., a thiE gene) of the thiamine phosphate synthase in the microorganism.

The reduction of the expression amount and/or activity of any of the above-mentioned thiamine phosphate synthase in the microorganism may be implemented by introducing a pGRB-thiE sgRNA plasmid and a ΔthiE-Up-Down fragment mentioned in the examples into the microorganism.

The preparation method further includes a step (d2): after completing the step (d1), reducing the expression amount and/or activity of any of the above-mentioned malate dehydrogenase in the microorganism.

In the preparation method, the reduction of the expression amount and/or activity of the malate dehydrogenase in the microorganism is implemented by knocking out or knocking down the encoding gene (i.e., a mdh gene) of the malate dehydrogenase in the microorganism.

The reduction of the expression amount and/or activity of the malate dehydrogenase in the microorganism may be implemented by introducing a pGRB-mdh sgRNA plasmid and a Δmdh-Up-Down fragment mentioned in the examples into the microorganism.

The preparation method for the recombinant bacterium protected by the present invention may specifically be a method for preparing an engineered bacterium E. The method may include a step (e1): reducing the expression amount and/or activity of any of the above-mentioned malate dehydrogenase in a microorganism, wherein the microorganism may produce valine.

In the preparation method, the reduction of the expression amount and/or activity of the malate dehydrogenase in the microorganism is implemented by knocking out or knocking down the encoding gene (i.e., a mdh gene) of the malate dehydrogenase in the microorganism.

The reduction of the expression amount and/or activity of the malate dehydrogenase in the microorganism may be implemented by introducing a pGRB-mdh sgRNA plasmid and a Δmdh-Up-Down fragment mentioned in the examples into the microorganism.

The present invention further protects a method for producing L-amino acid. The method may include the following steps: culturing any of the above-mentioned recombinant bacteria by means of fermentation, and collecting a fermentation product to obtain L-amino acid.

The present invention further protects use of any of the above-mentioned recombinant bacteria, which may be at least one of A2)-A5):
A2) the production of L-amino acid;
A3) the regulation of the yield of L-amino acid;
A4) the preparation of a product for producing L-amino acid; and
A5) the preparation of food, feed or drug containing L-amino acid.

As used herein, the culture may be carried out according to conventional methods in the art, including but not limited to: well plate culture, shaker flask culture, batch culture, continuous culture and batch feed culture, etc., and various culture conditions such as temperature, time and pH value of a medium can be appropriately adjusted according to actual situations.

As used herein, the L-amino acid may include L-valine, L-isoleucine, L-threonine, L-tryptophan, L-arginine, L-lysine, L-glutamic acid, L-glycine, L-alanine, L-leucine, L-methionine, L-proline, L-serine, L-tyrosine, L-cysteine, L-phenylalanine, L-asparagine, L-glutamine, L-aspartic acid and/or L-histidine.

Any of the above-mentioned L-amino acids may specifically be L-valine.

As used herein, the identity refers to the identity of an amino acid sequence. The identity of the amino acid sequence may be determined using a homology search site on Internet, such as a BLAST page of the NCBI homepage website. For example, in advanced BLAST2.1, with blastp as a program, an Expect value is set as 10, and all Filters are set as OFF; with BLOSUM62 as Matrix, the Gap existence cost, the Per residue gap cost, and the Lambda ratio are set as 11, 1 and 0.85 (default values), respectively; and the identity of the amino acid sequence is searched and calculated, and then the value of identity (%) can be obtained.

As used herein, the identity of 90% or more may be the identity of at least 90%, 91%, 92%, 93%, 94%, 95%, 96%, 97%, 98% or 99%.

As used herein, the microorganism may be yeast, bacteria, algae, or fungus. Bacteria may be derived from *Escherichia sp., Erwinia sp., Agrobacterium sp., Flavobacterium sp., Alcaligenes sp., Pseudomonas sp., Bacillus sp., Brevibacterium sp., Corynebacterium sp., Aerobacter sp., Enterobacteria sp., Micrococcus sp., Serratia sp., Salmonella sp., Streptomyces sp., Providencia sp.,* but is not limited thereto.

Further, the bacteria may be *Escherichia coli, Corynebacterium glutamicum, Brevibacterium lactofermentum, Brevibacterium flavum, Corynebacterium pekinense, Brevibacterium ammoniagenes, Corynebacterium crenatum* or *Pantoea,* but is not limited thereto. As used herein, the originating bacterium may be *Escherichia coli.*

In one or more embodiments of the present invention, the microorganism is *Escherichia coli.* Specifically, the *Escherichia coli* may be *Escherichia coli* YP045 CGMCC No. 22721 or *Escherichia coli* W3110.

The inventors of the present application have found through numerous experiments: (1) all engineered bacteria obtained by "knocking out a pflB gene", "knocking out a pflB gene and an adhE gene", "knocking out a pflB gene and an adhE gene and inserting an ilvE(B) gene from *Bacillus subtilis* initiated by ptrc while knocking out an ilvE(E) gene", "knocking out a pflB gene, an adhE gene and a thiE gene and inserting an ilvE(B) gene from *Bacillus subtilis* initiated by ptrc while knocking out an ilvE(E) gene", or "knocking out a pflB gene, an adhE gene, a thiE gene and an mdh gene and inserting an ilvE(B) gene from *Bacillus subtilis* initiated by ptrc while knocking out an ilvE(E) gene" can significantly increase the yield of L-amino acid (e.g., L-valine) in *Escherichia coli* (e.g., *Escherichia coli* YP045 CGMCC No. 22721 or *Escherichia coli* W3110) that can produce valine; (2) all engineered bacteria obtained by "knocking out an adhE gene", "knocking out an adhE gene and inserting an ilvE(B) gene from *Bacillus subtilis* initiated by ptrc while knocking out an ilvE(E) gene", "knocking out an adhE gene and a thiE gene and inserting an ilvE(B) gene from *Bacillus subtilis* initiated by ptrc while knocking out an ilvE(E) gene", or "knocking out an adhE gene, a thiE gene and an mdh gene and inserting an ilvE(B) gene from *Bacillus subtilis* initiated by ptrc while knocking out an ilvE(E) gene" can significantly increase the yield of L-amino acid (e.g., L-valine) in *Escherichia coli* (e.g., *Escherichia coli* YP045 CGMCC No. 22721 or *Escherichia coli* W3110) that can produce valine; all engineered bacteria obtained by "inserting an ilvE(B) gene from *Bacillus subtilis* initiated by ptrc while knocking out an ilvE(E) gene", "knocking out a thiE gene and inserting an ilvE(B) gene from *Bacillus subtilis* initiated by ptrc while knocking out an ilvE(E) gene", or "knocking out a thiE gene and an mdh gene and inserting an ilvE(B) gene from *Bacillus subtilis* initiated by ptrc while knocking out an ilvE(E) gene" can significantly increase the yield of L-amino acid (e.g., L-valine) in *Escherichia coli* (e.g., *Escherichia coli* YP045 CGMCC No. 22721 or *Escherichia coli* W3110) that can produce valine; and all engineered bacteria obtained by knocking out a thiE gene, knocking out an mdh gene, or knocking out an mdh gene while knocking out a thiE gene can significantly increase the yield of L-amino acid (e.g., L-valine) in *Escherichia coli* (e.g., *Escherichia coli* YP045 CGMCC No. 22721 or *Escherichia coli* W3110) that can produce valine. The present invention has an important application value.

### Preservation instructions

Strain name: *Escherichia coli*
Latin name: *Escherichia coli*
Classification name: *Escherichia coli*
Strain number: YP045
Title of preservation unit: China General Microbiological Culture Collection Center
Abbreviation of preservation unit: CGMCC
Address: No. 3, Courtyard 1, Beichen West Road, Chaoyang District, Beijing
Preservation date: June 15, 2021
Preservation Center registration number: CGMCC No. 22721

### Detailed Description of the Invention

The present application is described in further detail below in conjunction with the specific embodiments, and the given examples are only for the purpose of clarifying the present application, but not for the purpose of limiting the scope of the present application. The examples provided below may be used as a guide for further improvement by a person of ordinary skill in the art and do not in any way constitute a limitation of the present application.

The experimental methods in the following examples, unless otherwise specified, are conventional methods and are carried out in accordance with the techniques or conditions described in the literatures in the art or in accordance with the product instructions. The materials, reagents, etc. used in the following examples, unless otherwise specified, may be obtained commercially. *Escherichia coli* YP045 which can be used for producing valine had been preserved in the China General Microbiological Culture Collection Center (CGMCC for short, address: No. 3, Courtyard 1, Beichen West Road, Chaoyang District, Beijing) on June 15, 2021, with the preservation number of CGMCC No. 22721. The full name of *Escherichia coli* YP045 is *Escherichia coli* YP045 CGMCC No. 22721, hereinafter referred to as a valine-producing bacterium CGMCC 22721.

### Example 1: Modification I of engineered bacteria originating from valine-producing bacterium CGMCC 22721

After a large number of experiments, the inventors of the present invention obtained engineered bacteria YPVal-pflB01, YPVal-pflB02, YPVal-pflB03, YPVal-pflB04 and YPVal-pflB05 by means of modification originating from a valine-producing bacterium CGMCC 22721. Genotypes of the engineering bacteria YPVal-pflB01, YPVal-pflB02, YPVal-pflB03, YPVal-pflB04 and YPVal-pflB05 were shown in Table 1.

**Table 1**

| Strains | Genotypes |
|---|---|
| YPVal-pflB01 | CGMCC 22721-ΔpflB |
| YPVal-pflB02 | CGMCC 22721-ΔpflB-ΔadhE |
| YPVal-pflB03 | CGMCC 22721-ΔpflB-ΔadhE-ΔilvE(E)-ptrcilvE(B) |
| YPVal-pflB04 | CGMCC 22721-ΔpflB-ΔadhE-ΔilvE(E)-ptrcilvE(B)-ΔthiE |
| YPVal-pflB05 | CGMCC 22721-ΔpflB-ΔadhE-ΔilvE(E)-ptrcilvE(B)-ΔthiE-Δmdh |

### II. Obtainment of engineering bacterium YPVal-pflB01

According to a genome sequence of *Escherichia coli* W3110 published by NCBI, the engineered bacterium YPVal-pflB01 was obtained by knocking out a pflB gene from a genome of the valine-producing bacterium CGMCC 22721 using a CRISPR/Cas9 gene editing technology.

The pflB gene encodes pyruvate formate lyase with a Gene ID of 945514 and an amino acid sequence as shown in SEQ ID No. 13.

SEQ ID NO. 13 (pyruvate formate lyase pflB):
MSELNEKLATAWEGFTKGDWQNEVNVRDFIQKNYTPYEGDESFLAGATEATTTLWDKV
MEGVKLENRTHAPVDFDTAVASTITSHDAGYINKQLEKIVGLQTEAPLKRALIPFGGIKMIE
GSCKAYNRELDPMIKKIFTEYRKTHNQGVFDVYTPDILRCRKSGVLTGLPDAYGRGRIIGD
YRRVALYGIDYLMKDKLAQFTSLQADLENGVNLEQTIRLREEIAEQHRALGQMKEMAAK
YGYDISGPATNAQEAIQWTYFGYLAAVKSQNGAAMSFGRTSTFLDVYIERDLKAGKITEQ
EAQEMVDHLVMKLRMVRFLRTPEYDELFSGDPIWATESIGGMGLDGRTLVTKNSFRFLNT
LYTMGPSPEPNMTILWSEKLPLNFKKFAAKVSIDTSSLQYENDDLMRPDFNNDDYAIACC
VSPMIVGKQMQFFGARANLAKTMLYAINGGVDEKLKMQVGPKSEPIKGDVLNYDEVME
RMDHFMDWLAKQYITALNIIHYMHDKYSYEASLMALHDRDVIRTMACGIAGLSVAADSL
SAIKYAKVKPIRDEDGLAIDFEIEGEYPQFGNNDPRVDDLAVDLVERFMKKIQKLHTYRDA
IPTQSVLTITSNVVYGKKTGNTPDGRRAGAPFGPGANPMHGRDQKGAVASLTSVAKLPFAY
AKDGISYTFSIVPNALGKDDEVRKTNLAGLMDGYFHHEASIEGGQHLNVNVMNREMLLD
AMENPEKYPQLTIRVSGYAVRFNSLTKEQQQDVITRTFTQSM

The specific steps were as follows:

### 1. Construction of pGRB-pflB sgRNA plasmid

According to the genome sequence of *Escherichia coli* W3110 published by NCBI, an sgRNA target sequence GCGAATTTCTTGAAGTTCAGCGG (SEQ ID No. 1) from which a pf1B gene was knocked out was designed by using CRISPR RGEN Tools (http://www.rgenome.net/cas-designer/), and a linearized pGRB vector homologous arm sequence was added to ends 5' and 3' of the target sequence, in order to construct an sgRNA plasmid.
(1) A primer sgRNA-1F: 5'-TGACAGCTAGCTCAGTCCTAGTAGTAGATAATACTAGTgcgaatttttgagttcagcggGTTTAGA GCGGGTTTAGCAAGTTAAAATAAGG-3' (as shown in SEQ ID No. 19, underlined as a pGRB vector homologous arm sequence), a primer sgRNA-1R: 5'-CCTTATTTTAACTTGCTATTTCTAGCTCTAAAACccgctgaacttcaagaaattcgcACTAGTATTAT ACCTAGGACTGAGCTAGCTGTCA-3' (as shown in SEQ ID No. 20, underlined as a pGRB vector homologous arm sequence), a primer sgRNA-PF : 5'-GTCTCATGAGCGGATACATATTTG-3' (SEQ ID No. 21) and a primer sgRNA-PR: 5'-ATGAGAAAGCGCCACACGCT-3' (SEQ ID No. 22) were synthesized by Invitrogen.
(2) The primer sgRNA-1F and the primer sgRNA-1R were annealed (a reaction procedure: denaturation at 95°C for 5 min, and annealing at 50°C for 1 min); and then, a target fragment was recovered by a DNA purification kit, measured for a DNA concentration, and diluted in concentration to 100 ng/µL to obtain an annealing product. The annealing product contained an sgRNA-1 fragment with a nucleotide sequence as shown in SEQ ID No. 1.
(3) Approximately 2700 bp of DNA fragments were recovered by a DNA recovery kit (QIAGEN Gel Extraction Kit) by digesting a pGRB vector (Addgene's product, catalog number 71539) with a restriction enzyme *Spe*I (Takara's product, catalog number 1631).

The digestion system was 50 µL and composed of 5 µL of 10×Buffer (owned by the restriction enzyme *Spe*I), 2.5 µL of restriction enzyme *Spe*I, 3000-5000 ng of pGRB vector, and ddH₂O.

The digestion procedure was carried out at 37°C for 3 h.

(4) The DNA fragments recovered in step (3) were dephosphorylated (to prevent self-adhesion of the pGRB vector) and recovered by a DNA recovery kit to obtain a linearized pGRB vector.

The dephosphorylation system was 50 µL and composed of 5 µL of 10×Buffer (owned by CIAP), 1000-2000 ng of DNA fragments recovered in step (3), 2.5 µL of CIAP (Takara's product, catalog number 2250A), and ddH₂O.

The dephosphorylation procedure was carried out at 37°C for 1 h.

(5) The linearized pGRB vector obtained in step (4) and the annealing product obtained in step (2) were recombined by a Gibson Assembly kit (New England), and then transformed into competent cells (TAKARA) of *Escherichia coli* DH5α to obtain a pGRB-pflB sgRNA plasmid.

The recombination system was 5 µL and composed of 2 µL of linearized pGRB vector, 0.5 µL of annealing product, and 2.5 µL of assembly enzyme (owned by the Gibson Assembly kit).

The recombination procedure was carried out at 50°C for 30 min.

### 2. Obtainment of ΔpflB-Up-Down fragment

(1) According to a genome sequence of *Escherichia coli* W3110 published by NCBI, a primer pair for amplifying an upstream homologous arm and a primer pair for amplifying a downstream homologous arm were designed and synthesized by Invitrogen.

The primer pair for amplifying the upstream homologous arm was composed of a primer P1: 5'-CGTTGGTGTCCAGACAGGTATG-3' (as shown in SEQ ID No. 23) and a primer P2: 5'-GACATCCTGCGTTGCCGTAAATGAACCGTGAAATGCTGCTCG-3' (as shown in SEQ ID No. 24).

The primer pair for amplifying the downstream homologous arm was composed of a primer P3: 5'-CGAGCAGCATTTCACGGTTCATTTACGGCAACGCAGGATGTC-3' (as shown in SEQ ID No. 25) and a primer P4: 5'- TTTCTCACCTGACCGTGATG-3' (as shown in SEQ ID No. 26).

(2) Taking genomic DNA of *Escherichia coli* W3110 as a template, a primer pair composed of primers P1 and P2 was used for PCR amplification of a high-fidelity amplification enzyme KAPA HiFi HotStart, and an upstream homologous arm with a size of 686 bp was recovered by a DNA recovery kit.

(3) Taking genomic DNA of *Escherichia coli* W3110 as a template, a primer pair composed of primers P3 and P4 was used for PCR amplification of a high-fidelity amplification enzyme KAPA HiFi HotStart, and a downstream homologous arm with a size of 695 bp was recovered by a DNA recovery kit.

(4) Taking the upstream homologous arm recovered in step (2) and the downstream homologous arm recovered in step (3), which were mixed, as a template, a primer pair composed of primers P1 and P4 was used for overlap PCR to obtain a ΔpflB-Up-Down fragment as shown in SEQ ID No. 2.

SEQ ID No. 2: ΔpflB-Up-Down(1339bp)
CGTTGGTGTCCAGACAGGTATGAATGCCTTCTTTTTTGCAGGCGCGGAACCAGTCACG
AACAAACTCAGCTTGCAGGATTGCTTCACCGCCGGATGCGGTAACGCCGCCGCCGGAA
GCGTTCATAAAGTGGCGATAGGTCACCACTTCCTTCATCAAATCTTCAACGGTAACTTC
TTTACCGCCATGCGTGTCCCAGGTGTCGCGGTTATGACAATACAGGCAGCGCATCAGGC
AGCCCTGGAAAAAGGTGATAAAGCGAATACCTGGGCCGTCTACGGTTCCACAGGATTC
AAAGGAGTGAATGCGACCAATAACTGACATTGCGGTGTTTCTCCAGATGTGGCCCATC
TGAGGCCGTGTTGGTGCGCAGCTCGAAGGCTACGTCGAGTCTGTTTTGGCAGTCACCT
TAAAGTATAGATAGCTGACAAAAAAGGCTCTCGCGCTAAAAAAGGCCCCACTTTCGTG
GAGCCTTTATTGTACGCTTTTTACTGTACGATTTCAGTCAAATCTAATTACATAGATTGA
GTGAAGGTACGAGTAATAACGTCCTGCTGCTGTTCTTTAGTCAGCGAGTTGAAACGTA
CTGCGTAGCCAGATACACGGATGGTCAGCTGCGGATATTTTTCCGGGTTTTCCATCGCG
TCGAGCAGCATTTCACGGTTCATTTACGGCAACGCAGGATGTCCGGAGTGTAAACGTC

GAACACGCCCTGGTTGTGAGTTTTACGGTATTCAGTGAAGATTTTTTTGATCATCGGAT
CCAGTTCGCGGTTGTACGCTTTGCAGGAACCTTCGATCATTTTGATACCACCGAACGGG
ATAAGAGCACGTTTCAGCGGAGCTTCAGTCTGCAGACCAACGATTTTCTCAAGCTGCT
TGTTGATGTAGCCAGCGTCGTGAGAGGTGATGGTGGAAGCAACAGCGGTGTCAAAGT
CAACTGGCGCGTGAGTGCGGTTTTCCAGTTTAACGCCTTCCATTACTTTGTCCCACAGG
GTGGTGGTCGCTTCAGTAGCGCCAGCCAGGAAGGACTCGTCACCCTCGTACGGAGTGT
AGTTTTTCTGAATGAAGTCACGGACGTTTACTTCATTCTGCCAGTCACCTTTGGTAAAA
CCTTCCCAGGCTGTGGCTAACTTTTCATTAAGCTCGGACATGTAACACCTACCTTCTTA
AGTGGATTTTTTATTTACTGCGTACTTCGACAACCATTAATGGTGGTCGTTTTCACGCAG
GTAAATGACCCAGTATGTCAACCCAACCAACAAACCACCACCGATAATGTTGCCGATC
GTAACCGGAATCAGGTTATCAGTGATGAAATTCATCACGGTCAGGTGAGAAA

### 3. Obtainment of CGMCC 22721-Cas9 strain

(1) A plasmid pREDCas9 (Addgene's product, catalog number 71541; containing a Spectinomycin-resistant gene) was transformed into competent cells of a valine-producing bacterium CGMCC 22721, then coated on a 2-YT agar plate containing 100 mg/L Spectinomycin and cultured at 32°C to obtain Spectinomycin-resistant single colonies.

A preparation method for the 2-YT agar plate was as follows: dissolving 16 g of tryptone, 10 g of yeast extract powder, 5 g of sodium chloride and 16 g of agar powder in an appropriate amount of water, then metering a volume with water to 1 L, adjusting a pH value to 7.0 with sodium hydroxide, and sterilizing at 121°C for 20 min.

(2) Taking the single colonies obtained in step (1) as a template respectively, a PCR amplification product was obtained by performing PCR amplification using a primer pair composed of a primer pRedCas9-PF: 5'-GCAGTGGCGGTTTTCATG-3' (SEQ ID No. 27) and a primer pRedCas9-PR: 5'-CCTTGGTGATCTCGCCTTTC-3' (SEQ ID No.28). If the PCR amplification product obtained from a single colony contained a DNA fragment with a nucleotide sequence as shown in SEQ ID No. 3, this colony contained the plasmid pREDCas9. The strain of this colony was named as the CGMCC 22721-Cas9 strain.

SEQ ID No. 3: Identification of PCR amplification sequence (943 bp) of primer
pRedCas9-F/pRedCas9-R
GCAGTGGCGGTTTTCATGGCTTGTTATGACTGTTTTTTTGGGGTACAGTCTATGCCTCGG
GCATCCAAGCAGCAAGCGCGTTACGCCGTGGGTCGATGTTTGATGTTATGGAGCAGCA
ACGATGTTACGCAGCAGGGCAGTCGCCCTAAAACAAAGTTAAACATCATGAGGGAAG
CGGTGATCGCCGAAGTATCGACTCAACTATCAGAGGTAGTTGGCGTCATCGAGCGCCAT
CTCGAACCGACGTTGCTGGCCGTACATTTGTACGGCTCCGCAGTGGATGGCGGCCTGA
AGCCACACAGTGATATTGATTTGCTGGTTACGGTGACCGTAAGGCTTGATGAAACAAC

GCGGCGAGCTTTGATCAACGACCTTTTGGAAACTTCGGCTTCCCCTGGAGAGAGCGAG
ATTCTCCGCGCTGTAGAAGTCACCATTGTTGTGCACGACGACATCATTCCGTGGCGTTA
TCCAGCTAAGCGCGAACTGCAATTTGGAGAATGGCAGCGCAATGACATTCTTGCAGGT
ATCTTCGAGCCAGCCACGATCGACATTGATCTGGCTATCTTGCTGACAAAAGCAAGAG
AACATAGCGTTGCCTTGGTAGGTCCAGCGGCGGAGGAACTCTTTGATCCGGTTCCTGA
ACAGGATCTATTTGAGGCGCTAAATGAAACCTTAACGCTATGGAACTCGCCGCCCGACT
GGGCTGGCGATGAGCGAAATGTAGTGCTTACGTTGTCCCGCATTTGGTACAGCGCAGT
AACCGGCAAAATCGCGCCGAAGGATGTCGCTGCCGACTGGGCAATGGAGCGCCTGCC
GGCCCAGTATCAGCCCGTCATACTTGAAGCTAGACAGGCTTATCTTGGACAAGAAGAA
GATCGCTTGGCCTCGCGCGCAGATCAGTTGGAAGAATTTGTCCACTACGTGAAAGGCG
AGATCACCAAGG

### 4. Obtainment of engineering bacterium YPVal-pflB01

(1) Culture of CGMCC 22721-Cas9 strain; when the strain CGMCC 22721-Cas9 grew to OD₆₀₀ₙₘ of 0.1, IPTG was added such that the concentration of IPTG in the system was 0.1 mM, and continued to be cultured to induce λ-Red-mediated homologous recombination; and when the strain CGMCC 22721-Cas9 grew to OD₆₀₀ₙₘ of 0.6, bacterial cells were collected and competent cells of CGMCC 22721-Cas9 were prepared.
(2) The pGRB-pflB sgRNA plasmid obtained in step 1 and the ΔpflB-Up-Down fragment obtained in step 2 were transformed into competent cells of CGMCC 22721-Cas9, coated on a 2-YT agar plate containing 100 mg/L Spectinomycin and 100 mg/L Ampicillin, and cultured at 32°C to obtain single colonies.
(3) Taking the single colonies obtained in step (2) as a template respectively, a PCR amplification product was obtained by performing PCR amplification using a primer pair composed of primers P1 and P4. If the PCR amplification product obtained from a single colony contained a DNA fragment with a size of 1339 bp, this colony was preliminarily identified as a positive transformant.
(4) The positive transformant obtained in step (3) was inoculated on a 2-YT agar plate containing 100 mg/L Spectinomycin and 0.2% (m/v) arabinose and cultured at 32°C (for eliminating pGRB-pflB sgRNA plasmids); then, colonies that grew on a 2-YT agar plate containing 100 mg/L Spectinomycin but did not grow on a 2-YT agar plate containing 100 mg/L Ampicillin were picked out, then transferred to a 2-YT agar plate and cultured at 42°C (for eliminating plasmids pREDCas9); and finally, colonies which did not grow on a 2-YT agar plate containing 100 mg/L Spectinomycin but on an antibody-free 2-YT agar plate were picked out.
(5) Taking the single colonies obtained in step (4) as a template respectively, a PCR amplification product was obtained by performing PCR amplification using a primer pair composed of primers P1 and P4. If the PCR amplification product obtained from a single colony contained a DNA fragment with a size of 1339 bp, this colony was identified as a positive transformant. The positive transformant in which a pflB gene was knocked from a genome of a valine-producing bacterium CGMCC 22721 was named as the positive transformant YPVal-pflB01.

### II. Obtainment of engineering bacterium YPVal-pflB02

Taking the engineered bacterium YPVal-pflB01 obtained in step I as an originating bacterium, according to a genome sequence of *Escherichia coli* W3110 published by NCBI, an adhE gene was knocked from the engineered bacterium YPVal-pflB01 using a CRISPR/Cas9 gene editing technology.

The adhE gene encodes alcohol dehydrogenase with a Gene ID of 945837 and an amino acid sequence as shown in SEQ ID No. 14.

SEQ ID NO. 14(alcohol dehydrogenase adhE)
MAVTNVAELNALVERVKKAQREYASFTQEQVDKIFRAAALAAADARIPLAKMAVAESGM
GIVEDKVIKNHFASEYIYNAYKDEKTCGVLSEDDTFGTITIAEPIGIICGIVPTTNPTSTAIFKS
LISLKTRNAIIFSPHPRAKDATNKAADIVLQAAIAAGAPKDLIGWIDQPSVELSNALMHHP
DINLILATGGPGMVKAAYSSGKPAIGVGAGNTPVVIDETADIKRAVASVLMSKTFDNGVIC
ASEQSVVVVDSVYDAVRERFATHGGYLLQGKELKAVQDVILKNGALNAAIVGQPAYKIAE
LAGFSVPENTKILIGEVTVVDESEPFAHEKLSPTLAMYRAKDFEDAVEKAEKLVAMGGIGH
TSCLYTDQDNQPARVSYFGQKMKTARILINTPASQGGIGDLYNFKLAPSLTLGCGSWGGNS
ISENVGPKHLINKKTVAKRAENMLWHKLPKSIYFRRGSLPIALDEVITDGHKRALIVTDRF
LFNNGYADQITSVLKAAGVETEVFFEVEADPTLSIVRKGAELANSFKPDVIIALGGGSPMD
AAKIMWVMYEHPETHFEELALRFMDIRKRIYKFPKMGVKAKMIAVTTTSGTGSEVTPFAV
VTDDATGQKYPLADYALTPDMAIVDANLVMDMPKSLCAFGGLDAVTHAMEAYVSVLAS
EFSDGQALQALKLLKEYLPASYHEGSKNPVARERVHSAATIAGIAFANAFLGVCHSMAHK
LGSQFHIPHGLANALLICNVIRYNANDNPTKQTAFSQYDRPQARRRYAEIADHLGLSAPGD
RTAAKIEKLLAWLETLKAELGIPKSIREAGVQEADFLANVDKLSEDAFDDQCTGANPRYP
LISELKQILLDTYYGRDYVEGETAAKKEAAPAKAEKKAKKSA

The specific steps were as follows:

### 1. Construction of pGRB-adhE sgRNA plasmid

According to the genome sequence of *Escherichia coli* W3110 published by NCBI, an sgRNA target sequence AAGAACCACAACCCAGAGTCAGG (SEQ ID No. 4) from which an adhE was knocked out was designed by using CRISPR RGEN Tools (http://www.rgenome.net/cas-designer/), and a linearized pGRB vector homologous arm sequence was added to ends 5' and 3' of the target sequence, in order to construct an sgRNA plasmid.
(1) A primer sgRNA-2F: 5'- TGACAGCTAGCTCAGTCCTAGGTATAATACTAGTaagaaccacaacccagagtcaggGTTTTAGA GCTAGAAATAGCAAGTTAAAATAAGG-3' (as shown in SEQ ID No. 29, underlined as a pGRB vector homologous arm sequence) and a primer sgRNA-2R: 5'-CCTTATTTTAACTTGCTATTTCTAGCTCTAAAACcctgactctgggttgtggttcttACTAGTATTATA CCTAGGACTGAGCTAGCTGTCA-3' (as shown in SEQ ID No. 30, underlined as a pGRB vector homologous arm sequence) were synthesized by Invitrogen.
(2) The primer sgRNA-2F and the primer sgRNA-2R were annealed (a reaction procedure: denaturation at 95°C for 5 min, and annealing at 50°C for 1 min); and then, a target fragment was recovered by a DNA purification kit, measured for a DNA concentration, and diluted in concentration to 100 ng/µL to obtain an annealing product. The annealing product contained an sgRNA-2 fragment with a nucleotide sequence as shown in SEQ ID No. 4.
(3) Approximately 2700 bp of DNA fragments were recovered by a DNA recovery kit (QIAGEN Gel Extraction Kit) by digesting a pGRB vector with a restriction enzyme *Spe*I*.*

The digestion system was 50 µL and composed of 5 µL of 10×Buffer (owned by the restriction enzyme *Spe*I), 2.5 µL of restriction enzyme *Spe*I, 3000-5000 ng of pGRB vector, and ddH₂O.

The digestion procedure was carried out at 37°C for 3 h.

(4) The DNA fragments recovered in step (3) were dephosphorylated (to prevent self-adhesion of the pGRB vector) and recovered by a DNA recovery kit to obtain a linearized pGRB vector.

The dephosphorylation system was 50 µL and composed of 5 µL of 10×Buffer (owned by CIAP), 1000-2000 ng of DNA fragments recovered in step (3), 2.5 µL of CIAP and ddH₂O.

The dephosphorylation procedure was carried out at 37°C for 1 h.

(5) The linearized pGRB vector obtained in step (4) and the annealing product obtained in step (2) were recombined by a Gibson Assembly kit, and then transformed into competent cells of *Escherichia coli* DH5α to obtain a pGRB-adhE sgRNA plasmid.

The recombination system was 5 µL and composed of 2 µL of linearized pGRB vector, 0.5 µL of annealing product, and 2.5 µL of assembly enzyme (owned by the Gibson Assembly kit).

The recombination procedure was carried out at 50°C for 30 min.

### 2. Obtainment of ΔadhE-Up-Down fragment

(1) According to a genome sequence of *Escherichia coli* W3110 published by NCBI, a primer pair for amplifying an upstream homologous arm and a primer pair for amplifying a downstream homologous arm were designed and synthesized by Invitrogen.

The primer pair for amplifying the upstream homologous arm was composed of a primer P5: 5'-GTGCCAGTCATCCTTCAGGT-3' (as shown in SEQ ID No. 31) and a primer P6: 5'-CGTTCCGACCACTAACCCGACTTGGGTATTCCGAAATCTATCC-3' (as shown in SEQ ID No. 32).

The primer pair for amplifying the downstream homologous arm was composed of a primer P7: 5'-GGATAGATTTCGGAATACCCAAGTCGGGTTAGTGGTCGGAACG-3' (as shown in SEQ ID No. 33) and a primer P8: 5'-AAGCGATGCTGAAAGGTGTC-3' (as shown in SEQ ID No. 34).

(2) Taking genomic DNA of *Escherichia coli* W3110 as a template, a primer pair composed of primers P5 and P6 was used for PCR amplification of a high-fidelity amplification enzyme KAPA HiFi HotStart, and an upstream homologous arm with a size of 765 bp was recovered by a DNA recovery kit.

(3) Taking genomic DNA of *Escherichia coli* W3110 as a template, a primer pair composed of primers P7 and P8 was used for PCR amplification of a high-fidelity amplification enzyme KAPA HiFi HotStart, and a downstream homologous arm with a size of 643 bp was recovered by a DNA recovery kit.

(4) Taking the upstream homologous arm recovered in step (2) and the downstream homologous arm recovered in step (3), which were mixed, as a template, a primer pair composed of primers P5 and P8 was used for overlap PCR to obtain a ΔadhE-Up-Down fragment as shown in SEQ ID No. 5.

SEQ ID No. 5: ΔadhE-Up-Down(1365bp)
GTGCCAGTCATCCTTCAGGTAACGTTCCGCGCCCCTGTCTGTGCGGTCTGGCGGAAGA
GCCATTCCACTGGGGCGGCCCCCACGCGCTGACGCGCCTGGGTGACAGCGCTGCGGG
CCAGCAGGTTCATCCCCGCTTCGCCATCCGCGCTCAGGTTCAGACGGCGAACAACATC
GGTAATTGGCTCATTGCACCACCATCCAGATAACCATGTCCCCCGGTAAACGACGGCGG
CGAACGGTCGCATGAGCAGAAAGCGTCAGGCAGTGTTGTATCCACTCGGTGGGAAGG
TGTTCTGCAAATAGTTGTGCAGAGGGCGGAGGCATAAGCGGATGGTCACTGAAATCGA
GCAGATCATTGAGAAGTGGCATAAGAAAACGGCTCCCTGTTGTGGAAGCCGTTATAGT
GCCTCAGTTTAAGGATCGGTCAACTAATCCTTAACTGATCGGCATTGCCCAGAAGGGGC
CGTTTATGTTGCCAGACAGCGCTACTGATTAAGCGGATTTTTTCGCTTTTTTCTCAGCTT
TAGCCGGAGCAGCTTCTTTCTTCGCTGCAGTTTCACCTTCTACATAATCACGACCGTAG
TAGGTATCCAGCAGAATCTGTTTCAGCTCGGAGATCAGCGGGTAACGCGGGTTAGCGC
CGGTGCACTGGTCATCGAATGCATCTTCAGACAGTTTATCCACGTTCGCCAGGAAGTCT
GCTTCCTGAACGCCAGCTTCACGGATAGATTTCGGAATACCCAAGTCGGGTTAGTGGTC
GGAACGATACCGCAAATAATACCGATTGGTTCAGCGATAGTGATGGTACCAAAAGTGTC
GTCTTCAGACAGAACACCACAGGTTTTTTCATCTTTATAGGCGTTGTAGATATATTCAGA
AGCAAAGTGGTTTTTGATCACTTTATCTTCGACGATACCCATGCCGGATTCGGCAACGG
CCATTTTCGCGAGTGGGATTCGAGCATCTGCAGCAGCCAGAGCGGCGCGGAAGAT
TTTGTCTACTTGCTCTTGAGTGAAACTGGCATATTCACGCTGGGCTTTTTTTACACGCTC
TACGAGTGCGTTAAGTTCAGCGACATTAGTAACAGCCATAATGCTCTCCTGATAATGTTA

AACTTTTTTAGTAAATCATCTGCTCGAATACGAGAGTATAGTCAGTGCGGTGATGATTTG
CTTAACCTATGAAAATCAAAAGCTTACTCGCGCTCACACTCACTGTGATTTACTAAAAG
AGTTTAAACATTAGAGTTATTATCTCTAATGCGTCACTTCCAGGTGGCGTAAGCAAGATT
ACTCACTTCTGGGTACTGATTACGTGATCCAAATCAAATTTTTGCAAAGCTGACACCTT
TCAGCATCGCTT

### 3. Obtainment of YPVal- pflB01-Cas9 strain

(1) A plasmid pREDCas9 was transformed into competent cells of an engineered bacterium YPVal-pflB01, then coated on a 2-YT agar plate containing 100 mg/L Spectinomycin and cultured at 32°C to obtain Spectinomycin-resistant single colonies.
(2) Taking the single colonies obtained in step (1) as a template respectively, a PCR amplification product was obtained by performing PCR amplification using a primer pair composed of primers pRedCas9-PF and pRedCas9-PR. If the PCR amplification product obtained from a single colony contained a DNA fragment with a nucleotide sequence as shown in SEQ ID No. 3, this colony contained the plasmid pREDCas9. The strain of this colony was named as the YPVal-pflB01-Cas9 strain.

### 4. Obtainment of engineering bacterium YPVal-pflB02

(1) Culture of YPVal-pflB01-Cas9 strain; when the YPVal-pflB01-Cas9 strain grew to OD₆₀₀ₙₘ of 0.1, IPTG was added such that the concentration of IPTG in the system was 0.1 mM, and continued to be cultured to induce λ-Red-mediated homologous recombination; and when theYPVal-pflB01-Cas9 strain grew to OD₆₀₀ₙₘ of 0.6, bacterial cells were collected and competent cells of the YPVal-pflB01-Cas9 strain were prepared.
(2) The pGRB-adhE sgRNA plasmid obtained in step 1 and the ΔadhE-Up-Down fragment obtained in step 2 were transformed into competent cells of the YPVal-pflB01-Cas9 strain, coated on a 2-YT agar plate containing 100 mg/L Spectinomycin and 100 mg/L ampicillin, and cultured at 32°C to obtain single colonies.
(3) Taking the single colonies obtained in step (2) as a template respectively, a PCR amplification product was obtained by performing PCR amplification using a primer pair composed of primers P5 and P8. If the PCR amplification product obtained from a single colony contained a DNA fragment with a size of 1365 bp, this colony was preliminarily identified as a positive transformant.
(4) The positive transformant obtained in step (3) was inoculated on a 2-YT agar plate containing 100 mg/L Spectinomycin and 0.2% (m/v) arabinose and cultured at 32°C (for eliminating pGRB-adhE sgRNA plasmids); then, colonies that grew on a 2-YT agar plate containing 100 mg/L Spectinomycin but did not grow on a 2-YT agar plate containing 100 mg/L Ampicillin were picked out, then transferred to a 2-YT agar plate and cultured at 42°C (for eliminating plasmids pREDCas9); and finally, colonies which did not grow on a 2-YT agar plate containing 100 mg/L Spectinomycin but on an antibody-free 2-YT agar plate were picked out.
(5) Taking the single colonies obtained in step (4) as a template respectively, a PCR amplification product was obtained by performing PCR amplification using a primer pair composed of primers P5 and P8. If the PCR amplification product obtained from a single colony contained a DNA fragment with a size of 1365 bp, this colony was identified as a positive transformant in which an adhE gene was deleted from a genome of an engineered bacterium YPVal-pflB01. This positive transformant was named as the engineered bacterium YPVal-pflB02.

### III. Obtainment of engineering bacterium YPVal-pflB03

According to a genome sequence of *Escherichia coli* W3110 and a genome sequence of *Bacillus subtilis subsp. subtilis str.168* published by NCBI, an engineered bacterium YPVal-pflB03 (hereinafter referred to as YPVal-pflB03) was obtained by knocking in an ilvE gene (i.e., ptrc-ilvE(B) sequence) of *Bacillus subtilis* initiated by a ptrc promoter while knocking out an ilvE gene of a genome of an engineered bacterium YPVal-pflB02 obtained in step II by a CRISPR/Cas9 gene editing technology. A nucleotide sequence of the ptrc-ilvE(B) sequence was as shown in SEQ ID No. 6, in which positions 1096-1169 were a nucleotide sequence of the ptrc promoter from end 5', and positions 1-1095 were an ilvE(B) gene of *Bacillus subtilis.*

SEQ ID No. 6:
TTAACGTCTGCTTAATACACTGTGGCCGTTTTGCAAAAACTGGCTTCTTGAGCGGCGCA
TGCGTTCAATCCGTTCCTCTGCTAAGCGGTCAGCCGCTAAATATGCAGGAATGCCGTCA
CGCTGAGAAATCTCAAGTACACGCTCGATATTGCCGTAAATGCCTTCAACTTTTTTCAA
TGCACGTTCTGCATTATAGCCGTAAAGCTCATCTGCCACGTTGATGACACCGCCCGCGT
TAATCACGTAATCCGGTGCATAAACGATGCCCATTTCGTGAATTTGATCACCATGGCGTG
TCTCTTTTAATTGGTTGTTAGCCGCACCTGCGATCACTTTCGCCTTCAGCTGTTTAATGG
TGTCGTCGTTAATAGTCGCACCAAGGGCACACGGCGCATAAATATCGCAGTCTTGTGAA
TAAATGTCATCAGGATCTACCGCACGGGCGCCAAAATCTTCAACTGCACGCTGTACAGA
TTGTTTGTTGATATCCGTAACGATTAAGTTTGCTCCTTCTTCATGCAGGTGGCGGCAAA
GGTTATAGGCTACGTTCCCAACACCCTGTACAGCAATGGTTTTTCCTTCAAGAGAGTCG
GTTCCGAAAGCAGCTTTAGCTGCTGCCTTCATTCCTCTGTACACCCCGTACGCTGTGAC
TGGGGACGGATTTCCAGAAGAGCCGAAAGCAGGAGAAATCCCTGTGACATAGTCTGT
CTCATCATGAATGATATCCATATCCTCGACCGTTGTGCCCACATCTTCAGCCGTGATGTA
TCTGCCATTCAGTCCTTGAATATAGCGGCCAAACGCGCGGAACATTTCCTCATTTTTGT
CTTTGCGCGGATCGCCGATAATGACTGTTTTTCCGCCGCCAAGGTTTAAGCCTGCCGCC
GCGTTCTTATAGGTCATGCCTCTTGCCAATCTGAGCGCATCTTCAATTGCCGCTTCTTCA
TTTTCATATGTCCACATTCTCGTTCCGCCAAGCGCCGGACCAAGCGTTGTATCATGAAT

GGCGATAATCGCTTTTAATCCAGATTGTTCATCCTGGCAGAATACCAATTGTTCGTAATC
GTATTTCTCCATATATTTAAAAAGTTCCATGGTCTGTTTCCTGTGTGAAATTGTTATCCGC
TCACAATTCCACACATTATACGAGCCGGATGATTAATTGTCAA

The ilvE(E) gene of the engineering bacterium YPVal-pflB02 encodes branched-chain amino acid transaminase, with Gene ID of 948278 and an amino acid sequence as shown in SEQ ID No. 15.

SEQ ID NO. 15 (branched-chain amino acid transaminase ilvE)
MTTKKADYIWFNGEMVRWEDAKVHVMSHALHYGTSVFEGIRCYDSHKGPVVFRHREH
MQRLHDSAKIYRFPVSQSIDELMEACRDVIRKNNLTSAYIRPLIFVGDVGMGVNPPAGYST
DVIIAAFPWGAYLGAEALEQGIDAMVSSWNRAAPNTIPTAAKAGGNYLSSLLVGSEARRH
GYQEGIALDVNGYISEGAGENLFEVKDGVLFTPPFTSSALPGITRDAIIKLAKELGIEVREQ
VLSRESLYLADEVFMSGTAAEITPVRSVDGIQVGEGRCGPVTKRIQQAFFGLFTGETEDKW
GWLDQVNQ

The ilvE(B) gene of *Bacillus subtilis* encodes branched-chain amino acid transaminase, with Gene ID of 938420 and an amino acid sequence as shown in SEQ ID No. 16.

SEQ ID NO. 16 (branched-chain amino acid transaminase ilvE)
MNKLIEREKTVYYKEKPDPSSLGFGQYFTDYMFVMDYEEGIGWHHPRIAPYAPLTLDPSS
SVFHYGQAVFEGLKAYRTDDGRVLLFRPDQNIKRLNRSCERMSMPPLDEELVLEALTQLV
ELEKDWVPKEKGTSLYIRPFVIATEPSLGVKASRSYTFMIVLSPVGSYYGDDQLKPVRIYV
EDEYVRAVNGGVGFAKTAGNYAASLQAQRKANELGYDQVLWLDAIEKKYVEEVGSMNI
FFVINGEAVTPALSGSILSGVTRASAIELIRSWGIPVREERISIDEVYAASARGELTEVFGTGT
AAVVTPVGELNIHGKTVIVGDGQIGDLSKKLYETITDIQLGKVKGPFNWTVEV

The specific steps were as follows:

### 1. Construction of pGRB-ilvE sgRNA plasmid

(1) A primer sgRNA-3F: 5'-TGACAGCTAGCTCAGTCCTAGGTATAATACTAGTgaaagcagcgataatcacgtcgGTTTTAGAG CTAGAAATAGCAAGTTAAAATAAGG-3' (as shown in SEQ ID No. 35, underlined as a pGRB vector homologous arm sequence) and a primer sgRNA-3R: 5'-CCTTATTTTAACTTGCTATTTCTAGCTCTAAAACccgacgtgattatcgctgctttcACTAGTATTATA CCTAGGACTGAGCTAGCTGTCA-3' (as shown in SEQ ID No. 36, underlined as a pGRB vector homologous arm sequence) were synthesized by Invitrogen.
(2) The primer sgRNA-3F and the primer sgRNA-3R were annealed (a reaction procedure: denaturation at 95°C for 5 min, and annealing at 50°C for 1 min); and then, a target fragment was recovered by a DNA purification kit, measured for a DNA concentration, and diluted in concentration to 100 ng/µL to obtain an annealing product. The annealing product contained an sgRNA-3 fragment with a nucleotide sequence: GAAAGCAGCGATAATCACGTCGG (as shown in SEQ ID No. 7).
(3) Approximately 2700 bp of DNA fragments were recovered by a DNA recovery kit (QIAGEN Gel Extraction Kit) by digesting a pGRB vector (Addgene's product, catalog number 71539) with a restriction enzyme *Spe*I (Takara's product, catalog number 1631).

The digestion system was 50 µL and composed of 5 µL of 10×Buffer (owned by the restriction enzyme *Spe*I)*,* 2.5 µL of restriction enzyme *Spe*I, 3000-5000 ng of pGRB vector, and ddH₂O.

The digestion procedure was carried out at 37°C for 3 h.

(4) The DNA fragments recovered in step (3) were dephosphorylated (to prevent self-adhesion of the pGRB vector) and recovered by a DNA recovery kit to obtain a linearized pGRB vector.

The dephosphorylation system was 50 µL and composed of 5 µL of 10×Buffer (owned by CIAP), 1000-2000 ng of DNA fragments recovered in step (3), 2.5 µL of CIAP (Takara's product, catalog number 2250A), and ddH₂O.

The dephosphorylation procedure was carried out at 37°C for 1 h.

(5) The linearized pGRB vector obtained in step (4) and the annealing product obtained in step (2) were recombined by a Gibson Assembly kit (New England), and then transformed into competent cells (TAKARA) of *Escherichia coli* DH5α to obtain a pGRB-ilvE sgRNA plasmid.

The recombination system was 5 µL and composed of 2 µL of linearized pGRB vector, 0.5 µL of annealing product, and 2.5 µL of assembly enzyme (owned by the Gibson Assembly kit).

The recombination procedure was carried out at 50°C for 30 min.

### 2. Obtainment of ptrc-ilvE(B)-Up-Down fragment

(1) According to a genome sequence of *Escherichia coli* W3110 published by NCBI, a primer pair for amplifying an upstream homologous arm and a primer pair for amplifying a downstream homologous arm were designed and synthesized by Invitrogen. According to the genome sequence of *Bacillus subtilis subsp. subtilis str.168* published by NCBI, a primer pair was designed and synthesized by Invitrogen for amplifying ptrc-ilvE(B).

The primer pair for amplifying the upstream homologous arm was composed of a primer P9: 5'-CAGGCAGTTCATTGAGTTAGCG-3' (as shown in SEQ ID No. 37) and a primer P10: 5'-CACAGTGTATTAAGCAGACGTTAAATACAAAAAATGGGACGGCAC-3' (as shown in SEQ ID No. 38).

The primer pair for amplifying the downstream homologous arm was composed of a primer P13: 5'-GTTATCCGCTCAATTCCACACACATTATACGAGCCGGATGATTATGTCAATTGTCAATT TTATTTTTTGCGCTC-3' (as shown in SEQ ID No. 39, underlined as a partial sequence of the ptrc promoter) and a primer P14: 5'-ACGGTTAGGGATGGTTCGAC-3' (SEQ ID No. 40).

The primer pair for amplifying ptrc-ilvE(B) was composed of a primer P11: 5'-GTGCCGTCCCATTTTTTGTATTTAACGTCTGCTTAATACACTGTG-3' (as shown in SEQ ID No. 41) and a primer P12: 5'-GTATAATGTGTGGAATTGTGAGCGGATAACAATTTCACACAGGAAACAGACCATGGA ACTTTTTAAATATATGGAG-3' (SEQ ID No. 42, underlined as a partial sequence of the ptrc promoter).

(2) Taking genomic DNA of *Escherichia coli* W3110 as a template, a primer pair composed of primers P9 and P10 was used for PCR amplification of a high-fidelity amplification enzyme KAPA HiFi HotStart, and an upstream homologous arm with a size of 709 bp was recovered by a DNA recovery kit.

(3) Taking genomic DNA of *Escherichia coli* W3110 as a template, a primer pair composed of primers P13 and P14 was used for PCR amplification of a high-fidelity amplification enzyme KAPA HiFi HotStart, and a downstream homologous arm with a size of 611 bp was recovered by a DNA recovery kit.

(4) Taking genomic DNA of *Bacillus subtilis subsp.subtilis str.168* as a template, a primer pair composed of primers P11 and P12 was used for PCR amplification of a high-fidelity amplification enzyme KAPA HiFi HotStart, and an ilvE(B) fragment with a size of 1168 bp was recovered by a DNA recovery kit.

(5) Taking the upstream homologous arm recovered in step (2), the downstream homologous arm recovered in step (3) and the ilvE(B) fragment recovered in step (4), which were mixed, as a template, a primer pair composed of primers P9 and P14 was used for overlap PCR to obtain a ptrc-ilvE(B)-Up-Down fragment as shown in SEQ ID No. 8.

SEQ ID No. 8: ptrc-ilvE(B)-Up-Down(2413bp)
CAGGCAGTTCATTGAGTTAGCGGTAAACATCCCGGAGCAGGAACCGCAGGTCGGACA
CGCGGAACGTTCAACCTGATCGCTCTGGGAGTCAGATACTTTCGGGTCTGCGCCCTGG
ATCATCGCATCAACCAGATCGAGCTTGATGATCTGATCGGAAAGTTTGGTTTTCCCGGC
CTCCATCGGGCCGCCGGAAACAAAGATCACCGGAATATTCAGGCGCAGGGAAGCCATC
AGCATCCCCGGGGTGATTTTGTCGCAGTTAGAGATGCAGACCATGGCGTCGGCGCAGT
GGGCGTTGACCATATACTCAACGGAATCAGCGATCAGTTCGCGAGATGGCAGTGAATA
AAGCATCCCCCCGTGGCCCATGGCAATCCCATCATCCACCGCAATGGTGTTGAACTCTT
TGGCAACGCCGCCAGCCGCTTCAATTTGTTCGGCGACCAGTTTACCGAGATCGCGCAG
ATGGACGTGACCCGGTACAAATTGGGTGAACGAGTTCACAACCGCGATAATCGGCTTA
CCGAAATCGGCGTCGGTCATTCCGGTGGCGCGCCACAGCGCACGAGCACCCGCCATAT
TACGACCATGAGTGGTGGTGGCGGAACGGTACTTAGGCATACTTTATTTACTCCCAGTG
TCTGTCTCGTAAATGGGACGGTGCGTGCCGTCCCATTTTTTGTATTTAACGTCTGCTTAA
TACACTGTGGCCGTTTTGCAAAAACTGGCTTCTTGAGCGGCGCATGCGTTCAATCCGTT
CCTCTGCTAAGCGGTCAGCCGCTAAATATGCAGGAATGCCGTCACGCTGAGAAATCTC

AAGTACACGCTCGATATTGCCGTAAATGCCTTCAACTTTTTTCAATGCACGTTCTGCATT
ATAGCCGTAAAGCTCATCTGCCACGTTGATGACACCGCCCGCGTTAATCACGTAATCCG
GTGCATAAACGATGCCCATTTCGTGAATTTGATCACCATGGCGTGTCTCTTTTAATTGGT
TGTTAGCCGCACCTGCGATCACTTTCGCCTTCAGCTGTTTAATGGTGTCGTCGTTAATAG
TCGCACCAAGGGCACACGGCGCATAAATATCGCAGTCTTGTGAATAAATGTCATCAGGA
TCTACCGCACGGGCGCCAAAATCTTCAACTGCACGCTGTACAGATTGTTTGTTGATATC
CGTAACGATTAAGTTTGCTCCTTCTTCATGCAGGTGGCGGCAAAGGTTATAGGCTACGT
TCCCAACACCCTGTACAGCAATGGTTTTTCCTTCAAGAGAGTCGGTTCCGAAAGCAGC
TTTAGCTGCTGCCTTCATTCCTCTGTACACCCCGTACGCTGTGACTGGGGACGGATTTC
CAGAAGAGCCGAAAGCAGGAGAAATCCCTGTGACATAGTCTGTCTCATCATGAATGAT
ATCCATATCCTCGACCGTTGTGCCCACATCTTCAGCCGTGATGTATCTGCCATTCAGTCC
TTGAATATAGCGGCCAAACGCGCGGAACATTTCCTCATTTTTGTCTTTGCGCGGATCGC
CGATAATGACTGTTTTTCCGCCGCCAAGGTTTAAGCCTGCCGCCGCGTTCTTATAGGTC
ATGCCTCTTGCCAATCTGAGCGCATCTTCAATTGCCGCTTCTTCATTTTCATATGTCCAC
ATTCTCGTTCCGCCAAGCGCCGGACCAAGCGTTGTATCATGAATGGCGATAATCGCTTT
TAATCCAGATTGTTCATCCTGGCAGAATACCAATTGTTCGTAATCGTATTTCTCCATATAT
TTAAAAAGTTCCATGGTCTGTTTCCTGTGTGAAATTGTTATCCGCTCACAATTCCACACA
TTATACGAGCCGGATGATTAATTGTCAATTTTATATTCCTTTTGCGCTCAGGCGCGGATTT
GTTGTGATGTGGTTGTGCTCTGGCAGATGGCAACGTGTGCGACGTCCACCAGTTTATTT
AACTGACTAAACAGTAAGTCGACCGACCGTGGGCTGGCAACGGTCAATTCGATATTTAT
ATTTTGTGCATCGCTGGCGGCGGCCATATTCATTGAGCAGACGTGGAAACCACGATGAC
GCACCACGCGTAAAACACGTTCTAAGGTTTCTGGATTGAAGCGAGCCGATACATTGAC
CTGATGTTGCATCATGATAATTTCTCCAACATTTCTGAATTACTGGCGCCAGGCGGCACC
AGCGGCCAGACGTTCTCAAGTTCGTCGATTGAGACATGAAGCAGGTATGGCCCATCAC
TGTTCAGCATGGTGTCGAGTGCCGCTTCAACCTGGTCTTTCCGGGTGATGTGTTGGCCA
TGGATGCCGAAGGCGCTGGCTAACATGAGGAAATCGGGGTTATCAGTAAGGGTGGTTT
CGCTGTATCGTTCCTGAAAAAACAGTTGCTGCCATTGTCGAACCATCCCTAACCGT

### 3. Obtainment of YPVal- pflB02-Cas9 strain

(1) A plasmid pREDCas9 was transformed into competent cells of an engineered bacterium YPVal-pflB02, then coated on a 2-YT agar plate containing 100 mg/L Spectinomycin and cultured at 32°C to obtain Spectinomycin-resistant single colonies.
(2) Taking the single colonies obtained in step (1) as a template respectively, a PCR amplification product was obtained by performing PCR amplification using a primer pair composed of primers pRedCas9-PF and pRedCas9-PR. If the PCR amplification product obtained from a single colony contained a DNA fragment with a nucleotide sequence as shown in SEQ ID No. 3, this colony contained the plasmid pREDCas9. The strain of this colony was named as the YPVal-pflB02-Cas9 strain.

### 4. Obtainment of engineering bacterium YPVal-pflB03

(1) Culture of YPVal-pflB02-Cas9 strain; when the YPVal-pflB02-Cas9 strain grew to OD₆₀₀ₙₘ of 0.1, IPTG was added such that the concentration of IPTG in the system was 0.1 mM, and continued to be cultured to induce λ-Red-mediated homologous recombination; and when the YPVal-pflB02-Cas9 strain grew to OD₆₀₀ₙₘ of 0.6, bacterial cells were collected and competent cells of the YPVal-pflB02-Cas9 strain were prepared.
(2) The pGRB-ilvE sgRNA plasmid obtained in step 1 and the ptrc-ilvE(B)-Up-Down fragment obtained in step 2 were transformed into competent cells of the YPVal-pflB02-Cas9 strain, coated on a 2-YT agar plate containing 100 mg/L Spectinomycin and 100 mg/L Ampicillin, and cultured at 32°C to obtain single colonies.
(3) Taking the single colonies obtained in step (2) as a template respectively, a PCR amplification product was obtained by performing PCR amplification using a primer pair composed of primers P11 and P12. If the PCR amplification product obtained from a single colony contained a DNA fragment with a size of 1168 bp, this colony was preliminarily identified as a positive transformant.
(4) The positive transformant obtained in step (3) was inoculated on a 2-YT agar plate containing 100 mg/L Spectinomycin and 0.2% (m/v) arabinose and cultured at 32°C (for eliminating pGRB-ilvE sgRNA plasmids); then, colonies that grew on a 2-YT agar plate containing 100 mg/L Spectinomycin but did not grow on a 2-YT agar plate containing 100 mg/L Ampicillin were picked out, then transferred to a 2-YT agar plate and cultured at 42°C (for eliminating plasmids pREDCas9); and finally, colonies which did not grow on a 2-YT agar plate containing 100 mg/L Spectinomycin but on an antibody-free 2-YT agar plate were picked out.
(5) Taking the single colonies obtained in step (4) as a template respectively, a PCR amplification product was obtained by performing PCR amplification using a primer pair composed of primers P11 and P12. If the PCR amplification product obtained from a single colony contained a DNA fragment with a size of 1168 bp, this colony was identified as a positive transformant. The positive transformant in which an ilvE(B) gene (i.e., a ptrc-ilvE(B) sequence) of *Bacillus subtilis* initiated by a ptrc promoter was knocked in while an ilvE(E) gene was knocked from a genome of an engineered bacterium YPVal-pflB02 was named as YPVal-pflB03.

### IV. Obtainment of engineering bacterium YPVal-pflB04

Taking the engineered bacterium YPVal-pflB03 as an originating bacterium, according to a genome sequence of *Escherichia coli* W3110 published by NCBI, a thiE gene was knocked from the engineered bacterium YPVal-pflB03 using a CRISPR/Cas9 gene editing technology.

The thiE gene encodes thiamine phosphate synthase with a Gene ID of 948491 and an amino acid sequence as shown in SEQ ID No. 17.

SEQ ID NO. 17 (thiamine phosphate synthase thiE)
MYQPDFPPVPFRSGLYPVVDSVQWIERLLDAGVRTLQLRIKDRRDEEVEADVVAAIALGR
RYNARLFINDYWRLAIKHQAYGVHLGQEDLQATDLNAIRAAGLRLGVSTHDDMEIDVAL
AARPSYIALGHVFPTQTKQMPSAPQGLEQLARHVERLADYPTVAIGGISLARAPAVIATGV
GSIAVVSAITQAADWRLATAQLLEIAGVGDE

The specific steps were as follows:

### 1. Construction of pGRB-thiE sgRNA plasmid

According to the genome sequence of *Escherichia coli* W3110 published by NCBI, an sgRNA target sequence CGCCCCTCTTATATCGCGCTGGG (SEQ ID No. 9) from which a thiE gene was knocked out was designed by using CRISPR RGEN Tools (http://www.rgenome.net/cas-designer/), and a linearized pGRB vector homologous arm sequence was added to ends 5' and 3' of the target sequence, in order to construct an sgRNA plasmid.
(1) A primer sgRNA-4F: 5'-TGACAGCTAGCTCAGTCCTAGGTATAATACTAGTcgcccctcttatatcgcgctgggGTTTTAGAG CTAGAAATAGCAAGTTAAAATAAGG-3' (as shown in SEQ ID No. 43, underlined as a pGRB vector homologous arm sequence) and a primer sgRNA-4R: 5'-CCTTATTTTAACTTGCTATTTCTAGCTCTAAAACcccagcgcgatataagaggggcgACTAGTATTA TACCTAGGACTGAGCTAGCTGTCA-3' (as shown in SEQ ID No. 44, underlined as a pGRB vector homologous arm sequence) were synthesized by Invitrogen.
(2) The primer sgRNA-4F and the primer sgRNA-4R were annealed (a reaction procedure: denaturation at 95°C for 5 min, and annealing at 50°C for 1 min); and then, a target fragment was recovered by a DNA purification kit, measured for a DNA concentration, and diluted in concentration to 100 ng/µL to obtain an annealing product. The annealing product contained an sgRNA-4 fragment with a nucleotide sequence as shown in SEQ ID No. 9.
(3) Approximately 2700 bp of DNA fragments were recovered by a DNA recovery kit (QIAGEN Gel Extraction Kit) by digesting a pGRB vector with a restriction enzyme *Spe*I.

The digestion system was 50 µL and composed of 5 µL of 10×Buffer (owned by the restriction enzyme *Spe*I), 2.5 µL of restriction enzyme *Spe*I, 3000-5000 ng of pGRB vector, and ddH₂O.

The digestion procedure was carried out at 37°C for 3 h.

(4) The DNA fragments recovered in step (3) were dephosphorylated (to prevent self-adhesion of the pGRB vector) and recovered by a DNA recovery kit to obtain a linearized pGRB vector.

The dephosphorylation system was 50 µL and composed of 5 µL of 10×Buffer (owned by CIAP), 1000-2000 ng of DNA fragments recovered in step (3), 2.5 µL of CIAP and ddH₂O.

The dephosphorylation procedure was carried out at 37°C for 1 h.

(5) The linearized pGRB vector obtained in step (4) and the annealing product obtained in step (2) were recombined by a Gibson Assembly kit, and then transformed into competent cells of *Escherichia coli* DH5α to obtain a pGRB-thiE sgRNA plasmid.

The recombination system was 5 µL and composed of 2 µL of linearized pGRB vector, 0.5 µL of annealing product, and 2.5 µL of assembly enzyme (owned by the Gibson Assembly kit).

The recombination procedure was carried out at 50°C for 30 min.

### 2. Obtainment of ΔthiE-Up-Down fragment

(1) According to a genome sequence of *Escherichia coli* W3110 published by NCBI, a primer pair for amplifying an upstream homologous arm and a primer pair for amplifying a downstream homologous arm were designed and synthesized by Invitrogen.

The primer pair for amplifying the upstream homologous arm was composed of a primer P15: 5'-TTCTATTCAGGACGCCAACG-3' (as shown in SEQ ID No. 45) and a primer P16: 5'-GCTATAACGCATAAAGTCACGGCACGCTTCCTCCTTACGCAGG-3' (as shown in SEQ ID No. 46).

The primer pair for amplifying the downstream homologous arm was composed of a primer P17: 5'-CCTGCGTAAGGAGGAAGCGTGCCGTGACTTTATGCGTTATAGC-3' (as shown in SEQ ID No. 47) and a primer P18: 5'-GCCTGCAAAGTGCCCATAACCC-3' (as shown in SEQ ID No. 48).

(2) Taking genomic DNA of *Escherichia coli* W3110 as a template, a primer pair composed of primers P15 and P16 was used for PCR amplification of a high-fidelity amplification enzyme KAPA HiFi HotStart, and an upstream homologous arm with a size of 721 bp was recovered by a DNA recovery kit.

(3) Taking genomic DNA of *Escherichia coli* W3110 as a template, a primer pair composed of primers P17 and P18 was used for PCR amplification of a high-fidelity amplification enzyme KAPA HiFi HotStart, and a downstream homologous arm with a size of 618 bp was recovered by a DNA recovery kit.

(4) Taking the upstream homologous arm recovered in step (2) and the downstream homologous arm recovered in step (3), which were mixed, as a template, a primer pair composed of primers P15 and P18 was used for overlap PCR to obtain a ΔthiE-Up-Down fragment as shown in SEQ ID No. 10.

SEQ ID No. 10: ΔthiE-Up-Down(1296bp)
TTCTATTCAGGACGCCAACGATGAAGCGCAGTTTGCCGAGCTGCATACGCTGGGCGAA
CTGACCAAAATTGCCTGGGAATATGACGTGCAGGTGATGATTGAAGGCCCAGGCCACG
TGCCGATGCAGATGATCCGCCGCAATATGACCGAGGAGTTAGAGCACTGCCACGAAGC

GCCGTTTTACACTCTGGGGCCGCTAACTACCGATATTGCGCCGGGCTATGACCACTTCA
CGTCGGGGATTGGTGCGGCGATGATTGGCTGGTTTGGCTGCGCGATGCTCTGTTACGTA
ACGCCAAAAGAGCATCTGGGTCTGCCCAATAAAGAAGATGTTAAGCAGGGGCTTATCA
CCTATAAGATTGCTGCCCACGCCGCTGACCTGGCGAAAGGGCATCCGGGCGCGCAAAT
TCGCGATAACGCCATGTCGAAAGCCCGCTTCGAATTTCGCTGGGAAGACCAGTTTAAT
CTGGCCCTCGACCCGTTTACCGCCCGCGCTTATCACGATGAAACCCTGCCGCAAGAGT
CAGGTAAAGTCGCCCATTTTTGCTCCATGTGTGGGCCGAAATTCTGCTCGATGAAAATC
AGCCAGGAAGTGCGTGATTACGCCGCCACGCAAACTATTGAAATGGGAATGGCGGATA
TGTCGGAGAACTTCCGTGCCAGAGGCGGAGAAATCTACCTGCGTAAGGAGGAAGCGT
GCCGTGACTTTATGCGTTATAGCCGCCAAATCCTGCTCGACGATATCGCTCTGGACGGG
CAGCAAAAACTGCTCGACAGCCAGGTGCTGATTATCGGTCTGGGCGGGCTGGGTACAC
CTGCTGCGCTGTACCTGGCGGGCGCTGGCGTCGGGACGCTGGTACTGGCAGATGACGA
CGATGTGCATTTAAGCAATCTGCAACGACAAATCCTCTTTACCACTGAAGATATCGATC
GCCCGAAATCGCAGGTCAGCCAACAGCGACTGACACAGTTGAATCCCGACATTCAACT
GACAGCATTACAACAACGGTTAACGGGTGAGGCGTTAAAAGATGCGGTTGCACGGGC
CGATGTGGTGCTCGACTGTACCGACAATATGGCGACTCGCCAGGAGATTAATGCCGCCT
GCGTGGCACTCAACACGCCGCTTATCACCGCCAGCGCGGTCGGATTTGGCGGTCAGTT
GATGGTACTGACGCCGCCCTGGGAGCAGGGGTGTTACCGCTGCCTGTGGCCAGATAAC
CAGGAGCCAGAACGCAACTGCCGCACGGCGGGCGTGGTTGGCCCGGTGGTCGGGGTT
ATGGGCACTTTGCAGGC

### 3. Obtainment of YPVal- pflB03-Cas9 strain

(1) A plasmid pREDCas9 was transformed into competent cells of an engineered bacterium YPVal-pflB03, then coated on a 2-YT agar plate containing 100 mg/L Spectinomycin and cultured at 32°C to obtain Spectinomycin-resistant single colonies.
(2) Taking the single colonies obtained in step (1) as a template respectively, a PCR amplification product was obtained by performing PCR amplification using a primer pair composed of primers pRedCas9-PF and pRedCas9-PR. If the PCR amplification product obtained from a single colony contained a DNA fragment with a nucleotide sequence as shown in SEQ ID No. 3, this colony contained the plasmid pREDCas9. The strain of this colony was named as the YPVal-pflB03-Cas9 strain.

### 4. Obtainment of engineering bacterium YPVal-pflB04

(1) Culture of YPVal-pflB03-Cas9 strain; when the YPVal-pflB03-Cas9 strain grew to OD₆₀₀ₙₘ of 0.1, IPTG was added such that the concentration of IPTG in the system was 0.1 mM, and continued to be cultured to induce λ-Red-mediated homologous recombination; and when the YPVal-pflB03-Cas9 strain grew to OD₆₀₀ₙₘ of 0.6, bacterial cells were collected and competent cells of the YPVal-pflB03-Cas9 strain were prepared.
(2) The pGRB-thiE sgRNA plasmid obtained in step 1 and the ΔthiE-Up-Down fragment obtained in step 2 were transformed into competent cells of the YPVal-pflB03-Cas9 strain, coated on a 2-YT agar plate containing 100 mg/L Spectinomycin and 100 mg/L Ampicillin, and cultured at 32°C to obtain single colonies.
(3) Taking the single colonies obtained in step (2) as a template respectively, a PCR amplification product was obtained by performing PCR amplification using a primer pair composed of primers P15 and P18. If the PCR amplification product obtained from a single colony contained a DNA fragment with a size of 1296 bp, this colony was preliminarily identified as a positive transformant.
(4) The positive transformant obtained in step (3) was inoculated on a 2-YT agar plate containing 100 mg/L Spectinomycin and 0.2% (m/v) arabinose and cultured at 32°C (for eliminating pGRB-thiE sgRNA plasmids); then, colonies that grew on a 2-YT agar plate containing 100 mg/L Spectinomycin but did not grow on a 2-YT agar plate containing 100 mg/L Ampicillin were picked out, then transferred to a 2-YT agar plate and cultured at 42°C (for eliminating plasmids pREDCas9); and finally, colonies which did not grow on a 2-YT agar plate containing 100 mg/L Spectinomycin but on an antibody-free 2-YT agar plate were picked out.
(5) Taking the single colonies obtained in step (4) as a template respectively, a PCR amplification product was obtained by performing PCR amplification using a primer pair composed of primers P15 and P18. If the PCR amplification product obtained from a single colony contained a DNA fragment with a size of 1296 bp, this colony was identified as a positive transformant in which a thiE gene was deleted from a genome of an engineered bacterium YPVal-pflB03. This positive transformant was named as the engineered bacterium YPVal-pflB04.

### IV. Obtainment of engineering bacterium YPVal-pflB05

Taking the engineered bacterium YPVal-pflB04 as an originating bacterium, according to a genome sequence of *Escherichia coli* W3110 published by NCBI, an mdh gene was knocked from a genome of an engineered bacterium YPVal-pflB04 using a CRISPR/Cas9 gene editing technology.

The mdh gene encodes malate dehydrogenase with a Gene ID of 947854 and an amino acid sequence as shown in SEQ ID No. 18.

SEQ ID NO. 18 (malate dehydrogenase mdh)
MKVAVLGAAGGIGQALALLLKTQLPSGSELSLYDIAPVTPGVAVDLSHIPTAVKIKGFSGED
ATPALEGADVVLISAGVARKPGMDRSDLFNVNAGIVKNLVQQVAKTCPKACIGIITNPVNT
TVAIAAEVLKKAGVYDKNKLFGVTTLDIIRSNTFVAELKGKQPGEVEVPVIGGHSGVTILP
LLSQVPGVSFTEQEVADLTKRIQNAGTEVVEAKAGGGSATLSMGQAAARFGLSLVRALQG

EQGVVECAYVEGDGQYARFFSQPLLLGKNGVEERKSIGTLSAFEQNALEGMLDTLKKDIA
LGEEFVNK

The specific steps were as follows:

### 1. Construction of pGRB-mdh sgRNA plasmid

According to the genome sequence of *Escherichia coli* W3110 published by NCBI, an sgRNA target sequence GCCTTTCAGTTCCGCAACAAAGG (SEQ ID No. 11) from which an mdh gene was knocked out was designed by using CRISPR RGEN Tools (http://www.rgenome.net/cas-designer/), and a linearized pGRB vector homologous arm sequence was added to ends 5' and 3' of the target sequence, in order to construct an sgRNA plasmid.
(1) A primer sgRNA-5F: 5'-TGACAGCTAGCTCAGTCCTAGGTATAATACTAGTgcctttcagttccgcaacaaaggGTTTTAGAG CTAGAAATAGCAAGTTAAAATAAGG-3' (as shown in SEQ ID No. 49, underlined as a pGRB vector homologous arm sequence) and a primer sgRNA-5R: 5'-CCTTATTTTAACTTGCTATTTCTAGCTCTAAAACcctttgttgcggaactgaaaggcACTAGTATTAT ACCTAGGACTGAGCTAGCTGTCA-3' (as shown in SEQ ID No. 50, underlined as a pGRB vector homologous arm sequence) were synthesized by Invitrogen.
(2) The primer sgRNA-5F and the primer sgRNA-5R were annealed (a reaction procedure: denaturation at 95°C for 5 min, and annealing at 50°C for 1 min); and then, a target fragment was recovered by a DNA purification kit, measured for a DNA concentration, and diluted in concentration to 100 ng/µL to obtain an annealing product. The annealing product contained an sgRNA-5 fragment with a nucleotide sequence as shown in SEQ ID No. 11.
(3) Approximately 2700 bp of DNA fragments were recovered by a DNA recovery kit (QIAGEN Gel Extraction Kit) by digesting a pGRB vector with a restriction enzyme *Spe*I.

The digestion system was 50 µL and composed of 5 µL of 10×Buffer (owned by the restriction enzyme *Spe*I), 2.5 µL of restriction enzyme *Spe*I, 3000-5000 ng of pGRB vector, and ddH₂O.

The digestion procedure was carried out at 37°C for 3 h.

(4) The DNA fragments recovered in step (3) were dephosphorylated (to prevent self-adhesion of the pGRB vector) and recovered by a DNA recovery kit to obtain a linearized pGRB vector.

The dephosphorylation system was 50 µL and composed of 5 µL of 10×Buffer (owned by CIAP), 1000-2000 ng of DNA fragments recovered in step (3), 2.5 µL of CIAP, and ddH₂O.

The dephosphorylation procedure was carried out at 37°C for 1 h.

(5) The linearized pGRB vector obtained in step (4) and the annealing product obtained in step (2) were recombined by a Gibson Assembly kit, and then transformed into competent cells of *Escherichia coli* DH5α to obtain a pGRB-mdh sgRNA plasmid.

The recombination system was 5 µL and composed of 2 µL of linearized pGRB vector, 0.5 µL of annealing product, and 2.5 µL of assembly enzyme (owned by the Gibson Assembly kit).

The recombination procedure was carried out at 50°C for 30 min.

### 2. Obtainment of Δmdh-Up-Down fragment

(1) According to a genome sequence of *Escherichia coli* W3110 published by NCBI, a primer pair for amplifying an upstream homologous arm and a primer pair for amplifying a downstream homologous arm were designed and synthesized by Invitrogen.

The primer pair for amplifying the upstream homologous arm was composed of a primer P19: 5'-AACTTCCTCCAAACCGATGC-3' (as shown in SEQ ID No. 51) and a primer P20: 5'-CAATATAATAAGGAGTTTAGGTTGATTAGCGGATAATAAAAAACC-3' (as shown in SEQ ID No. 52).

The primer pair for amplifying the downstream homologous arm was composed of a primer P21: 5'-GGTTTTTTATTATCCGCTAATCAACCTAAACTCCTTATTATATTG-3' (as shown in SEQ ID No. 53) and a primer P22: 5'-TCTTCAATGGACTGGAGGTG-3' (as shown in SEQ ID No. 54).

(2) Taking genomic DNA of *Escherichia coli* W3110 as a template, a primer pair composed of primers P19 and P20 was used for PCR amplification of a high-fidelity amplification enzyme KAPA HiFi HotStart, and an upstream homologous arm with a size of 590 bp was recovered by a DNA recovery kit.

(3) Taking genomic DNA of *Escherichia coli* W3110 as a template, a primer pair composed of primers P21 and P22 was used for PCR amplification of a high-fidelity amplification enzyme KAPA HiFi HotStart, and a downstream homologous arm with a size of 708 bp was recovered by a DNA recovery kit.

(4) Taking the upstream homologous arm recovered in step (2) and the downstream homologous arm recovered in step (3), which were mixed, as a template, a primer pair composed of primers P19 and P22 was used for overlap PCR to obtain a Δmdh-Up-Down fragment as shown in SEQ ID No. 12.

SEQ ID No. 12: Δmdh-Up-Down(1253bp)
AACTTCCTCCAAACCGATGCTTCCATTAACCACGGTAACTCTGGCGGCGCGCTGGTGA
ACTCGCTGGGCGAACTGATGGGCATTAATACGCTGTCGTTTGATAAGAGTAACGATGGC
GAAACGCCGGAAGGTATCGGCTTTGCGATTCCTTTCCAGTTAGCAACCAAAATTATGGA
TAAGCTGATCCGCGATGGTCGCGTGATCCGCGGCTACATTGGTATCGGCGGACGTGAGA
TCGCACCACTGCACGCGCAGGGCGGTGGTATAGATCAACTGCAAGGGATCGTGGTTAA
TGAAGTGTCACCTGACGGCCCGGCGGCGAATGCGGGTATTCAGGTCAACGATCTGATT
ATTTCGGTGGATAACAAACCGGCCATCTCTGCTCTGGAGACGATGGATCAGGTGGCGG
AAATTCGCCCTGGTTCGGTGATCCCTGTAGTAGTGATGCGTGATGATAAGCAGTTAACG
CTGCAGGTCACCATTCAGGAATATCCGGCAACCAATTAAGTCGTGCGCTCAAAACAAA

AAACCGGAGTCTGTGCTCCGGTTTTTTATTATCCGCTAATCAACCTAAACTCCTTATTATA
TTGATAAACTAAGATATGTTGCTCCGCTGCCGCGACCTTAATCCACAAAATTGCCGTTTA
CAATGACCACATCTCAAGAATGTGTAGTCACGCAAGTTTAGCGTTTATGCATTTAATTG
CCGTAATCAGGAACCTAACTTACGTAATTAACAGTCGCTTATGCATTAGCGCAACATTCC
AACAGGTGGTGACAATATACCCTACCGTTCAGCCAAAACAACATCAATTTGATAACAAT
TAATTTACTTTTAAGCAGAATTTGCATGCCGTGACGCAGGCATGTTTCTCAATAACGAA
ATTTGATAAAATCCCGCTCTTTCATAACATTATTTCAGCCTTCTTCAGGGCTGACTGTTT
GCATAAAAATTCATCTGTATGCACAATAATGTTGTATCAACCACCATATCGGGTGACTTA
TGCGAAGCTCGGCTAAGCAAGAAGAACTAGTTAAAGCATTTAAAGCATTACTTAAAGA
AGAGAAATTTAGCTCCCAGGGCGAAATCGTCGCCGCGTTGCAGGAGCAAGGCTTTGA
CAATATTAATCAGTCTAAAGTCTCGCGGATGTTGACCAAGTTTGGTGCTGTACGTACAC
GCAATGCCAAAATGGAAATGGTTTACTGCCTGCCAGCTGAACTGGGTGTACCAACCAC
CTCCAGTCCATTGAAGA

### 3. Obtainment of YPVal- pflB04-Cas9 strain

(1) A plasmid pREDCas9 was transformed into competent cells of an engineered bacterium YPVal-pflB04, then coated on a 2-YT agar plate containing 100 mg/L Spectinomycin and cultured at 32°C to obtain Spectinomycin-resistant single colonies.
(2) Taking the single colonies obtained in step (1) as a template respectively, a PCR amplification product was obtained by performing PCR amplification using a primer pair composed of primers pRedCas9-PF and pRedCas9-PR. If the PCR amplification product obtained from a single colony contained a DNA fragment with a nucleotide sequence as shown in SEQ ID No. 3, this colony contained the plasmid pREDCas9. The strain of this colony was named as the YPVal-pflB04-Cas9 strain.

### 4. Obtainment of engineering bacterium YPVal-pflB05

(1) Culture of YPVal-pflB04-Cas9 strain; when the YPVal-pflB04-Cas9 strain grew to OD₆₀₀ₙₘ of 0.1, IPTG was added such that the concentration of IPTG in the system was 0.1 mM, and continued to be cultured to induce λ-Red-mediated homologous recombination; and when the YPVal-pflB04-Cas9 strain grew to OD₆₀₀ₙₘ of 0.6, bacterial cells were collected and competent cells of the YPVal-pflB04-Cas9 strain were prepared.
(2) The pGRB-mdh sgRNA plasmid obtained in step 1 and the Δmdh-Up-Down fragment obtained in step 2 were transformed into competent cells of the YPVal-pflB04-Cas9 strain, coated on a 2-YT agar plate containing 100 mg/L Spectinomycin and 100 mg/L Ampicillin, and cultured at 32°C to obtain single colonies.
(3) Taking the single colonies obtained in step (2) as a template respectively, a PCR amplification product was obtained by performing PCR amplification using a primer pair composed of primers P19 and P22. If the PCR amplification product obtained from a single colony contained a DNA fragment with a size of 1253 bp, this colony was preliminarily identified as a positive transformant.
(4) The positive transformant obtained in step (3) was inoculated on a 2-YT agar plate containing 100 mg/L Spectinomycin and 0.2% (m/v) arabinose and cultured at 32°C (for eliminating pGRB-thiE sgRNA plasmids); then, colonies that grew on a 2-YT agar plate containing 100 mg/L Spectinomycin but did not grow on a 2-YT agar plate containing 100 mg/L Ampicillin were picked out, then transferred to a 2-YT agar plate and cultured at 42°C (for eliminating plasmids pREDCas9); and finally, colonies which did not grow on a 2-YT agar plate containing 100 mg/L Spectinomycin but on an antibody-free 2-YT agar plate were picked out.
(5) Taking the single colonies obtained in step (4) as a template respectively, a PCR amplification product was obtained by performing PCR amplification using a primer pair composed of primers P19 and P22. If the PCR amplification product obtained from a single colony contained a DNA fragment with a size of 1253 bp, this colony was identified as a positive transformant in which an mdh gene was deleted from a genome of an engineered bacterium YPVal-pflB04. This positive transformant was named as the engineered bacterium YPVal-pflB05.

### Example 2: Modification I of engineered bacteria originating from Escherichia coli W3110

After a large number of experiments, the inventors of the present invention obtained engineered bacteria YPVal-pflB06, YPVal-pflB07, YPVal-pflB08, YPVal-pflB09 and YPVal-pflB10 by means of modification originating from *Escherichia coli* W3110. Genotypes of the engineering bacteria YPVal-pflB06, YPVal-pflB07, YPVal-pflB08, YPVal-pflB09 and YPVal-pflB10 were shown in Table 2.

**Table 2**

| Strains | Genotypes |
|---|---|
| YPVal-pflB06 | W3110-ΔpflB |
| YPVal-pflB07 | W3110-ΔpflB-ΔadhE |
| YPVal-pflB08 | W3110-ΔpflB-ΔadhE-ΔilvE(E)-ptrcilvE(B) |
| YPVal-pflB09 | W3110-ΔpflB-ΔadhE-ΔilvE(E)-ptrcilvE(B)-ΔthiE |
| YPVal-pflB10 | W3110-ΔpflB-ΔadhE-ΔilvE(E)-ptrcilvE(B)-ΔthiE-Δmdh |

### I. Obtainment of engineering bacterium YPVal-pflB06

### 1. Construction of pGRB-pflB sgRNA plasmid

The same as 1 in step I in Example 1.

### 2. Obtainment of ΔpflB-Up-Down fragment

The same as 2 in step I in Example 1.

### 3. Obtainment of W3110-Cas9 strain

According to 3 in step I in Example 1, competent cells of a valine-producing bacterium CGMCC 22721 were replaced with competent cells of *Escherichia coli* W3110, and the other steps were unchanged, obtaining the W3110-Cas9 strain.

### 4. Obtainment of engineering bacterium YPVal-pflB06

According to 4 in step I in Example **1,** a CGMCC 22721-Cas9 strain was replaced with a W3110-Cas9 strain, and the other steps were unchanged, obtaining the engineered bacterium YPVal-pflB06.

### II. Obtainment of engineering bacterium YPVal-pflB07

### 1. Construction of pGRB-adhE sgRNA plasmid

The same as 1 in step II in Example 1.

### 2. Obtainment of ΔadhE-Up-Down fragment

The same as 2 in step II in Example 1.

### 3. Obtainment of YPVal- pflB06-Cas9 strain

According to 3 in step II in Example 1, competent cells of an engineered bacterium YPVal-pflB01 were replaced with competent cells of an engineered bacterium YPVal-pflB06, and the other steps were unchanged, obtaining the YPVal-pflB06-Cas9 strain.

### 4. Obtainment of engineering bacterium YPVal-pflB07

According to 4 in step II in Example 1, a YPVal-pflB01-Cas9 strain was replaced with a YPVal-pflB06-Cas9 strain, and the other steps were unchanged, obtaining the engineered bacterium YPVal-pflB07.

### III. Obtainment of engineering bacterium YPVal-pflB08

### 1. Construction of pGRB-ilvE sgRNA plasmid

The same as 1 in step III in Example 1.

### 2. Obtainment of ptrc-ilvE(B)-Up-Down fragment

The same as 2 in step III in Example 1.

### 3. Obtainment of YPVal- pflB07-Cas9 strain

According to 3 in step III in Example 1, competent cells of an engineered bacterium YPVal-pflB02 were replaced with competent cells of an engineered bacterium YPVal-pflB07, and the other steps were unchanged, obtaining the YPVal-pflB07-Cas9 strain.

### 4. Obtainment of engineering bacterium YPVal-pflB08

According to 4 in step III in Example 1, a YPVal-pflB02-Cas9 strain was replaced with a YPVal-pflB07-Cas9 strain, and the other steps were unchanged, obtaining the engineered bacterium YPVal-pflB08.

### IV. Obtainment of engineering bacterium YPVal-pflB09

### 1. Construction of pGRB-thiE sgRNA plasmid

The same as 1 in step IV in Example 1.

### 2. Obtainment of ΔthiE-Up-Down fragment

The same as 2 in step IV in Example 1.

### 3. Obtainment of YPVal- pflB08-Cas9 strain

According to 3 in step IV in Example 1, competent cells of an engineered bacterium YPVal-pflB03 were replaced with competent cells of an engineered bacterium YPVal-pflB08, and the other steps were unchanged, obtaining the YPVal-pflB08-Cas9 strain.

### 4. Obtainment of engineering bacterium YPVal-pflB09

According to 4 in step IV in Example 1, a YPVal-pflB03-Cas9 strain was replaced with a YPVal-pflB08-Cas9 strain, and the other steps were unchanged, obtaining the engineered bacterium YPVal-pflB09.

### V. Obtainment of engineering bacterium YPVal-pflB10

### 1. Construction of pGRB-mdh sgRNA plasmid

The same as 1 in step V in Example 1.

### 2. Obtainment of Δmdh-Up-Down fragment

The same as 2 in step V in Example 1.

### 3. Obtainment of YPVal- pflB09-Cas9 strain

According to 3 in step V in Example 1, competent cells of an engineered bacterium YPVal-pflB04 were replaced with competent cells of an engineered bacterium YPVal-pflB09, and the other steps were unchanged, obtaining the YPVal-pflB09-Cas9 strain.

### 4. Obtainment of engineering bacterium YPVal-pflB10

According to 4 in step V in Example 1, a YPVal-pflB04-Cas9 strain was replaced with a YPVal-pflB09-Cas9 strain, and the other steps were unchanged, obtaining the engineered bacterium YPVal-pflB10.

### Example 3: Production of L-valine from engineered bacteria modified in Example 1 and Example 2 by means of fermentation

1. The engineered bacteria YPVal-pflB01, YPVal-pflB02, YPVal-pflB03, YPVal-pflB04, YPVal-pflB05, YPVal-pflB06, YPVal-pflB07, YPVal-pflB08, YPVal-pflB09 and YPVal-pflB10 modified in Example 1 and Example 2, as well as a valine-producing bacterium CGMCC 22721 and *Escherichia coli* W3110 as originating bacteria were respectively fermented in a fermentation tank (Shanghai Bailun Biotechnology Co., Ltd., a model of BLBIO-5GC-4-H) to obtain fermentation broth.

### Each strain was repeated three times

The composition of a fermentation medium during fermentation was shown in Table 3.

The fermentation control process was shown in Table 4.

**Table 3**

| Components | Concentration |
|---|---|
| Ammonium sulfate | 14 g/L |
| Potassium dihydrogen phosphate | 1 g/L |
| Dipotassium hydrogen phosphate | 1 g/L |
| Magnesium sulfate | 0.5 g/L |
| Yeast powder | 2 g/L |
| Ferrous sulfate | 18 mg/L |
| Manganese sulfate | 4.2 mg/L |
| Biotin | 0.02 mg/L |
| Vitamin B1 | 2 mg/L |
| Antifoam (CB-442) | 0.5 mL/L |
| 70% glucose (base sugar) | 40 g/L |
| Water | - |

**Table 4**

| | |
|---|---|
| Corrected to DO100% | Temperature of 37°C, air volume of 1 L/min, speed of 400 rpm, tank pressure of 0.01 Mpa, and calibration after 5 min |
| Inoculation amount | 3.5% |
| Culture temperature °C | 33°C |
| pH | pH7.0±0.05 |
| Dissolved oxygen DO | 10-20% |
| Initial conditions | Temperature of 33°C, pH of 7.0, tank pressure of 0 Mpa, air volume of 0.1 L/min, and speed of 400 rpm |
| Whole-process control | Temperature of 33°C, pH of 7.0, tank pressure of 0 Mpa, air volume of 0.2 L/min, and speed of 400 rpm |
| Residual sugar control | 0.1-0.2% before F12h; ≤ 0.02% after F12h in combination with DO requirements |
| Culture maturity standards | OD₆₁₀ of 30-35; ventilation was stopped and static for 2 h after OD₆₁₀>30 (according to the purpose of batch experiments, bacterial cell isolation or continuous catalysis was carried out) |
| Material fed-batch | ammonia water, 70% of concentrated sugar, and 5% of Paodi |
| Fermentation cycle | About 18-20 h |

### 2. The yield of L-valine in the fermentation broth was detected by high-performance liquid chromatography

The detection results of a valine-producing bacterium CGMCC 22721 and the engineered bacteria YPVal-pflB01, YPVal-pflB02, YPVal-pflB03, YPVal-pflB04 and YPVal-pflB05 obtained from a valine-producing bacterium CGMCC 22721 by means of three fermentations were shown in Table 5 (P-value<0.01 indicated a very significant difference). The results showed that compared with the valine-producing bacterium CGMCC 22721, YPVal-pflB01, YPVal-pflB02, YPVal-pflB03, YPVal-pflB04 and YPVal-pflB05 could significantly increase the yield of L-valine. That is, all engineered bacteria obtained by "knocking out a pflB gene", "knocking out a pflB gene and an adhE gene", "knocking out a pflB gene and an adhE gene and inserting ilvE(B) from *Bacillus subtilis* initiated by ptrc while knocking out an ilvE(E) gene", "knocking out a pflB gene, an adhE gene and a thiE gene and inserting ilvE(B) from *Bacillus subtilis* initiated by ptrc while knocking out an ilvE(E) gene" or "knocking out a pflB gene, an adhE gene, a thiE gene and an mdh gene and inserting ilvE (B) from *Bacillus subtilis* initiated by ptrc while knocking out an ilvE(E) gene" could all increase the yield of L-valine in the valine-producing bacterium CGMCC 22721.

**Table 5**

| Strains | L-valine concentration-1 (g/L) | L-valine concentration-2 (g/L) | L-valine concentration-3 (g/L) | Mean (g/L) | P-value |
|---|---|---|---|---|---|
| Valine-producing bacterium CGMCC 22721 | 78.10 | 77.88 | 78.23 | 78.07±0.18 | |
| YPVal-pflB01 | 79.98 | 80.36 | 80.18 | 80.17±0.19 | P<0.01 |
| YPVal-pflB02 | 81.79 | 81.96 | 81.98 | 81.91±0.10 | P<0.01 |
| YPVal-pflB03 | 83.69 | 83.47 | 83.58 | 83.58±0.11 | P<0.01 |
| YPVal-pflB04 | 86.10 | 86.16 | 86.35 | 86.20±0.13 | P<0.01 |
| YPVal-pflB05 | 88.02 | 87.9 | 88.06 | 87.99±0.08 | P<0.01 |

The detection results of *Escherichia coli* W3110 and engineered bacteria YPVal-pflB06, YPVal-pflB07, YPVal-pflB08, YPVal-pflB09 and YPVal-pflB10 obtained from *Escherichia coli* W3110 by means of three fermentations were shown in Table 6 (P-value<0.01 indicated a very significant difference). The results showed that compared with *Escherichia coli* W3110, YPVal-pflB06, YPVal-pflB07, YPVal-pflB08, YPVal-pflB09 and YPVal-pflB10 could significantly increase the yield of L-valine. That is, all engineered bacteria obtained by "knocking out a pflB gene", "knocking out a pflB gene and an adhE gene", "knocking out a pflB gene and an adhE gene and inserting ilvE(B) from *Bacillus subtilis* initiated by ptrc while knocking out an ilvE(E) gene", "knocking out a pflB gene, an adhE gene and a thiE gene and inserting ilvE(B) from *Bacillus subtilis* initiated by ptrc while knocking out an ilvE(E) gene" or "knocking out a pflB gene, an adhE gene, a thiE gene and an mdh gene and inserting ilvE (B) from *Bacillus subtilis* initiated by ptrc while knocking out an ilvE(E) gene" could increase the yield of L-valine in the *Escherichia coli* W3110.

**Table 6**

| Strains | L-valine concentration-1 (g/L) | L-valine concentration-2 (g/L) | L-valine concentration-3 (g/L) | Mean (g/L) | P-value |
|---|---|---|---|---|---|
| *Escherichia coli* W3110 | 0.009 | 0.005 | 0.007 | 0.007±0.002 | |
| YPVal-pflB06 | 0.145 | 0.156 | 0.150 | 0.150±0.006 | P<0.01 |
| YPVal-pflB07 | 0.238 | 0.254 | 0.269 | 0.254±0.016 | P<0.01 |
| YPVal-pflB08 | 0.319 | 0.330 | 0.310 | 0.320±0.010 | P<0.01 |
| YPVal-pflB09 | 0.458 | 0.432 | 0.420 | 0.437±0.019 | P<0.01 |
| YPVal-pflB10 | 0.573 | 0.543 | 0.569 | 0.562±0.016 | P<0.01 |

### Example 4: Modification II of engineered bacteria originating from valine-producing bacterium CGMCC 22721

After a large number of experiments, the inventors of the present invention obtained engineered bacteria YPVal-adhE01, YPVal-adhE02, YPVal-adhE03 and YPVal-adhE04 by means of modification originating from a valine-producing bacterium CGMCC 22721. Genotypes of the engineered bacteria YPVal-adhE01, YPVal-adhE02, YPVal-adhE03 and YPVal-adhE04 were shown in Table 7.

**Table 7**

| Strains | Genotypes |
|---|---|
| YPVal-adhE01 | CGMCC 22721-ΔadhE |
| YPVal-adhE02 | CGMCC 22721-ΔadhE-ΔilvE(E)-ptrcilvE(B) |
| YPVal-adhE03 | CGMCC 22721-ΔadhE-ΔilvE(E)-ptrcilvE(B)-ΔthiE |
| YPVal-adhE04 | CGMCC 22721-ΔadhE-ΔilvE(E)-ptrcilvE(B)-ΔthiE-Δmdh |

### I. Obtainment of engineering bacterium YPVal-adhE01

According to a genome sequence of *Escherichia coli* W3110 published by NCBI, an adhE gene was knocked from a genome of a valine-producing bacterium CGMCC 22721 using a CRISPR/Cas9 gene editing technology.

The adhE gene encodes alcohol dehydrogenase with a Gene ID of 945837 and an amino acid sequence as shown in SEQ ID No. 14.

The specific steps were as follows:

### 1. Construction of pGRB-adhE sgRNA plasmid

According to the genome sequence of *Escherichia coli* W3110 published by NCBI, an sgRNA target sequence (SEQ ID No. 4) from which an adhE gene was knocked out was designed by using CRISPR RGEN Tools (http://www.rgenome.net/cas-designer/), and a linearized pGRB vector homologous arm sequence was added to ends 5' and 3' of the target sequence, in order to construct an sgRNA plasmid.
(1) A primer sgRNA-2F: 5'-TGACAGCTAGCTCAGTCCTAGGTATAATACTAGTaagaaccacaacccagagtcaggGTTTTAGA GCTAGAAATAGCAAGTTAAAATAAGG-3' (as shown in SEQ ID No. 29, underlined as a pGRB vector homologous arm sequence), a primer sgRNA-2R: 5'-CCTTATTTTAACTTGCTATTTCTAGCTCTAAAACcctgactctgggttgtggttcttACTAGTATTATA CCTAGGACTGAGCTAGCTGTCA-3' (as shown in SEQ ID No. 30), underlined as a pGRB vector homologous arm sequence), a primer sgRNA-PF: 5'-GTCTCATGAGCGGATACATATTTG-3'(SEQ ID No. 21) and a primer sgRNA-PR: 5'-ATGAGAAAGCGCCACGCT-3'(SEQ ID No. 22) were synthesized by Invitrogen.
(2) The primer sgRNA-2F and the primer sgRNA-2R were annealed (a reaction procedure: denaturation at 95°C for 5 min, and annealing at 50°C for 1 min); and then, a target fragment was recovered by a DNA purification kit, measured for a DNA concentration, and diluted in concentration to 100 ng/µL to obtain an annealing product. The annealing product contained an sgRNA-2 fragment with a nucleotide sequence as shown in SEQ ID No. 4.
(3) Approximately 2700 bp of DNA fragments were recovered by a DNA recovery kit (QIAGEN Gel Extraction Kit) by digesting a pGRB vector with a restriction enzyme *Spe*I.

The digestion system was 50 µL and composed of 5 µL of 10×Buffer (owned by the restriction enzyme *Spe*I), 2.5 µL of restriction enzyme *Spe*I, 3000-5000 ng of pGRB vector, and ddH₂O.

The digestion procedure was carried out at 37°C for 3 h.

(4) The DNA fragments recovered in step (3) were dephosphorylated (to prevent self-adhesion of the pGRB vector) and recovered by a DNA recovery kit to obtain a linearized pGRB vector.

The dephosphorylation system was 50 µL and composed of 5 µL of 10×Buffer (owned by CIAP), 1000-2000 ng of DNA fragments recovered in step (3), 2.5 µL of CIAP, and ddH₂O.

The dephosphorylation procedure was carried out at 37°C for 1 h.

(5) The linearized pGRB vector obtained in step (4) and the annealing product obtained in step (2) were recombined by a Gibson Assembly kit, and then transformed into competent cells of *Escherichia coli* DH5α to obtain a pGRB-adhE sgRNA plasmid.

The recombination system was 5 µL and composed of 2 µL of linearized pGRB vector, 0.5 µL of annealing product, and 2.5 µL of assembly enzyme (owned by the Gibson Assembly kit).

The recombination procedure was carried out at 50°C for 30 min.

### 2. Obtainment of ΔadhE-Up-Down fragment

(1) According to a genome sequence of *Escherichia coli* W3110 published by NCBI, a primer pair for amplifying an upstream homologous arm and a primer pair for amplifying a downstream homologous arm were designed and synthesized by Invitrogen.

The primer pair for amplifying the upstream homologous arm was composed of a primer P5: 5'-GTGCCAGTCATCCTTCAGGT-3' (as shown in SEQ ID No. 31) and a primer P6: 5'-CGTTCCGACCACTAACCCGACTTGGGTATTCCGAAATCTATCC-3' (as shown in SEQ ID No. 32).

The primer pair for amplifying the downstream homologous arm was composed of a primer P7: 5'-GGATAGATTTCGGAATACCCAAGTCGGGTTAGTGGTCGGAACG-3' (as shown in SEQ ID No. 33) and a primer P8: 5'-AAGCGATGCTGAAAGGTGTC-3' (as shown in SEQ ID No. 34).

(2) Taking genomic DNA of *Escherichia coli* W3110 as a template, a primer pair composed of primers P5 and P6 was used for PCR amplification of a high-fidelity amplification enzyme KAPA HiFi HotStart, and an upstream homologous arm with a size of 765 bp was recovered by a DNA recovery kit.

(3) Taking genomic DNA of *Escherichia coli* W3110 as a template, a primer pair composed of primers P7 and P8 was used for PCR amplification of a high-fidelity amplification enzyme KAPA HiFi HotStart, and a downstream homologous arm with a size of 643 bp was recovered by a DNA recovery kit.

(4) Taking the upstream homologous arm recovered in step (2) and the downstream homologous arm recovered in step (3), which were mixed, as a template, a primer pair composed of primers P5 and P8 was used for overlap PCR to obtain a ΔadhE-Up-Down fragment as shown in SEQ ID No. 5.

### 3. Obtainment of CGMCC 22721-Cas9 strain

(1) A plasmid pREDCas9 (Addgene, catalog number 71541; containing a Spectinomycin-resistant gene) was transformed into competent cells of a valine-producing bacterium CGMCC 22721, then coated on a 2-YT agar plate containing 100 mg/L Spectinomycin and cultured at 32°C to obtain Spectinomycin-resistant single colonies.

A preparation method for the 2-YT agar plate was as follows: dissolving 16 g of tryptone, 10 g of yeast extract powder, 5 g of sodium chloride and 16 g of agar powder in an appropriate amount of water, then metering a volume with water to 1 L, adjusting a pH value to 7.0 with sodium hydroxide, and sterilizing at 121°C for 20 min.

(2) Taking the single colonies obtained in step (1) as a template respectively, a PCR amplification product was obtained by performing PCR amplification using a primer pair composed of a primer pRedCas9-PF: 5'-GCAGTGGCGGTTTTCATG-3' (SEQ ID No. 27) and a primer pRedCas9-PR: 5'-CCTTGGTGATCTCGCCTTTC-3' (SEQ ID No. 28). If the PCR amplification product obtained from a single colony contained a DNA fragment with a nucleotide sequence as shown in SEQ ID No. 3, this colony contained the plasmid pREDCas9. The strain of this colony was named as the CGMCC 22721-Cas9 strain.

### 4. Obtainment of engineering bacterium YPVal-adhE01

(1) Culture of CGMCC 22721-Cas9 strain; when the strain CGMCC 22721-Cas9 grew to OD₆₀₀ₙₘ of 0.1, IPTG was added such that the concentration of IPTG in the system was 0.1 mM, and continued to be cultured to induce λ-Red-mediated homologous recombination; and when the strain CGMCC 22721-Cas9 grew to OD₆₀₀ₙₘ of 0.6, bacterial cells were collected and competent cells of CGMCC 22721-Cas9 were prepared.
(2) The pGRB-adhE sgRNA plasmid obtained in step 1 and the ΔadhE-Up-Down fragment obtained in step 2 were transformed into competent cells of CGMCC 22721-Cas9, coated on a 2-YT agar plate containing 100 mg/L Spectinomycin and 100 mg/L Ampicillin, and cultured at 32°C to obtain single colonies.
(3) Taking the single colonies obtained in step (2) as a template respectively, a PCR amplification product was obtained by performing PCR amplification using a primer pair composed of primers P5 and P8. If the PCR amplification product obtained from a single colony contained a DNA fragment with a size of 1365 bp, this colony was preliminarily identified as a positive transformant.
(4) The positive transformant obtained in step (3) was inoculated on a 2-YT agar plate containing 100 mg/L Spectinomycin and 0.2% (m/v) arabinose and cultured at 32°C (for eliminating pGRB-adhE sgRNA plasmids); then, colonies that grew on a 2-YT agar plate containing 100 mg/L Spectinomycin but did not grow on a 2-YT agar plate containing 100 mg/L Ampicillin were picked out, then transferred to a 2-YT agar plate and cultured at 42°C (for eliminating plasmids pREDCas9); and finally, colonies which did not grow on a 2-YT agar plate containing 100 mg/L Spectinomycin but on an antibody-free 2-YT agar plate were picked out.
(5) Taking the single colonies obtained in step (4) as a template respectively, a PCR amplification product was obtained by performing PCR amplification using a primer pair composed of primers P5 and P8. If the PCR amplification product obtained from a single colony contained a DNA fragment with a size of 1365 bp, this colony was identified as a positive transformant. The positive transformant in which an adhE gene was knocked from a genome of a valine-producing bacterium CGMCC 22721 was named as the engineered bacterium YPVal-adhE01.

### II. Obtainment of engineering bacterium YPVal-adhE02

According to a genome sequence of *Escherichia coli* W3110 and a genome sequence of *Bacillus subtilis subsp. subtilis str.168* published by NCBI, an engineered bacterium YPVal-adhE02 (hereinafter referred to as YPVal-adhE02) was obtained by knocking in an ilvE gene (i.e., ptrc-ilvE(B) sequence) of *Bacillus subtilis* initiated by a ptrc promoter while knocking out an ilvE(E) gene from a genome of an engineered bacterium YPVal-adhE01 obtained in step I by a CRISPR/Cas9 gene editing technology. A nucleotide sequence of the ptrc-ilvE(B) sequence was as shown in SEQ ID No. 6, in which positions 1096-1169 were a nucleotide sequence of the ptrc promoter from end 5', and positions 1-1095 were an ilvE(B) gene of *Bacillus subtilis.*

The ilvE(E) gene of the engineering bacterium YPVal-adhE01 encodes branched-chain amino acid transaminase, with Gene ID of 948278 and an amino acid sequence as shown in SEQ ID No. 15. The ilvE(B) gene of *Bacillus subtilis* encodes branched-chain amino acid transaminase, with Gene ID of 938420 and an amino acid sequence as shown in SEQ ID No. 16.

The specific steps were as follows:

### 1. Construction of pGRB-ilvE sgRNA plasmid

(1) A primer sgRNA-3F: 5'-TGACAGCTAGCTCAGTCCTAGGTATAATACTAGTgaaagcagcgataatcacgtcgGTTTTAGAG CTAGAAATAGCAAGTTAAAATAAGG-3' (as shown in SEQ ID No. 35, underlined as a pGRB vector homologous arm sequence) and a primer sgRNA-3R: 5'-CCTTATTTTAACTTGCTATTTCTAGCTCTAAAACccgacgtgattatcgctgctttcACTAGTATTATA CCTAGGACTGAGCTAGCTGTCA-3' (as shown in SEQ ID No. 36, underlined as a pGRB vector homologous arm sequence) were synthesized by Invitrogen.
(2) The primer sgRNA-3F and the primer sgRNA-3R were annealed (a reaction procedure: denaturation at 95°C for 5 min, and annealing at 50°C for 1 min); and then, a target fragment was recovered by a DNA purification kit, measured for a DNA concentration, and diluted in concentration to 100 ng/µL to obtain an annealing product. The annealing product contained an sgRNA-3 fragment with a nucleotide sequence as shown in SEQ ID No. 7.
(3) Approximately 2700 bp of DNA fragments were recovered by a DNA recovery kit (QIAGEN Gel Extraction Kit) by digesting a pGRB vector (Addgene's product, catalog number 71539) with a restriction enzyme *SpeI* (Takara's product, catalog number 1631).

The digestion system was 50 µL and composed of 5 µL of 10×Buffer (owned by the restriction enzyme *Spe*I), 2.5 µL of restriction enzyme *Spe*I, 3000-5000 ng of pGRB vector, and ddH₂O.

The digestion procedure was carried out at 37°C for 3 h.

(4) The DNA fragments recovered in step (3) were dephosphorylated (to prevent self-adhesion of the pGRB vector) and recovered by a DNA recovery kit to obtain a linearized pGRB vector.

The dephosphorylation system was 50 µL and composed of 5 µL of 10×Buffer (owned by CIAP), 1000-2000 ng of DNA fragments recovered in step (3), 2.5 µL of CIAP (Takara's product, catalog number 2250A), and ddH₂O.

The dephosphorylation procedure was carried out at 37°C for 1 h.

(5) The linearized pGRB vector obtained in step (4) and the annealing product obtained in step (2) were recombined by a Gibson Assembly kit (New England), and then transformed into competent cells (TAKARA) of *Escherichia coli* DH5α to obtain a pGRB-ilvE sgRNA plasmid.

The recombination system was 5 µL and composed of 2 µL of linearized pGRB vector, 0.5 µL of annealing product, and 2.5 µL of assembly enzyme (owned by the Gibson Assembly kit).

The recombination procedure was carried out at 50°C for 30 min.

### 2. Obtainment of ptrc-ilvE(B)-Up-Down fragment

(1) According to a genome sequence of *Escherichia coli* W3110 published by NCBI, a primer pair for amplifying an upstream homologous arm and a primer pair for amplifying a downstream homologous arm were designed and synthesized by Invitrogen. According to the genome sequence of *Bacillus subtilis subsp. subtilis str.168* published by NCBI, a primer pair was designed and synthesized by Invitrogen for amplifying ptrc-ilvE(B).

The primer pair for amplifying the upstream homologous arm was composed of a primer P9: 5'-CAGGCAGTTCATTGAGTTAGCG-3' (as shown in SEQ ID No. 37) and a primer P10: 5'-CACAGTGTATTAAGCAGACGTTAAATACAAAAAATGGGACGGCAC-3' (as shown in SEQ ID No. 38).

The primer pair for amplifying the downstream homologous arm was composed of a primer P13: 5'-GTTATCCGCTCAATTCCACACACATTATACGAGCCGGATGATTATGTCAATTGTCAATT TTATTTTTTGCGCTC-3' (as shown in SEQ ID No. 39, underlined as a partial sequence of the ptrc promoter) and a primer P14: 5'-ACGGTTAGGGATGGTTCGAC-3' (SEQ ID No. 40).

The primer pair for amplifying ptrc-ilvE(B) was composed of a primer P11: 5'-GTGCCGTCCCATTTTTTGTATTTAACGTCTGCTTAATACACTGTG-3' (as shown in SEQ ID No. 41) and a primer P12: 5'-GTATAATGTGTGGAATTGTGAGCGGATAACAATTTCACACAGGAAACAGACCATGGA ACTTTTTAAATATATGGAG-3' (SEQ ID No. 42, underlined as a partial sequence of the ptrc promoter).

(2) Taking genomic DNA of *Escherichia coli* W3110 as a template, a primer pair composed of primers P9 and P10 was used for PCR amplification of a high-fidelity amplification enzyme KAPA HiFi HotStart, and an upstream homologous arm with a size of 709 bp was recovered by a DNA recovery kit.

(3) Taking genomic DNA of *Escherichia coli* W3110 as a template, a primer pair composed of primers P13 and P14 was used for PCR amplification of a high-fidelity amplification enzyme KAPA HiFi HotStart, and a downstream homologous arm with a size of 611 bp was recovered by a DNA recovery kit.

(4) Taking genomic DNA of *Bacillus subtilis subsp.subtilis str.168* as a template, a primer pair composed of primers P11 and P12 was used for PCR amplification of a high-fidelity amplification enzyme KAPA HiFi HotStart, and an ilvE(B) fragment with a size of 1168 bp was recovered by a DNA recovery kit.

(6)Taking the upstream homologous arm recovered in step (2), the downstream homologous arm recovered in step (3) and the ilvE(B) fragment recovered in step (4), which were mixed, as a template, a primer pair composed of primers P9 and P14 was used for overlap PCR to obtain a ptrc-ilvE(B)-Up-Down fragment as shown in SEQ ID No. 8.

### 3. Obtainment of YPVal- adhE01-Cas9 strain

(1) A plasmid pREDCas9 was transformed into competent cells of an engineered bacterium YPVal-adhE01, then coated on a 2-YT agar plate containing 100 mg/L Spectinomycin and cultured at 32°C to obtain Spectinomycin-resistant single colonies.
(2) Taking the single colonies obtained in step (1) as a template respectively, a PCR amplification product was obtained by performing PCR amplification using a primer pair composed of primers pRedCas9-PF and pRedCas9-PR. If the PCR amplification product obtained from a single colony contained a DNA fragment with a nucleotide sequence as shown in SEQ ID No. 3, this colony contained the plasmid pREDCas9. The strain of this colony was named as the YPVal-adhE01-Cas9 strain.

### 4. Obtainment of engineering bacterium YPVal-adhE02

(1) Culture of YPVal-adhE01-Cas9 strain; when the YPVal-adhE01-Cas9 strain grew to OD₆₀₀ₙₘ of 0.1, IPTG was added such that the concentration of IPTG in the system was 0.1 mM, and continued to be cultured to induce λ-Red-mediated homologous recombination; and when theYPVal-adhE01-Cas9 strain grew to OD₆₀₀ₙₘ of 0.6, bacterial cells were collected and competent cells of the YPVal-adhE01-Cas9 strain were prepared.
(2) The pGRB-ilvE sgRNA plasmid obtained in step 1 and the ptrc-ilvE(B)-Up-Down fragment obtained in step 2 were transformed into competent cells of the YPVal-adhE01-Cas9 strain, coated on a 2-YT agar plate containing 100 mg/L Spectinomycin and 100 mg/L Ampicillin, and cultured at 32°C to obtain single colonies.
(3) Taking the single colonies obtained in step (2) as a template respectively, a PCR amplification product was obtained by performing PCR amplification using a primer pair composed of primers P11 and P12. If the PCR amplification product obtained from a single colony contained a DNA fragment with a size of 1168 bp, this colony was preliminarily identified as a positive transformant.
(4) The positive transformant obtained in step (3) was inoculated on a 2-YT agar plate containing 100 mg/L Spectinomycin and 0.2% (m/v) arabinose and cultured at 32°C (for eliminating pGRB-ilvE sgRNA plasmids); then, colonies that grew on a 2-YT agar plate containing 100 mg/L Spectinomycin but did not grow on a 2-YT agar plate containing 100 mg/L Ampicillin were picked out, then transferred to a 2-YT agar plate and cultured at 42°C (for eliminating plasmids pREDCas9); and finally, colonies which did not grow on a 2-YT agar plate containing 100 mg/L Spectinomycin but on an antibody-free 2-YT agar plate were picked out.
(5) Taking the single colonies obtained in step (4) as a template respectively, a PCR amplification product was obtained by performing PCR amplification using a primer pair composed of primers P11 and P12. If the PCR amplification product obtained from a single colony contained a DNA fragment with a size of 1168 bp, this colony was identified as a positive transformant. The positive transformant in which an ilvE(B) gene (i.e., a ptrc-ilvE(B) sequence) of *Bacillus subtilis* initiated by a ptrc promoter was knocked in while the ilvE(E) gene was knocked from a genome of the engineered bacterium YPVal-adhE01 was named as YPVal-adhE02.

### III. Obtainment of engineering bacterium YPVal-adhE03

Taking the engineered bacterium YPVal-adhE02 as an originating bacterium, according to a genome sequence of *Escherichia coli* W3110 published by NCBI, a thiE gene is knocked from the engineered bacterium YPVal-adhE02 using a CRISPR/Cas9 gene editing technology.

The thiE gene encodes thiamine phosphate synthase with a Gene ID of 948491 and an amino acid sequence as shown in SEQ ID No. 17.

The specific steps were as follows:

### 1. Construction of pGRB-thiE sgRNA plasmid

According to the genome sequence of *Escherichia coli* W3110 published by NCBI, an sgRNA target sequence (SEQ ID No. 9) from which a thiE gene was knocked out was designed by using CRISPR RGEN Tools (http://www.rgenome.net/cas-designer/), and a linearized pGRB vector homologous arm sequence was added to ends 5' and 3' of the target sequence, in order to construct an sgRNA plasmid.
(1) A primer sgRNA-4F: 5'-TGACAGCTAGCTCAGTCCTAGGTATAATACTAGTcgcccctcttatatcgcgctggGTTTTAGAG CTAGAAATAGCAAGTTAAAATAAGG-3' (as shown in SEQ ID No. 43, underlined as a pGRB vector homologous arm sequence) and a primer sgRNA-4R: 5'-CCTTATTTTAACTTGCTATTTCTAGCTCTAAAACcccagcgcgatataagaggggcgACTAGTATTA TACCTAGGACTGAGCTAGCTGTCA-3' (as shown in SEQ ID No. 44, underlined as a pGRB vector homologous arm sequence) were synthesized by Invitrogen.
(2) The primer sgRNA-4F and the primer sgRNA-4R were annealed (a reaction procedure: denaturation at 95°C for 5 min, and annealing at 50°C for 1 min); and then, a target fragment was recovered by a DNA purification kit, measured for a DNA concentration, and diluted in concentration to 100 ng/µL to obtain an annealing product. The annealing product contained an sgRNA-4 fragment with a nucleotide sequence as shown in SEQ ID No. 9.
(3) Approximately 2700 bp of DNA fragments were recovered by a DNA recovery kit (QIAGEN Gel Extraction Kit) by digesting a pGRB vector with a restriction enzyme *Spe*I*.*

The digestion system was 50 µL and composed of 5 µL of 10×Buffer (owned by the restriction enzyme *Spe*I), 2.5 µL of restriction enzyme *Spe*I, 3000-5000 ng of pGRB vector, and ddH₂O.

The digestion procedure was carried out at 37°C for 3 h.

(4) The DNA fragments recovered in step (3) were dephosphorylated (to prevent self-adhesion of the pGRB vector) and recovered by a DNA recovery kit to obtain a linearized pGRB vector.

The dephosphorylation system was 50 µL and composed of 5 µL of 10×Buffer (owned by CIAP), 1000-2000 ng of DNA fragments recovered in step (3), 2.5 µL of CIAP, and ddH₂O.

The dephosphorylation procedure was carried out at 37°C for 1 h.

(5) The linearized pGRB vector obtained in step (4) and the annealing product obtained in step (2) were recombined by a Gibson Assembly kit, and then transformed into competent cells of *Escherichia coli* DH5α to obtain a pGRB-thiE sgRNA plasmid.

The recombination system was 5 µL and composed of 2 µL of linearized pGRB vector, 0.5 µL of annealing product, and 2.5 µL of assembly enzyme (owned by the Gibson Assembly kit).

The recombination procedure was carried out at 50°C for 30 min.

### 2. Obtainment of ΔthiE-Up-Down fragment

(1) According to a genome sequence of *Escherichia coli* W3110 published by NCBI, a primer pair for amplifying an upstream homologous arm and a primer pair for amplifying a downstream homologous arm were designed and synthesized by Invitrogen.

The primer pair for amplifying the upstream homologous arm was composed of a primer P15: 5'-TTCTATTCAGGACGCCAACG-3' (as shown in SEQ ID No. 45) and a primer P16: 5'-GCTATAACGCATAAAGTCACGGCACGCTTCCTCCTTACGCAGG-3' (as shown in SEQ ID No. 46).

The primer pair for amplifying the downstream homologous arm was composed of a primer P17: 5'-CCTGCGTAAGGAGGAAGCGTGCCGTGACTTTATGCGTTATAGC-3' (as shown in SEQ ID No. 47) and a primer P18: 5'-GCCTGCAAAGTGCCCATAACCC-3' (as shown in SEQ ID No. 48).

(2) Taking genomic DNA of *Escherichia coli* W3110 as a template, a primer pair composed of primers P15 and P16 was used for PCR amplification of a high-fidelity amplification enzyme KAPA HiFi HotStart, and an upstream homologous arm with a size of 721 bp was recovered by a DNA recovery kit.

(3) Taking genomic DNA of *Escherichia coli* W3110 as a template, a primer pair composed of primers P17 and P18 was used for PCR amplification of a high-fidelity amplification enzyme KAPA HiFi HotStart, and a downstream homologous arm with a size of 618 bp was recovered by a DNA recovery kit.

(4) Taking the upstream homologous arm recovered in step (2) and the downstream homologous arm recovered in step (3), which were mixed, as a template, a primer pair composed of primers P15 and P18 was used for overlap PCR to obtain a ΔthiE-Up-Down fragment as shown in SEQ ID No. 10.

### 3. Obtainment of YPVal- adhE02-Cas9 strain

(1) A plasmid pREDCas9 was transformed into competent cells of an engineered bacterium YPVal-adhE02, then coated on a 2-YT agar plate containing 100 mg/L Spectinomycin and cultured at 32°C to obtain Spectinomycin-resistant single colonies.
(2) Taking the single colonies obtained in step (1) as a template respectively, a PCR amplification product was obtained by performing PCR amplification using a primer pair composed of primers pRedCas9-PF and pRedCas9-PR. If the PCR amplification product obtained from a single colony contained a DNA fragment with a nucleotide sequence as shown in SEQ ID No. 3, this colony contained the plasmid pREDCas9. The strain of this colony was named as the YPVal-adhE02-Cas9 strain.

### 4. Obtainment of engineering bacterium YPVal-adhE03

(1) Culture of YPVal-adhE02-Cas9 strain; when the YPVal-adhE02-Cas9 strain grew to OD₆₀₀ₙₘ of 0.1, IPTG was added such that the concentration of IPTG in the system was 0.1 mM, and continued to be cultured to induce λ-Red-mediated homologous recombination; and when theYPVal-adhE02-Cas9 strain grew to OD₆₀₀ₙₘ of 0.6, bacterial cells were collected and competent cells of the YPVal-adhE02-Cas9 strain were prepared.
(2) The pGRB-thiE sgRNA plasmid obtained in step 1 and the ΔthiE-Up-Down fragment obtained in step 2 were transformed into competent cells of the YPVal-adhE02-Cas9, coated on a 2-YT agar plate containing 100 mg/L Spectinomycin and 100 mg/L Ampicillin, and cultured at 32°C to obtain single colonies.
(3) Taking the single colonies obtained in step (2) as a template respectively, a PCR amplification product was obtained by performing PCR amplification using a primer pair composed of primers P15 and P18. If the PCR amplification product obtained from a single colony contained a DNA fragment with a size of 1296 bp, this colony was preliminarily identified as a positive transformant.
(4) The positive transformant obtained in step (3) was inoculated on a 2-YT agar plate containing 100 mg/L Spectinomycin and 0.2% (m/v) arabinose and cultured at 32°C (for eliminating pGRB-thiE sgRNA plasmids); then, colonies that grew on a 2-YT agar plate containing 100 mg/L Spectinomycin but did not grow on a 2-YT agar plate containing 100 mg/L Ampicillin were picked out, then transferred to a 2-YT agar plate and cultured at 42°C (for eliminating plasmids pREDCas9); and finally, colonies which did not grow on a 2-YT agar plate containing 100 mg/L Spectinomycin but on an antibody-free 2-YT agar plate were picked out.
(5) Taking the single colonies obtained in step (4) as a template respectively, a PCR amplification product was obtained by performing PCR amplification using a primer pair composed of primers P15 and P18. If the PCR amplification product obtained from a single colony contained a DNA fragment with a size of 1296 bp, this colony was identified as a positive transformant in which a thiE gene was deleted from a genome of an engineered bacterium YPVal-adhE02. This positive transformant was named as the engineered bacterium YPVal-adhE03.

### IV. Obtainment of engineering bacterium YPVal-adhE04

Taking the engineered bacterium YPVal-adhE03 as an originating bacterium, according to a genome sequence of *Escherichia coli* W3110 published by NCBI, an mdh gene was knocked from a genome of the engineered bacterium YPVal-adhE03 using a CRISPR/Cas9 gene editing technology.

The mdh gene encodes malate dehydrogenase with a Gene ID of 947854 and an amino acid sequence as shown in SEQ ID No. 18.

The specific steps were as follows:

### 1. Construction of pGRB-mdh sgRNA plasmid

According to the genome sequence of *Escherichia coli* W3110 published by NCBI, an sgRNA target sequence (SEQ ID No. 11) from which an mdh gene was knocked out was designed by using CRISPR RGEN Tools (http://www.rgenome.net/cas-designer/), and a linearized pGRB vector homologous arm sequence was added to ends 5' and 3' of the target sequence, in order to construct an sgRNA plasmid.
(1) A primer sgRNA-5F: 5'-TGACAGCTAGCTCAGTCCTAGGTATAATACTAGTgcctttcagttccgcaacaaaggGTTTTAGAG CTAGAAATAGCAAGTTAAAATAAGG-3' (as shown in SEQ ID No. 49, underlined as a pGRB vector homologous arm sequence) and a primer sgRNA-5R: 5'-CCTTATTTTAACTTGCTATTTCTAGCTCTAAAACcctttgttgcggaactgaaaggcACTAGTATTAT ACCTAGGACTGAGCTAGCTGTCA-3' (as shown in SEQ ID No. 50, underlined as a pGRB vector homologous arm sequence) were synthesized by Invitrogen.
(2) The primer sgRNA-5F and the primer sgRNA-5R were annealed (a reaction procedure: denaturation at 95°C for 5 min, and annealing at 50°C for 1 min); and then, a target fragment was recovered by a DNA purification kit, measured for a DNA concentration, and diluted in concentration to 100 ng/µL to obtain an annealing product. The annealing product contained an sgRNA-5 fragment with a nucleotide sequence as shown in SEQ ID No. 11.
(3) Approximately 2700 bp of DNA fragments were recovered by a DNA recovery kit (QIAGEN Gel Extraction Kit) by digesting a pGRB vector with a restriction enzyme *Spe*I*.*

The digestion system was 50 µL and composed of 5 µL of 10×Buffer (owned by the restriction enzyme *Spe*I), 2.5 µL of restriction enzyme *Spe*I, 3000-5000 ng of pGRB vector, and ddH₂O.

The digestion procedure was carried out at 37°C for 3 h.

(4) The DNA fragments recovered in step (3) were dephosphorylated (to prevent self-adhesion of the pGRB vector) and recovered by a DNA recovery kit to obtain a linearized pGRB vector.

The dephosphorylation system was 50 µL and composed of 5 µL of 10×Buffer (owned by CIAP), 1000-2000 ng of DNA fragments recovered in step (3), 2.5 µL of CIAP, and ddH₂O.

The dephosphorylation procedure was carried out at 37°C for 1 h.

(5) The linearized pGRB vector obtained in step (4) and the annealing product obtained in step (2) were recombined by a Gibson Assembly kit, and then transformed into competent cells of *Escherichia coli* DH5α to obtain a pGRB-mdh sgRNA plasmid.

The recombination system was 5 µL and composed of 2 µL of linearized pGRB vector, 0.5 µL of annealing product, and 2.5 µL of assembly enzyme (owned by the Gibson Assembly kit).

The recombination procedure was carried out at 50°C for 30 min.

### 2. Obtainment of Δmdh-Up-Down fragment

(1) According to a genome sequence of *Escherichia coli* W3110 published by NCBI, a primer pair for amplifying an upstream homologous arm and a primer pair for amplifying a downstream homologous arm were designed and synthesized by Invitrogen.

The primer pair for amplifying the upstream homologous arm was composed of a primer P19: 5'-AACTTCCTCCAAACCGATGC-3' (as shown in SEQ ID No. 51) and a primer P20: 5'-CAATATAATAAGGAGTTTAGGTTGATTAGCGGATAATAAAAAACC-3' (as shown in SEQ ID No. 52).

The primer pair for amplifying the downstream homologous arm was composed of a primer P21: 5'-GGTTTTTTATTATCCGCTAATCAACCTAAACTCCTTATTATATTG-3' (as shown in SEQ ID No. 53) and a primer P22: 5'-TCTTCAATGGACTGGAGGTG-3' (as shown in SEQ ID No. 54).

(2) Taking genomic DNA of *Escherichia coli* W3110 as a template, a primer pair composed of primers P19 and P20 was used for PCR amplification of a high-fidelity amplification enzyme KAPA HiFi HotStart, and an upstream homologous arm with a size of 590 bp was recovered by a DNA recovery kit.

(3) Taking genomic DNA of *Escherichia coli* W3110 as a template, a primer pair composed of primers P21 and P22 was used for PCR amplification of a high-fidelity amplification enzyme KAPA HiFi HotStart, and a downstream homologous arm with a size of 708 bp was recovered by a DNA recovery kit.

(4) Taking the upstream homologous arm recovered in step (2) and the downstream homologous arm recovered in step (3), which were mixed, as a template, a primer pair composed of primers P19 and P22 was used for overlap PCR to obtain a Δmdh-Up-Down fragment as shown in SEQ ID No. 12.

### 3. Obtainment of YPVal- adhE03-Cas9 strain

(1) A plasmid pREDCas9 was transformed into competent cells of an engineered bacterium YPVal-adhE03, then coated on a 2-YT agar plate containing 100 mg/L Spectinomycin and cultured at 32°C to obtain Spectinomycin-resistant single colonies.
(2) Taking the single colonies obtained in step (1) as a template respectively, a PCR amplification product was obtained by performing PCR amplification using a primer pair composed of primers pRedCas9-PF and pRedCas9-PR. If the PCR amplification product obtained from a single colony contained a DNA fragment with a nucleotide sequence as shown in SEQ ID No. 3, this colony contained the plasmid pREDCas9. The strain of this colony was named as the YPVal-adhE03-Cas9 strain.

### 4. Obtainment of engineering bacterium YPVal-adhE04

(1) Culture of YPVal-adhE03-Cas9 strain; when the YPVal-adhE03-Cas9 strain grew to OD₆₀₀ₙₘ of 0.1, IPTG was added such that the concentration of IPTG in the system was 0.1 mM, and continued to be cultured to induce λ-Red-mediated homologous recombination; and when the YPVal-adhE03-Cas9 strain grew to OD₆₀₀ₙₘ of 0.6, bacterial cells were collected and competent cells of the YPVal-adhE03-Cas9 strain were prepared.
(2) The pGRB-mdh sgRNA plasmid obtained in step 1 and the Δmdh-Up-Down fragment obtained in step 2 were transformed into competent cells of the YPVal-adhE03-Cas9 strain, coated on a 2-YT agar plate containing 100 mg/L Spectinomycin and 100 mg/L Ampicillin, and cultured at 32°C to obtain single colonies.
(3) Taking the single colonies obtained in step (2) as a template respectively, a PCR amplification product was obtained by performing PCR amplification using a primer pair composed of primers P19 and P22. If the PCR amplification product obtained from a single colony contained a DNA fragment with a size of 1253 bp, this colony was preliminarily identified as a positive transformant.
(4) The positive transformant obtained in step (3) was inoculated on a 2-YT agar plate containing 100 mg/L Spectinomycin and 0.2% (m/v) arabinose and cultured at 32°C (for eliminating pGRB-thiE sgRNA plasmids); then, colonies that grew on a 2-YT agar plate containing 100 mg/L Spectinomycin but did not grow on a 2-YT agar plate containing 100 mg/L Ampicillin were picked out, then transferred to a 2-YT agar plate and cultured at 42°C (for eliminating plasmids pREDCas9); and finally, colonies which did not grow on a 2-YT agar plate containing 100 mg/L Spectinomycin but on an antibody-free 2-YT agar plate were picked out.
(5) Taking the single colonies obtained in step (4) as a template respectively, a PCR amplification product was obtained by performing PCR amplification using a primer pair composed of primers P19 and P22. If the PCR amplification product obtained from a single colony contained a DNA fragment with a size of 1253 bp, this colony was identified as a positive transformant in which a thiE gene was deleted from a genome of an engineered bacterium YPVal-adhE03. This positive transformant was named as the engineered bacterium YPVal-adhE04.

Example 5: Modification II of engineered bacteria originating from *Escherichia coli* W3110 After a large number of experiments, the inventors of the present invention obtained engineered bacteria YPVal-adhE05, YPVal-adhE06 YPVal-adhE07 and YPVal-adhE08 by means of modification originating from *Escherichia coli* W3110. Genotypes of the engineered bacteria YPVal-adhE05, YPVal-adhE06, YPVal-adhE07 and YPVal-adhE08 were shown in Table 8.

**Table 8**

| Strains | Genotypes |
|---|---|
| YPVal-adhE05 | W3110-ΔadhE |
| YPVal-adhE06 | W3110-ΔadhE-ΔilvE(E)-ptrcilvE(B) |
| YPVal-adhE07 | W3110-ΔadhE-ΔilvE(E)-ptrcilvE(B)-ΔthiE |
| YPVal-adhE08 | W3110-ΔadhE-ΔilvE(E)-ptrcilvE(B)-ΔthiE-Δmdh |

### I. Obtainment of engineering bacterium YPVal-adhE05

### 1. Construction of pGRB-adhE sgRNA plasmid

The same as 1 in step I in Example 4.

### 2. Obtainment of ΔadhE-Up-Down fragment

The same as 2 in step I in Example 4.

### 3. Obtainment of W3110-Cas9 strain

According to 3 in step I in Example 4, competent cells of a valine-producing bacterium CGMCC 22721 were replaced with competent cells of *Escherichia coli* W3110, and the other steps were unchanged, obtaining the W3110-Cas9 strain.

### 4. Obtainment of engineering bacterium YPVal-adhE05

According to 4 in step I in Example 4, a CGMCC 22721-Cas9 strain was replaced with a W3110-Cas9 strain, and the other steps were unchanged, obtaining the engineered bacterium YPVal-adhE05.

### II. Obtainment of engineering bacterium YPVal-adhE06

### 1. Construction of pGRB-ilvE sgRNA plasmid

The same as 1 in step II in Example 4.

### 2. Obtainment of ptrc-ilvE(B)-Up-Down fragment

The same as 2 in step II in Example 4.

### 3. Obtainment of YPVal- adhE05-Cas9 strain

According to 3 in step II in Example 4, competent cells of an engineered bacterium YPVal-adhE01 were replaced with competent cells of an engineered bacterium YPVal-adhE05, and the other steps were unchanged, obtaining the YPVal-adhE05-Cas9 strain.

### 4. Obtainment of engineering bacterium YPVal-adhE06

According to 4 in step II in Example 4, a YPVal-adhE01-Cas9 strain was replaced with a YPVal-adhE05-Cas9 strain, and the other steps were unchanged, obtaining the engineered bacterium YPVal-adhE06.

### III. Obtainment of engineering bacterium YPVal-adhE07

### 1. Construction of pGRB-thiE sgRNA plasmid

The same as 1 in step III in Example 4.

### 2. Obtainment of ΔthiE-Up-Down fragment

The same as 2 in step III in Example 4.

### 3. Obtainment of YPVal- adhE06-Cas9 strain

According to 3 in step III in Example 4, competent cells of an engineered bacterium YPVal-adhE02 were replaced with competent cells of an engineered bacterium YPVal-adhE06, and the other steps were unchanged, obtaining the YPVal-adhE06-Cas9 strain.

### 4. Obtainment of engineering bacterium YPVal-adhE07

According to 4 in step III in Example 4, a YPVal-adhE02-Cas9 strain was replaced with a YPVal-adhE06-Cas9 strain, and the other steps were unchanged, obtaining the engineered bacterium YPVal-adhE07.

### IV. Obtainment of engineering bacterium YPVal-adhE08

### 1. Construction of pGRB-mdh sgRNA plasmid

The same as 1 in step IV in Example 4.

### 2. Obtainment of Δmdh-Up-Down fragment

The same as 2 in step IV in Example 4.

### 3. Obtainment of YPVal- adhE07-Cas9 strain

According to 3 in step IV in Example 4, competent cells of an engineered bacterium YPVal-adhE03 were replaced with competent cells of an engineered bacterium YPVal-adhE07, and the other steps were unchanged, obtaining the YPVal-adhE07-Cas9 strain.

### 4. Obtainment of engineering bacterium YPVal-adhE08

According to 4 in step IV in Example 4, a YPVal-adhE03-Cas9 strain was replaced with a YPVal-adhE07-Cas9 strain, and the other steps were unchanged, obtaining the engineered bacterium YPVal-adhE08.

### Example 6: Production of L-valine from engineered bacteria modified in Example 4 and Example 5 by means of fermentation

1. The engineered bacteria YPVal-adhE01, YPVal-adhE02, YPVal-adhE03, YPVal-adhE04, YPVal-adhE05, YPVal-adhE06, YPVal-adhE07 and YPVal-adhE08 modified in Example 4 and Example 5, as well as a valine-producing bacterium CGMCC 22721 and *Escherichia coli* W3110 as originating bacteria were respectively fermented in a fermentation tank (Shanghai Bailun Biotechnology Co., Ltd., a model of BLBIO-5GC-4-H) to obtain fermentation broth.

### Each strain was repeated three times

The composition of a fermentation medium during fermentation was shown in Table 3.

The fermentation control process was shown in Table 4.

### 2. The yield of L-valine in the fermentation broth was detected by high-performance liquid chromatography

The detection results of the engineered bacteria YPVal-adhE01, YPVal-adhE02, YPVal-adhE03 and YPVal-adhE04 obtained from a valine-producing bacterium CGMCC 22721 and a valine-producing bacterium CGMCC 22721 by means of three fermentations were shown in Table 9 (P-value<0.01 indicated a very significant difference). The results showed that compared with the valine-producing bacterium CGMCC 22721, YPVal-adhE01, YPVal-adhE02, YPVal-adhE03 and YPVal-adhE04 could significantly increase the yield of L-valine. That is, all engineered bacteria obtained by "knocking out an adhE gene", "knocking out an adhE gene and inserting ilvE(B) from *Bacillus subtilis* initiated by ptrc while knocking out an ilvE(E) gene", "knocking out an adhE gene and a thiE gene and inserting ilvE(B) from *Bacillus subtilis* initiated by ptrc while knocking out an ilvE(E) gene" or "knocking out an adhE gene, a thiE gene and an mdh gene and inserting ilvE (B) from *Bacillus subtilis* initiated by ptrc while knocking out an ilvE(E) gene" could increase the yield of L-valine in the valine-producing bacterium CGMCC 22721.

**Table 9**

| Strains | L-valine concentration-1 (g/L) | L-valine concentration-2 (g/L) | L-valine concentration-3 (g/L) | Mean (g/L) | P-value |
|---|---|---|---|---|---|
| Valine-producing bacterium CGMCC 22721 | 78.10 | 77.88 | 78.23 | 78.07±0.18 | |
| YPVal-adhE01 | 80.42 | 80.37 | 80.50 | 80.43±0.07 | P<0.01 |
| YPVal-adhE02 | 82.26 | 82.54 | 82.66 | 82.49±0.21 | P<0.01 |
| YPVal-adhE03 | 84.52 | 84.24 | 84.85 | 84.54±0.31 | P<0.01 |
| YPVal-adhE04 | 85.86 | 85.95 | 86.04 | 85.95±0.09 | P<0.01 |

The detection results of *Escherichia coli* W3110 and engineered bacteria YPVal-adhE05, YPVal-adhE06, YPVal-adhE07 and YPVal-adhE08 obtained from *Escherichia coli* W3110 by means of three fermentations were shown in Table 10 (P-value<0.01 indicated a very significant difference). The results showed that compared with the *Escherichia coli* W3110, YPVal-adhE05, YPVal-adhE06, YPVal-adhE07 and YPVal-adhE08 could significantly increase the yield of L-valine. That is, all engineered bacteria obtained by "knocking out an adhE gene", "knocking out an adhE gene and inserting ilvE(B) from *Bacillus subtilis* initiated by ptrc while knocking out an ilvE(E) gene", "knocking out an adhE gene and a thiE gene and inserting ilvE(B) from *Bacillus subtilis* initiated by ptrc while knocking out an ilvE(E) gene" or "knocking out an adhE gene, a thiE gene and an mdh gene and inserting ilvE (B) from *Bacillus subtilis* initiated by ptrc while knocking out an ilvE(E) gene" could increase the yield of L-valine in the *Escherichia coli* W3110.

**Table 10**

| Strains | L-valine concentration-1 (g/L) | L-valine concentration-2 (g/L) | L-valine concentration-3 (g/L) | Mean (g/L) | P-value |
|---|---|---|---|---|---|
| *Escherichia coli* W3110 | 0.009 | 0.005 | 0.007 | 0.007±0.002 | |
| YPVal-adhE05 | 0.121 | 0.109 | 0.102 | 0.111±0.010 | P<0.01 |
| YPVal-adhE06 | 0.229 | 0.210 | 0.243 | 0.227±0.017 | P<0.01 |
| YPVal-adhE07 | 0.309 | 0.335 | 0.341 | 0.328±0.017 | P<0.01 |
| YPVal-adhE08 | 0.442 | 0.416 | 0.419 | 0.426±0.014 | P<0.01 |

### Example 7: Modification III of engineered bacteria originating from valine-producing bacterium CGMCC 22721

After a large number of experiments, the inventors of the present invention obtained engineered bacteria YPVal-ilvE01, YPVal-ilvE02 and YPVal-ilvE03 by means of modification originating from a valine-producing bacterium CGMCC 22721. Genotypes of the engineered bacteria YPVal-ilvE01, YPVal-ilvE02 and YPVal-ilvE03 were shown in Table 11.

**Table 11**

| Engineered bacteria | Genotypes |
|---|---|
| YPVal-ilvE01 | CGMCC 22721-ΔilvE(E)-ptrcilvE(B) |
| YPVal-ilvE02 | CGMCC 22721-ΔilvE(E)-ptrcilvE(B)-ΔthiE |
| YPVal-ilvE03 | CGMCC 22721-ΔilvE(E)-ptrcilvE(B)-ΔthiE-Δmdh |

### I. Obtainment of engineering bacterium YPVal-ilvE01

According to a genome sequence of *Escherichia coli* W3110 and a genome sequence of *Bacillus subtilis subsp. subtilis str.168* published by NCBI, an engineered bacterium YPVal-ilvE01 (hereinafter referred to as YPVal-ilvE01) was obtained by knocking in an ilvE gene (i.e., ptrc-ilvE(B) sequence) of *Bacillus subtilis* initiated by a ptrc promoter while knocking out the ilvE(E) gene from a genome of the valine-producing bacterium CGMCC 22721 by a CRISPR/Cas9 gene editing technology. A nucleotide sequence of the ptrc-ilvE(B) sequence was as shown in SEQ ID No. 6, in which positions 1096-1169 were a nucleotide sequence of the ptrc promoter from end 5', and positions 1-1095 were an ilvE(B) gene of *Bacillus subtilis.*

The ilvE(E) gene of the valine-producing bacterium CGMCC 22721 encodes branched-chain amino acid transaminase, with Gene ID of 948278 and an amino acid sequence as shown in SEQ ID No. 15.

The ilvE(B) gene of *Bacillus subtilis* encodes branched-chain amino acid transaminase, with Gene ID of 938420 and an amino acid sequence as shown in SEQ ID No. 16.

The specific steps were as follows:

### 1. Construction of pGRB-ilvE sgRNA plasmid

(1) A primer sgRNA-3F: 5'-TGACAGCTAGCTCAGTCCTAGGTATAATACTAGTgaaagcagcgataatcacgtcgGTTTTAGAG CTAGAAATAGCAAGTTAAAATAAGG-3' (as shown in SEQ ID No. 35, underlined as a pGRB vector homologous arm sequence), a primer sgRNA-3R: 5'-CCTTATTTTAACTTGCTATTTCTAGCTCTAAAACccgacgtgattatcgctgctttcACTAGTATTATA CCTAGGACTGAGCTAGCTGTCA-3' (as shown in SEQ ID No. 36, underlined as a pGRB vector homologous arm sequence), a primer sgRNA-PF : 5'-GTCTCATGAGCGGATACATATTTG-3' (SEQ ID No. 21) and a primer sgRNA-PR: 5'-ATGAGAAAGCGCCACGCT-3' (SEQ ID No. 22) were synthesized by Invitrogen.
(2) The primer sgRNA-3F and the primer sgRNA-3R were annealed (a reaction procedure: denaturation at 95°C for 5 min, and annealing at 50°C for 1 min); and then, a target fragment was recovered by a DNA purification kit, measured for a DNA concentration, and diluted in concentration to 100 ng/µL to obtain an annealing product. The annealing product contained an sgRNA-3 fragment with a nucleotide sequence as shown in SEQ ID No. 7.
(3) Approximately 2700 bp of DNA fragments were recovered by a DNA recovery kit (QIAGEN Gel Extraction Kit) by digesting a pGRB vector (Addgene's product, catalog number 71539) with a restriction enzyme *Spe*I (Takara's product, catalog number 1631).

The digestion system was 50 µL and composed of 5 µL of 10×Buffer (owned by the restriction enzyme *Spe*I), 2.5 µL of restriction enzyme *Spe*I, 3000-5000 ng of pGRB vector, and ddH₂O.

The digestion procedure was carried out at 37°C for 3 h.

(4) The DNA fragments recovered in step (3) were dephosphorylated (to prevent self-adhesion of the pGRB vector) and recovered by a DNA recovery kit to obtain a linearized pGRB vector.

The dephosphorylation system was 50 µL and composed of 5 µL of 10×Buffer (owned by CIAP), 1000-2000 ng of DNA fragments recovered in step (3), 2.5 µL of CIAP (Takara's product, catalog number 2250A), and ddH₂O.

The dephosphorylation procedure was carried out at 37°C for 1 h.

(5) The linearized pGRB vector obtained in step (4) and the annealing product obtained in step (2) were recombined by a Gibson Assembly kit (New England), and then transformed into competent cells (TAKARA) of *Escherichia coli* DH5α to obtain a pGRB-ilvE sgRNA plasmid.

The recombination system was 5 µL and composed of 2 µL of linearized pGRB vector, 0.5 µL of annealing product, and 2.5 µL of assembly enzyme (owned by the Gibson Assembly kit).

The recombination procedure was carried out at 50°C for 30 min.

### 2. Obtainment of ptrc-ilvE(B)-Up-Down fragment

(1) According to a genome sequence of *Escherichia coli* W3110 published by NCBI, a primer pair for amplifying an upstream homologous arm and a primer pair for amplifying a downstream homologous arm were designed and synthesized by Invitrogen. According to the genome sequence of *Bacillus subtilis subsp. subtilis str.168* published by NCBI, a primer pair was designed and synthesized by Invitrogen for amplifying ptrc-ilvE(B).

The primer pair for amplifying the upstream homologous arm was composed of a primer P9: 5'-CAGGCAGTTCATTGAGTTAGCG-3' (as shown in SEQ ID No. 37) and a primer P10: 5'-CACAGTGTATTAAGCAGACGTTAAATACAAAAAATGGGACGGCAC-3' (as shown in SEQ ID No. 38).

The primer pair for amplifying the downstream homologous arm was composed of a primer P13: 5'-GTTATCCGCTCAATTCCACACACATTATACGAGCCGGATGATTATGTCAATTGTCAATT TTATTTTTTGCGCTC-3' (as shown in SEQ ID No. 39, underlined as a partial sequence of the ptrc promoter) and a primer P14: 5'-ACGGTTAGGGATGGTTCGAC-3' (SEQ ID No. 40).

The primer pair for amplifying ptrc-ilvE(B) was composed of a primer P11: 5'-GTGCCGTCCCATTTTTTGTATTTAACGTCTGCTTAATACACTGTG-3' (as shown in SEQ ID No. 41) and a primer P12: 5'-GTATAATGTGTGGAATTGTGAGCGGATAACAATTTCACACAGGAAACAGACCATGGA ACTTTTTAAATATATGGAG-3' (SEQ ID No. 42, underlined as a partial sequence of the ptrc promoter).

(2) Taking genomic DNA of *Escherichia coli* W3110 as a template, a primer pair composed of primers P9 and P10 was used for PCR amplification of a high-fidelity amplification enzyme KAPA HiFi HotStart, and an upstream homologous arm with a size of 709 bp was recovered by a DNA recovery kit.

(3) Taking genomic DNA of *Escherichia coli* W3110 as a template, a primer pair composed of primers P13 and P14 was used for PCR amplification of a high-fidelity amplification enzyme KAPA HiFi HotStart, and a downstream homologous arm with a size of 611 bp was recovered by a DNA recovery kit.

(4) Taking genomic DNA of *Bacillus subtilis subsp.subtilis str.168* as a template, a primer pair composed of primers P11 and P12 was used for PCR amplification of a high-fidelity amplification enzyme KAPA HiFi HotStart, and an ilvE(B) fragment with a size of 1168 bp was recovered by a DNA recovery kit.

(5) Taking the upstream homologous arm recovered in step (2), the downstream homologous arm recovered in step (3) and the ilvE(B) fragment recovered in step (4), which were mixed, as a template, a primer pair composed of primers P9 and P14 was used for overlap PCR to obtain a ptrc-ilvE(B)-Up-Down fragment as shown in SEQ ID No. 8.

### 3. Obtainment of CGMCC 22721-Cas9 strain

(1) A plasmid pREDCas9 (Addgene's product, catalog number 71541; containing a Spectinomycin-resistant gene) was transformed into competent cells of a valine-producing bacterium CGMCC 22721, then coated on a 2-YT agar plate containing 100 mg/L Spectinomycin and cultured at 32°C to obtain Spectinomycin-resistant single colonies.

A preparation method for the 2-YT agar plate was as follows: dissolving 16 g of tryptone, 10 g of yeast extract powder, 5 g of sodium chloride and 16 g of agar powder in an appropriate amount of water, then metering a volume with water to 1 L, adjusting a pH value to 7.0 with sodium hydroxide, and sterilizing at 121°C for 20 min.

(2) Taking the single colonies obtained in step (1) as a template respectively, a PCR amplification product was obtained by performing PCR amplification using a primer pair composed of a primer pRedCas9-PF: 5'-GCAGTGGCGGTTTTCATG-3' (SEQ ID No. 27) and a primer pRedCas9-PR: 5'-CCTTGGTGATCTCGCCTTTC-3' (SEQ ID No.28). If the PCR amplification product obtained from a single colony contained a DNA fragment with a nucleotide sequence as shown in SEQ ID No. 3, this colony contained the plasmid pREDCas9. The strain of this colony was named as the CGMCC 22721-Cas9 strain.

### 4. Obtainment of engineering bacterium YPVal-ilvE01

(1) Culture of CGMCC 22721-Cas9 strain; when the strain CGMCC 22721-Cas9 grew to OD₆₀₀ₙₘ of 0.1, IPTG was added such that the concentration of IPTG in the system was 0.1 mM, and continued to be cultured to induce λ-Red-mediated homologous recombination; and when the strain CGMCC 22721-Cas9 grew to OD₆₀₀ₙₘ of 0.6, bacterial cells were collected and competent cells of CGMCC 22721-Cas9 were prepared.
(2) The pGRB-ilvE sgRNA plasmid obtained in step 1 and the ptrc-ilvE(B)-Up-Down fragment obtained in step 2 were transformed into competent cells of CGMCC 22721-Cas9, coated on a 2-YT agar plate containing 100 mg/L Spectinomycin and 100 mg/L Ampicillin, and cultured at 32°C to obtain single colonies.
(3) Taking the single colonies obtained in step (2) as a template respectively, a PCR amplification product was obtained by performing PCR amplification using a primer pair composed of primers P11 and P12. If the PCR amplification product obtained from a single colony contained a DNA fragment with a size of 1168 bp, this colony was preliminarily identified as a positive transformant.
(4) The positive transformant obtained in step (3) was inoculated on a 2-YT agar plate containing 100 mg/L Spectinomycin and 0.2% (m/v) arabinose and cultured at 32°C (for eliminating pGRB-ilvE sgRNA plasmids); then, colonies that grew on a 2-YT agar plate containing 100 mg/L Spectinomycin but did not grow on a 2-YT agar plate containing 100 mg/L Ampicillin were picked out, then transferred to a 2-YT agar plate and cultured at 42°C (for eliminating plasmids pREDCas9); and finally, colonies which did not grow on a 2-YT agar plate containing 100 mg/L Spectinomycin but on an antibody-free 2-YT agar plate were picked out.
(5) Taking the single colonies obtained in step (4) as a template respectively, a PCR amplification product was obtained by performing PCR amplification using a primer pair composed of primers P11 and P12. If the PCR amplification product obtained from a single colony contained a DNA fragment with a size of 1168 bp, this colony was identified as a positive transformant. The positive transformant in which a *Bacillus subtilis* ilvE(B) gene (i.e., a ptrc-ilvE(B) sequence) initiated by a ptrc promoter was knocked in while an ilvE(E) gene was knocked out from a genome of the engineered bacterium CGMCC 22721 was named as the engineered bacterium YPVal-ilvE01.

### II. Obtainment of engineering bacterium YPVal-ilvE02

Taking the engineered bacterium YPVal-ilvE01 obtained in step 1 as an originating bacterium, according to a genome sequence of *Escherichia coli* W3110 published by NCBI, a thiE gene was knocked from the genome of the engineered bacterium YPVal-ilvE01 using a CRISPR/Cas9 gene editing technology.

The thiE gene encodes thiamine phosphate synthase with a Gene ID of 948491 and an amino acid sequence as shown in SEQ ID No. 17.

The specific steps were as follows:

### 1. Construction of pGRB-thiE sgRNA plasmid

According to the genome sequence of *Escherichia coli* W3110 published by NCBI, an sgRNA target sequence (SEQ ID No. 9) from which a thiE gene was knocked out was designed by using CRISPR RGEN Tools (http://www.rgenome.net/cas-designer/), and a linearized pGRB vector homologous arm sequence was added to ends 5' and 3' of the target sequence, in order to construct an sgRNA plasmid.
(1) A primer sgRNA-4F: 5'-TGACAGCTAGCTCAGTCCTAGGTATAATACTAGTcgcccctcttatatcgcgctggGTTTTAGAG CTAGAAATAGCAAGTTAAAATAAGG-3' (as shown in SEQ ID No. 43, underlined as a pGRB vector homologous arm sequence) and a primer sgRNA-4R: 5'-CCTTATTTTAACTTGCTATTTCTAGCTCTAAAACcccagcgcgatataagaggggcgACTAGTATTA TACCTAGGACTGAGCTAGCTGTCA-3' (as shown in SEQ ID No. 44, underlined as a pGRB vector homologous arm sequence) were synthesized by Invitrogen.
(2) The primer sgRNA-4F and the primer sgRNA-4R were annealed (a reaction procedure: denaturation at 95°C for 5 min, and annealing at 50°C for 1 min); and then, a target fragment was recovered by a DNA purification kit, measured for a DNA concentration, and diluted in concentration to 100 ng/µL to obtain an annealing product. The annealing product contained an sgRNA-4 fragment with a nucleotide sequence as shown in SEQ ID No. 9.
(3) Approximately 2700 bp of DNA fragments were recovered by a DNA recovery kit (QIAGEN Gel Extraction Kit) by digesting a pGRB vector with a restriction enzyme *Spe*I*.*

The digestion system was 50 µL and composed of 5 µL of 10×Buffer (owned by the restriction enzyme *Spe*I), 2.5 µL of restriction enzyme *Spe*I, 3000-5000 ng of pGRB vector, and ddH₂O.

The digestion procedure was carried out at 37°C for 3 h.

(4) The DNA fragments recovered in step (3) were dephosphorylated (to prevent self-adhesion of the pGRB vector) and recovered by a DNA recovery kit to obtain a linearized pGRB vector.

The dephosphorylation system was 50 µL and composed of 5 µL of 10×Buffer (owned by CIAP), 1000-2000 ng of DNA fragments recovered in step (3), 2.5 µL of CIAP, and ddH₂O.

The dephosphorylation procedure was carried out at 37°C for 1 h.

(5) The linearized pGRB vector obtained in step (4) and the annealing product obtained in step (2) were recombined by a Gibson Assembly kit, and then transformed into competent cells of *Escherichia coli* DH5α to obtain a pGRB-thiE sgRNA plasmid.

The recombination system was 5 µL and composed of 2 µL of linearized pGRB vector, 0.5 µL of annealing product, and 2.5 µL of assembly enzyme (owned by the Gibson Assembly kit).

The recombination procedure was carried out at 50°C for 30 min.

### 2. Obtainment of ΔthiE-Up-Down fragment

(1) According to a genome sequence of *Escherichia coli* W3110 published by NCBI, a primer pair for amplifying an upstream homologous arm and a primer pair for amplifying a downstream homologous arm were designed and synthesized by Invitrogen.

The primer pair for amplifying the upstream homologous arm was composed of a primer P15: 5'-TTCTATTCAGGACGCCAACG-3' (as shown in SEQ ID No. 45) and a primer P16: 5'-GCTATAACGCATAAAGTCACGGCACGCTTCCTCCTTACGCAGG-3' (as shown in SEQ ID No. 46).

The primer pair for amplifying the downstream homologous arm was composed of a primer P17: 5'-CCTGCGTAAGGAGGAAGCGTGCCGTGACTTTATGCGTTATAGC-3' (as shown in SEQ ID No. 47) and a primer P18: 5'-GCCTGCAAAGTGCCCATAACCC-3' (as shown in SEQ ID No. 48).

(2) Taking genomic DNA of *Escherichia coli* W3110 as a template, a primer pair composed of primers P15 and P16 was used for PCR amplification of a high-fidelity amplification enzyme KAPA HiFi HotStart, and an upstream homologous arm with a size of 721 bp was recovered by a DNA recovery kit.

(3) Taking genomic DNA of *Escherichia coli* W3110 as a template, a primer pair composed of primers P17 and P18 was used for PCR amplification of a high-fidelity amplification enzyme KAPA HiFi HotStart, and a downstream homologous arm with a size of 618 bp was recovered by a DNA recovery kit.

(4) Taking the upstream homologous arm recovered in step (2) and the downstream homologous arm recovered in step (3), which were mixed, as a template, a primer pair composed of primers P15 and P18 was used for overlap PCR to obtain a ΔthiE-Up-Down fragment as shown in SEQ ID No. 10.

### 3. Obtainment of YPVal- ilvE01-Cas9 strain

(1) A plasmid pREDCas9 was transformed into competent cells of an engineered bacterium YPVal-ilvE01, then coated on a 2-YT agar plate containing 100 mg/L Spectinomycin and cultured at 32°C to obtain Spectinomycin-resistant single colonies.
(2) Taking the single colonies obtained in step (1) as a template respectively, a PCR amplification product was obtained by performing PCR amplification using a primer pair composed of primers pRedCas9-PF and pRedCas9-PR. If the PCR amplification product obtained from a single colony contained a DNA fragment with a nucleotide sequence as shown in SEQ ID No. 3, this colony contained the plasmid pREDCas9. The strain of this colony was named as the YPVal-ilvE01-Cas9 strain.

### 4. Obtainment of engineering bacterium YPVal-ilvE02

(1) Culture of YPVal-ilvE01-Cas9 strain; when the YPVal-ilvE01-Cas9 strain grew to OD₆₀₀ₙₘ of 0.1, IPTG was added such that the concentration of IPTG in the system was 0.1 mM, and continued to be cultured to induce λ-Red-mediated homologous recombination; and when theYPVal-ilvE01-Cas9 strain grew to OD₆₀₀ₙₘ of 0.6, bacterial cells were collected and competent cells of the YPVal-ilvE01-Cas9 strain were prepared.
(2) The pGRB-thiE sgRNA plasmid obtained in step 1 and the ΔthiE-Up-Down fragment obtained in step 2 were transformed into competent cells of the YPVal-ilvE01-Cas9 strain, coated on a 2-YT agar plate containing 100 mg/L Spectinomycin and 100 mg/L Ampicillin, and cultured at 32°C to obtain single colonies.
(3) Taking the single colonies obtained in step (2) as a template respectively, a PCR amplification product was obtained by performing PCR amplification using a primer pair composed of primers P15 and P18. If the PCR amplification product obtained from a single colony contained a DNA fragment with a size of 1296 bp, this colony was preliminarily identified as a positive transformant.
(4) The positive transformant obtained in step (3) was inoculated on a 2-YT agar plate containing 100 mg/L Spectinomycin and 0.2% (m/v) arabinose and cultured at 32°C (for eliminating pGRB-thiE sgRNA plasmids); then, colonies that grew on a 2-YT agar plate containing 100 mg/L Spectinomycin but did not grow on a 2-YT agar plate containing 100 mg/L Ampicillin were picked out, then transferred to a 2-YT agar plate and cultured at 42°C (for eliminating plasmids pREDCas9); and finally, colonies which did not grow on a 2-YT agar plate containing 100 mg/L Spectinomycin but on an antibody-free 2-YT agar plate were picked out.
(5) Taking the single colonies obtained in step (4) as a template respectively, a PCR amplification product was obtained by performing PCR amplification using a primer pair composed of primers P15 and P18. If the PCR amplification product obtained from a single colony contained a DNA fragment with a size of 1296 bp, this colony was identified as a positive transformant in which a thiE gene was deleted from a genome of an engineered bacterium YPVal-ilvE01. This positive transformant was named as the engineered bacterium YPVal-ilvE02.

### III. Obtainment of engineering bacterium YPVal-ilvE03

Taking the engineered bacterium YPVal-ilvE02 obtained in step II as an originating bacterium, according to a genome sequence of *Escherichia coli* W3110 published by NCBI, an mdh gene was knocked out from a genome of the engineered bacterium YPVal-ilvE02 using a CRISPR/Cas9 gene editing technology.

The mdh gene encodes malate dehydrogenase with a Gene ID of 947854 and an amino acid sequence as shown in SEQ ID No. 18.

The specific steps were as follows:

### 1. Construction of pGRB-mdh sgRNA plasmid

According to the genome sequence of *Escherichia coli* W3110 published by NCBI, an sgRNA target sequence (SEQ ID No. 11) from which an mdh gene was knocked out was designed by using CRISPR RGEN Tools (http://www.rgenome.net/cas-designer/), and a linearized pGRB vector homologous arm sequence was added to ends 5' and 3' of the target sequence, in order to construct an sgRNA plasmid.
(1) A primer sgRNA-5F: 5'-TGACAGCTAGCTCAGTCCTAGGTATAATACTAGTgcctttcagttccgcaacaaaggGTTTTAGAG CTAGAAATAGCAAGTTAAAATAAGG-3' (as shown in SEQ ID No. 49, underlined as a pGRB vector homologous arm sequence) and a primer sgRNA-5R: 5'-CCTTATTTTAACTTGCTATTTCTAGCTCTAAAACcctttgttgcggaactgaaaggcACTAGTATTAT ACCTAGGACTGAGCTAGCTGTCA-3' (as shown in SEQ ID No. 50, underlined as a pGRB vector homologous arm sequence) were synthesized by Invitrogen.
(2) The primer sgRNA-5F and the primer sgRNA-5R were annealed (a reaction procedure: denaturation at 95°C for 5 min, and annealing at 50°C for 1 min); and then, a target fragment was recovered by a DNA purification kit, measured for a DNA concentration, and diluted in concentration to 100 ng/µL to obtain an annealing product. The annealing product contained an sgRNA-5 fragment with a nucleotide sequence as shown in SEQ ID No. 11.
(3) Approximately 2700 bp of DNA fragments were recovered by a DNA recovery kit (QIAGEN Gel Extraction Kit) by digesting a pGRB vector with a restriction enzyme *Spe*I*.*

The digestion system was 50 µL and composed of 5 µL of 10×Buffer (owned by the restriction enzyme *Spe*I), 2.5 µL of restriction enzyme *Spe*I, 3000-5000 ng of pGRB vector, and ddH₂O.

The digestion procedure was carried out at 37°C for 3 h.

(4) The DNA fragments recovered in step (3) were dephosphorylated (to prevent self-adhesion of the pGRB vector) and recovered by a DNA recovery kit to obtain a linearized pGRB vector.

The dephosphorylation system was 50 µL and composed of 5 µL of 10×Buffer (owned by CIAP), 1000-2000 ng of DNA fragments recovered in step (3), 2.5 µL of CIAP, and ddH₂O.

The dephosphorylation procedure was carried out at 37°C for 1 h.

(5) The linearized pGRB vector obtained in step (4) and the annealing product obtained in step (2) were recombined by a Gibson Assembly kit, and then transformed into competent cells of *Escherichia coli* DH5α to obtain a pGRB-mdh sgRNA plasmid.

The recombination system was 5 µL and composed of 2 µL of linearized pGRB vector, 0.5 µL of annealing product, and 2.5 µL of assembly enzyme (owned by the Gibson Assembly kit).

The recombination procedure was carried out at 50°C for 30 min.

### 2. Obtainment of Δmdh-Up-Down fragment

(1) According to a genome sequence of *Escherichia coli* W3110 published by NCBI, a primer pair for amplifying an upstream homologous arm and a primer pair for amplifying a downstream homologous arm were designed and synthesized by Invitrogen.

The primer pair for amplifying the upstream homologous arm was composed of a primer P19: 5'-AACTTCCTCCAAACCGATGC-3' (as shown in SEQ ID No. 51) and a primer P20: 5'-CAATATAATAAGGAGTTTAGGTTGATTAGCGGATAATAAAAAACC-3' (as shown in SEQ ID No. 52).

A primer pair for amplifying a downstream homologous arm was composed of a primer P21: 5'-GGTTTTTTATTATCCGCTAATCAACCTAAACTCCTTATTATATTG-3' (as shown in SEQ ID No. 53) and a primer P22: 5'-TCTTCAATGGACTGGAGGTG-3' (as shown in SEQ ID No. 54).

(2) Taking genomic DNA of *Escherichia coli* W3110 as a template, a primer pair composed of primers P19 and P20 was used for PCR amplification of a high-fidelity amplification enzyme KAPA HiFi HotStart, and an upstream homologous arm with a size of 590 bp was recovered by a DNA recovery kit.

(3) Taking genomic DNA of *Escherichia coli* W3110 as a template, a primer pair composed of primers P21 and P22 was used for PCR amplification of a high-fidelity amplification enzyme KAPA HiFi HotStart, and a downstream homologous arm with a size of 708 bp was recovered by a DNA recovery kit.

(4) Taking the upstream homologous arm recovered in step (2) and the downstream homologous arm recovered in step (3), which were mixed, as a template, a primer pair composed of primers P19 and P22 was used for overlap PCR to obtain a Δmdh-Up-Down fragment as shown in SEQ ID No. 12.

### 3. Obtainment of YPVal- ilvE02-Cas9 strain

(1) A plasmid pREDCas9 was transformed into competent cells of an engineered bacterium YPVal-ilvE02, then coated on a 2-YT agar plate containing 100 mg/L Spectinomycin and cultured at 32°C to obtain Spectinomycin-resistant single colonies.
(2) Taking the single colonies obtained in step (1) as a template respectively, a PCR amplification product was obtained by performing PCR amplification using a primer pair composed of primers pRedCas9-PF and pRedCas9-PR. If the PCR amplification product obtained from a single colony contained a DNA fragment with a nucleotide sequence as shown in SEQ ID No. 3, this colony contained the plasmid pREDCas9. The strain of this colony was named as the YPVal-ilvE02-Cas9 strain.

### 4. Obtainment of engineering bacterium YPVal-ilvE03

(1) Culture of YPVal-ilvE02-Cas9 strain; when the YPVal-ilvE02-Cas9 strain grew to OD₆₀₀ₙₘ of 0.1, IPTG was added such that the concentration of IPTG in the system was 0.1 mM, and continued to be cultured to induce λ-Red-mediated homologous recombination; and when theYPVal-ilvE02-Cas9 strain grew to OD₆₀₀ₙₘ of 0.6, bacterial cells were collected and competent cells of the YPVal-ilvE02-Cas9 strain were prepared.
(2) The pGRB-mdh sgRNA plasmid obtained in step 1 and the Δmdh-Up-Down fragment obtained in step 2 were transformed into competent cells of the YPVal-ilvE02-Cas9 strain, coated on a 2-YT agar plate containing 100 mg/L Spectinomycin and 100 mg/L Ampicillin, and cultured at 32°C to obtain single colonies.
(3) Taking the single colonies obtained in step (2) as a template respectively, a PCR amplification product was obtained by performing PCR amplification using a primer pair composed of primers P19 and P22. If the PCR amplification product obtained from a single colony contained a DNA fragment with a size of 1253 bp, this colony was preliminarily identified as a positive transformant.
(4) The positive transformant obtained in step (3) was inoculated on a 2-YT agar plate containing 100 mg/L Spectinomycin and 0.2% (m/v) arabinose and cultured at 32°C (for eliminating pGRB-thiE sgRNA plasmids); then, colonies that grew on a 2-YT agar plate containing 100 mg/L Spectinomycin but did not grow on a 2-YT agar plate containing 100 mg/L Ampicillin were picked out, then transferred to a 2-YT agar plate and cultured at 42°C (for eliminating plasmids pREDCas9); and finally, colonies which did not grow on a 2-YT agar plate containing 100 mg/L Spectinomycin but on an antibody-free 2-YT agar plate were picked out.
(5) Taking the single colonies obtained in step (4) as a template respectively, a PCR amplification product was obtained by performing PCR amplification using a primer pair composed of primers P19 and P22. If the PCR amplification product obtained from a single colony contained a DNA fragment with a size of 1253 bp, this colony was identified as a positive transformant in which an mdh gene was deleted from a genome of an engineered bacterium YPVal-ilvE02. This positive transformant was named as the engineered bacterium YPVal-ilvE03.

### Example 8: Modification III of engineered bacteria originating from Escherichia coli W3110

After a large number of experiments, the inventors of the present invention obtained engineered bacteria YPVal-ilvE04, YPVal-ilvE05 and YPVal-ilvE06 by means of modification originating from *Escherichia coli* W3110. Genotypes of the engineered bacteria YPVal-ilvE04, YPVal-ilvE05 and YPVal-ilvE06 were shown in Table 12.

**Table 12**

| Engineered bacteria | Genotypes |
|---|---|
| YPVal-ilvE04 | W3110-ΔilvE(E)-ptrcilvE(B) |
| YPVal-ilvE05 | W3110-ΔilvE(E)-ptrcilvE(B)-ΔthiE |
| YPVal-ilvE06 | W3110-ΔilvE(E)-ptrcilvE(B)-ΔthiE-Δmdh |

### I. Obtainment of engineering bacterium YPVal-ilvE04

### 1. Construction of pGRB-ilvE sgRNA plasmid

The same as 1 in step I in Example 7.

### 2. Obtainment of ptrc-ilvE(B)-Up-Down fragment

The same as 2 in step I in Example 7.

### 3. Obtainment of W3110-Cas9 strain

According to 3 in step I in Example 7, competent cells of a valine-producing bacterium CGMCC 22721 were replaced with competent cells of *Escherichia coli* W3110, and the other steps were unchanged, obtaining the W3110-Cas9 strain.

### 4. Obtainment of engineering bacterium YPVal-ilvE04

According to 4 in step I in Example 7, a CGMCC 22721-Cas9 strain was replaced with a W3110-Cas9 strain, and the other steps were unchanged, obtaining the engineered bacterium YPVal-ilvE04.

### II. Obtainment of engineering bacterium YPVal-ilvE05

### 1. Construction of pGRB-thiE sgRNA plasmid

The same as 1 in step II in Example 7.

### 2. Obtainment of ΔthiE-Up-Down fragment

The same as 2 in step II in Example 7.

### 3. Obtainment of YPVal- ilvE04-Cas9 strain

According to 3 in step II in Example 7, competent cells of an engineered bacterium YPVal-ilvE01 were replaced with competent cells of an engineered bacterium YPVal-ilvE04, and the other steps were unchanged, obtaining the YPVal-ilvE04-Cas9 strain.

### 4. Obtainment of engineering bacterium YPVal-ilvE05

According to 4 in step II in Example 7, a YPVal-ilvE01-Cas9 strain was replaced with a YPVal-ilvE04-Cas9 strain, and the other steps were unchanged, obtaining the engineered bacterium YPVal-ilvE05.

### III. Obtainment of engineering bacterium YPVal-ilvE06

### 1. Construction of pGRB-mdh sgRNA plasmid

The same as 1 in step III in Example 7.

### 2. Obtainment of Δmdh-Up-Down fragment

The same as 2 in step III in Example 7.

### 3. Obtainment of YPVal- ilvE05-Cas9 strain

According to 3 in step III in Example 7, competent cells of an engineered bacterium YPVal-ilvE02 were replaced with competent cells of an engineered bacterium YPVal-ilvE05, and the other steps were unchanged, obtaining the YPVal-ilvE05-Cas9 strain.

### 4. Obtainment of engineering bacterium YPVal-ilvE06

According to 4 in step III in Example 7, a YPVal-ilvE02-Cas9 strain was replaced with a YPVal-ilvE05-Cas9 strain, and the other steps were unchanged, obtaining the engineered bacterium YPVal-ilvE06.

### Example 9: Production of L-valine from engineered bacteria modified in Example 7 and Example 8 by means of fermentation

1. The engineered bacteria YPVal-ilvE01, YPVal-ilvE02, YPVal-ilvE03, YPVal-ilvE04, YPVal-ilvE05 and YPVal-ilvE06 modified in Example 7 and Example 8, as well as a valine-producing bacterium CGMCC 22721 and *Escherichia coli* W3110 as originating bacteria were respectively fermented in a fermentation tank (Shanghai Bailun Biotechnology Co., Ltd., a model of BLBIO-5GC-4-H) to obtain fermentation broth.

### Each strain was repeated three times

The composition of a fermentation medium during fermentation was shown in Table 3.

The fermentation control process was shown in Table 4.

### 2. The yield of L-valine in the fermentation broth was detected by high-performance liquid chromatography

The detection results of the valine-producing bacterium CGMCC 22721 and the engineered bacteria YPVal-ilvE01, YPVal-ilvE02 and YPVal-ilvE03 obtained from the valine-producing bacterium CGMCC 22721 by means of three fermentations were shown in Table 13 (P-value<0.01 indicated a very significant difference). The results showed that compared with the valine-producing bacterium CGMCC 22721, YPVal-ilvE01, YPVal-ilvE02 and YPVal-ilvE03 could significantly increase the yield of L-valine. That is, all engineered bacteria obtained by "inserting ilvE(B) from *Bacillus subtilis* initiated by ptrc while knocking out an ilvE(E) gene", or "inserting ilvE(B) from *Bacillus subtilis* initiated by ptrc while knocking out an ilvE(E) gene, and knocking out a thiE gene" or "inserting ilvE(B) from *Bacillus subtilis* initiated by ptrc while knocking out an ilvE(E) gene, and knocking out a thiE gene and an mdh gene" could increase the yield of L-valine in the valine-producing bacterium CGMCC 22721.

**Table 13**

| Strains | L-valine concentration-1 | L-valine concentration-2 | L-valine concentration-3 | Mean (g/L) | P-value |
|---|---|---|---|---|---|
| | (g/L) | (g/L) | (g/L) | | |
| Valine-producing bacterium CGMCC 22721 | 78.10 | 77.88 | 78.23 | 78.07±0.18 | |
| YPVal-ilvE01 | 80.05 | 80.16 | 80.89 | 80.03±0.14 | P<0.01 |
| YPVal-ilvE02 | 82.11 | 82.08 | 82.20 | 82.13±0.06 | P<0.01 |
| YPVal-ilvE03 | 84.37 | 84.44 | 84.14 | 84.32±0.16 | P<0.01 |

The detection results of *Escherichia coli* W3110 and engineered bacteria YPVal-ilvE04, YPVal-ilvE05 and YPVal-ilvE06 obtained from *Escherichia coli* W3110 by means of three fermentations were shown in Table 14 (P-value<0.01 indicated a very significant difference). The results showed that compared with the *Escherichia coli* W3110, YPVal-ilvE04, YPVal-ilvE05 and YPVal-ilvE06 could significantly increase the yield of L-valine. That is, all engineered bacteria obtained by "inserting in ilvE(B) from *Bacillus subtilis* initiated by ptrc while knocking out an ilvE(E) gene", or "inserting ilvE(B) from *Bacillus subtilis* initiated by ptrc while knocking out an ilvE(E) gene, and knocking out a thiE gene", or "inserting ilvE(B) from *Bacillus subtilis* initiated by ptrc while knocking out an ilvE(E) gene, and knocking out a thiE gene and an mdh gene" could increase the yield of L-valine in the *Escherichia coli* W3110.

**Table 14**

| Strains | L-valine concentration-1 (g/L) | L-valine concentration-2 (g/L) | L-valine concentration-3 (g/L) | Mean (g/L) | P-value |
|---|---|---|---|---|---|
| *Escherichia coli* W3110 | 0.009 | 0.005 | 0.007 | 0.007±0.002 | |
| YPVal-ilvE04 | 0.097 | 0.122 | 0.120 | 0.113±0.014 | P<0.01 |
| YPVal-ilvE05 | 0.258 | 0.224 | 0.230 | 0.237±0.018 | P<0.01 |
| YPVal-ilvE06 | 0.354 | 0.358 | 0.339 | 0.350±0.010 | P<0.01 |

### Example 10: Modification IV of engineered bacteria originating from valine-producing bacterium CGMCC 22721

After a large number of experiments, the inventors of the present invention obtained engineered bacteria YPVal-thiE01, YPVal-thiE02 and YPVal-mdh01 by means of modification originating from a valine-producing bacterium CGMCC 22721. Genotypes of the engineered bacteria YPVal-thiE01, YPVal-thiE02 and YPVal-mdh01 were shown in Table 15.

**Table 15**

| Engineered bacteria | Genotypes |
|---|---|
| YPVal-thiE01 | CGMCC 22721-ΔthiE |
| YPVal-thiE02 | CGMCC 22721-ΔthiE-Δmdh |
| YPVal-mdh01 | CGMCC 22721-Δmdh |

### I. Obtainment of engineering bacterium YPVal-thiE01

According to a genome sequence of *Escherichia coli* W3110 published by NCBI, the engineered bacterium YPVal-thiE01 (hereinafter referred to as YPVal-thiE01) was obtained by knocking out a thiE gene from a genome of the valine-producing bacterium CGMCC 22721 using a CRISPR/Cas9 gene editing technology.

The thiE gene encodes thiamine phosphate synthase with a Gene ID of 948491 and an amino acid sequence as shown in SEQ ID No. 17.

The specific steps were as follows:

### 1. Construction of pGRB-thiE sgRNA plasmid

According to the genome sequence of *Escherichia coli* W3110 published by NCBI, an sgRNA target sequence (SEQ ID No. 9) from which a thiE gene was knocked out was designed by using CRISPR RGEN Tools (http://www.rgenome.net/cas-designer/), and a linearized pGRB vector homologous arm sequence was added to ends 5' and 3' of the target sequence, in order to construct an sgRNA plasmid.
(1) A primer sgRNA-4F: 5'-TGACAGCTAGCTCAGTCCTAGGTATAATACTAGTcgcccctcttatatcgcgctggGTTTTAGAG CTAGAAATAGCAAGTTAAAATAAGG-3' (as shown in SEQ ID No. 43, underlined as a pGRB vector homologous arm sequence), a primer sgRNA-4R: 5'-CCTTATTTTAACTTGCTATTTCTAGCTCTAAAACcccagcgcgatataagaggggcgACTAGTATTA TACCTAGGACTGAGCTAGCTGTCA-3' (as shown in SEQ ID No. 44, underlined as a pGRB vector homologous arm sequence), a primer sgRNA-PF: 5'-GTCTCATGAGCGGATACATATTTG-3' (SEQ ID No. 21) and a primer sgRNA-PR: 5'-ATGAGAAAGCGCCACGCT-3' (SEQ ID No. 22) were synthesized by Invitrogen.
(2) The primer sgRNA-4F and the primer sgRNA-4R were annealed (a reaction procedure: denaturation at 95°C for 5 min, and annealing at 50°C for 1 min); and then, a target fragment was recovered by a DNA purification kit, measured for a DNA concentration, and diluted in concentration to 100 ng/µL to obtain an annealing product. The annealing product contained an sgRNA-4 fragment with a nucleotide sequence as shown in SEQ ID No. 9.
(3) Approximately 2700 bp of DNA fragments were recovered by a DNA recovery kit (QIAGEN Gel Extraction Kit) by digesting a pGRB vector with a restriction enzyme *Spe*I*.*

The digestion system was 50 µL and composed of 5 µL of 10×Buffer (owned by the restriction enzyme *SpeI*), 2.5 µL of restriction enzyme *Spe*I, 3000-5000 ng of pGRB vector, and ddH₂O.

The digestion procedure was carried out at 37°C for 3 h.

(4) The DNA fragments recovered in step (3) were dephosphorylated (to prevent self-adhesion of the pGRB vector) and recovered by a DNA recovery kit to obtain a linearized pGRB vector.

The dephosphorylation system was 50 µL and composed of 5 µL of 10×Buffer (owned by CIAP), 1000-2000 ng of DNA fragments recovered in step (3), 2.5 µL of CIAP, and ddH₂O.

The dephosphorylation procedure was carried out at 37°C for 1 h.

(5) The linearized pGRB vector obtained in step (4) and the annealing product obtained in step (2) were recombined by a Gibson Assembly kit, and then transformed into competent cells of *Escherichia coli* DH5α to obtain a pGRB-thiE sgRNA plasmid.

The recombination system was 5 µL and compsed of 2 µL of linearized pGRB vector, 0.5 µL of annealing product, and 2.5 µL of assembly enzyme (owned by the Gibson Assembly kit).

The recombination procedure was carried out at 50°C for 30 min.

### 2. Obtainment of ΔthiE-Up-Down fragment

(1) According to a genome sequence of *Escherichia coli* W3110 published by NCBI, a primer pair for amplifying an upstream homologous arm and a primer pair for amplifying a downstream homologous arm were designed and synthesized by Invitrogen.

The primer pair for amplifying the upstream homologous arm was composed of a primer P15: 5'-TTCTATTCAGGACGCCAACG-3' (as shown in SEQ ID No. 45) and a primer P16: 5'-GCTATAACGCATAAAGTCACGGCACGCTTCCTCCTTACGCAGG-3' (as shown in SEQ ID No. 46).

A primer pair for amplifying the downstream homologous arm was composed of a primer P17: 5'-CCTGCGTAAGGAGGAAGCGTGCCGTGACTTTATGCGTTATAGC-3' (as shown in SEQ ID No. 47) and a primer P18: 5'-GCCTGCAAAGTGCCCATAACCC-3' (as shown in SEQ ID No. 48).

(2) Taking genomic DNA of *Escherichia coli* W3110 as a template, a primer pair composed of primers P15 and P16 was used for PCR amplification of a high-fidelity amplification enzyme KAPA HiFi HotStart, and an upstream homologous arm with a size of 721 bp was recovered by a DNA recovery kit.

(3) Taking genomic DNA of *Escherichia coli* W3110 as a template, a primer pair composed of primers P17 and P18 was used for PCR amplification of a high-fidelity amplification enzyme KAPA HiFi HotStart, and a downstream homologous arm with a size of 618 bp was recovered by a DNA recovery kit.

(4) Taking the upstream homologous arm recovered in step (2) and the downstream homologous arm recovered in step (3), which were mixed, as a template, a primer pair composed of primers P15 and P18 was used for overlap PCR to obtain a ΔthiE-Up-Down fragment as shown in SEQ ID No. 10.

### 3. Obtainment of CGMCC 22721-Cas9 strain

(1) A plasmid pREDCas9 (Addgene's product, catalog number 71541; containing a Spectinomycin-resistant gene) was transformed into competent cells of a valine-producing bacterium CGMCC 22721, then coated on a 2-YT agar plate containing 100 mg/L Spectinomycin and cultured at 32°C to obtain Spectinomycin-resistant single colonies.

A preparation method for the 2-YT agar plate was as follows: dissolving 16 g of tryptone, 10 g of yeast extract powder, 5 g of sodium chloride and 16 g of agar powder in an appropriate amount of water, then metering a volume with water to 1 L, adjusting a pH value to 7.0 with sodium hydroxide, and sterilizing at 121°C for 20 min.

(2) Taking the single colonies obtained in step (1) as a template respectively, a PCR amplification product was obtained by performing PCR amplification using a primer pair composed of a primer pRedCas9-PF: 5'-GCAGTGGCGGTTTTCATG-3' (SEQ ID No. 27) and a primer pRedCas9-PR: 5'-CCTTGGTGATCTCGCCTTTC-3' (SEQ ID No. 28). If the PCR amplification product obtained from a single colony contained a DNA fragment with a nucleotide sequence as shown in SEQ ID No. 3, this colony contained the plasmid pREDCas9. The strain of this colony was named as the CGMCC 22721-Cas9 strain.

### 4. Obtainment of engineering bacterium YPVal-thiE01

(1) Culture of CGMCC 22721-Cas9 strain; when the strain CGMCC 22721-Cas9 grew to OD₆₀₀ₙₘ of 0.1, IPTG was added such that the concentration of IPTG in the system was 0.1 mM, and continued to be cultured to induce λ-Red-mediated homologous recombination; and when the strain CGMCC 22721-Cas9 grew to OD₆₀₀ₙₘ of 0.6, bacterial cells were collected and competent cells of CGMCC 22721-Cas9 were prepared.
(2) The pGRB-thiE sgRNA plasmid obtained in step 1 and the ΔthiE-Up-Down fragment obtained in step 2 were transformed into competent cells of the CGMCC 22721-Cas9, coated on a 2-YT agar plate containing 100 mg/L Spectinomycin and 100 mg/L Ampicillin, and cultured at 32°C to obtain single colonies.
(3) Taking the single colonies obtained in step (2) as a template respectively, a PCR amplification product was obtained by performing PCR amplification using a primer pair composed of primers P15 and P18. If the PCR amplification product obtained from a single colony contained a DNA fragment with a size of 1296 bp, this colony was preliminarily identified as a positive transformant.
(4) The positive transformant obtained in step (3) was inoculated on a 2-YT agar plate containing 100 mg/L Spectinomycin and 0.2% (m/v) arabinose and cultured at 32°C (for eliminating pGRB-thiE sgRNA plasmids); then, colonies that grew on a 2-YT agar plate containing 100 mg/L Spectinomycin but did not grow on a 2-YT agar plate containing 100 mg/L Ampicillin were picked out, then transferred to a 2-YT agar plate and cultured at 42°C (for eliminating plasmids pREDCas9); and finally, colonies which did not grow on a 2-YT agar plate containing 100 mg/L Spectinomycin but on an antibody-free 2-YT agar plate were picked out.
(5) Taking the single colonies obtained in step (4) as a template respectively, a PCR amplification product was obtained by performing PCR amplification using a primer pair composed of primers P15 and P18. If the PCR amplification product obtained from a single colony contained a DNA fragment with a size of 1296 bp, this colony was identified as a positive transformant in which a thiE gene was deleted from a genome of the valine-producing bacterium CGMCC 22721. This positive transformant was named as the engineered bacterium YPVal-thiE01.

### II. Obtainment of engineering bacterium YPVal-mdh01

According to a genome sequence of *Escherichia coli* W3110 published by NCBI, the engineered bacterium YPVal-mdh01 (hereinafter referred to as YPVal-mdh01) was obtained by knocking out an mdh gene from a genome of the valine-producing bacterium CGMCC 22721 using a CRISPR/Cas9 gene editing technology.

The mdh gene encodes malate dehydrogenase with a Gene ID of 947854 and an amino acid sequence as shown in SEQ ID No. 18.

The specific steps were as follows:

### 1. Construction of pGRB-mdh sgRNA plasmid

According to the genome sequence of *Escherichia coli* W3110 published by NCBI, an sgRNA target sequence (SEQ ID No. 11) from which an mdh gene was knocked out was designed by using CRISPR RGEN Tools (http://www.rgenome.net/cas-designer/), and a linearized pGRB vector homologous arm sequence was added to ends 5' and 3' of the target sequence, in order to construct an sgRNA plasmid.
(1) A primer sgRNA-5F: 5'-TGACAGCTAGCTCAGTCCTAGGTATAATACTAGTgcctttcagttccgcaacaaaggGTTTTAGAG CTAGAAATAGCAAGTTAAAATAAGG-3' (as shown in SEQ ID No. 49, underlined as a pGRB vector homologous arm sequence) and a primer sgRNA-5R: 5'-CCTTATTTTAACTTGCTATTTCTAGCTCTAAAACcctttgttgcggaactgaaaggcACTAGTATTAT ACCTAGGACTGAGCTAGCTGTCA-3' (as shown in SEQ ID No. 50, underlined as a pGRB vector homologous arm sequence) were synthesized by Invitrogen.
(2) The primer sgRNA-5F and the primer sgRNA-5R were annealed (a reaction procedure: denaturation at 95°C for 5 min, and annealing at 50°C for 1 min); and then, a target fragment was recovered by a DNA purification kit, measured for a DNA concentration, and diluted in concentration to 100 ng/µL to obtain an annealing product. The annealing product contained an sgRNA-5 fragment with a nucleotide sequence as shown in SEQ ID No. 11.
(3) Approximately 2700 bp of DNA fragments were recovered by a DNA recovery kit (QIAGEN Gel Extraction Kit) by digesting a pGRB vector with a restriction enzyme *Spe*I*.*

The digestion system was 50 µL and composed of 5 µL of 10×Buffer (owned by the restriction enzyme *Spe*I), 2.5 µL of restriction enzyme *Spe*I, 3000-5000 ng of pGRB vector, and ddH₂O.

The digestion procedure was carried out at 37°C for 3 h.

(4) The DNA fragments recovered in step (3) were dephosphorylated (to prevent self-adhesion of the pGRB vector) and recovered by a DNA recovery kit to obtain a linearized pGRB vector.

The dephosphorylation system was 50 µL and composed of 5 µL of 10×Buffer (owned by CIAP), 1000-2000 ng of DNA fragments recovered in step (3), 2.5 µL of CIAP and ddH₂O.

The dephosphorylation procedure was carried out at 37°C for 1 h.

(5) The linearized pGRB vector obtained in step (4) and the annealing product obtained in step (2) were recombined by a Gibson Assembly kit, and then transformed into competent cells of *Escherichia coli* DH5α to obtain a pGRB-mdh sgRNA plasmid.

The recombination system was 5 µL and composed of 2 µL of linearized pGRB vector, 0.5 µL of annealing product, and 2.5 µL of assembly enzyme (owned by the Gibson Assembly kit).

The recombination procedure was carried out at 50°C for 30 min.

### 2. Obtainment of Δmdh-Up-Down fragment

(1) According to a genome sequence of *Escherichia coli* W3110 published by NCBI, a primer pair for amplifying an upstream homologous arm and a primer pair for amplifying a downstream homologous arm were designed and synthesized by Invitrogen.

The primer pair for amplifying the upstream homologous arm was composed of a primer P19: 5'-AACTTCCTCCAAACCGATGC-3' (as shown in SEQ ID No. 51) and a primer P20: 5'-CAATATAATAAGGAGTTTAGGTTGATTAGCGGATAATAAAAAACC-3' (as shown in SEQ ID No. 52).

A primer pair for amplifying a downstream homologous arm was composed of a primer P21: 5'-GGTTTTTTATTATCCGCTAATCAACCTAAACTCCTTATTATATTG-3' (as shown in SEQ ID No. 53) and a primer P22: 5'-TCTTCAATGGACTGGAGGTG-3' (as shown in SEQ ID No. 54).

(2) Taking genomic DNA of *Escherichia coli* W3110 as a template, a primer pair composed of primers P19 and P20 was used for PCR amplification of a high-fidelity amplification enzyme KAPA HiFi HotStart, and an upstream homologous arm with a size of 590 bp was recovered by a DNA recovery kit.

(3) Taking genomic DNA of *Escherichia coli* W3110 as a template, a primer pair composed of primers P21 and P22 was used for PCR amplification of a high-fidelity amplification enzyme KAPA HiFi HotStart, and a downstream homologous arm with a size of 708 bp was recovered by a DNA recovery kit.

(4) Taking the upstream homologous arm recovered in step (2) and the downstream homologous arm recovered in step (3), which were mixed, as a template, a primer pair composed of primers P19 and P22 was used for overlap PCR to obtain a Δmdh-Up-Down fragment as shown in SEQ ID No. 12.

### 3. Obtainment of CGMCC 22721-Cas9 strain

The same as 3 in step I.

### 4. Obtainment of engineering bacterium YPVal-mdh01

(1) Culture of CGMCC 22721-Cas9 strain; when the strain CGMCC 22721-Cas9 grew to OD₆₀₀ₙₘ of 0.1, IPTG was added such that the concentration of IPTG in the system was 0.1 mM, and continued to be cultured to induce λ-Red-mediated homologous recombination; and when the strain CGMCC 22721-Cas9 grew to OD₆₀₀ₙₘ of 0.6, bacterial cells were collected and competent cells of CGMCC 22721-Cas9 were prepared.
(2) The pGRB-mdh sgRNA plasmid obtained in step 1 and the Δmdh-Up-Down fragment obtained in step 2 were transformed into competent cells of CGMCC 22721-Cas9, coated on a 2-YT agar plate containing 100 mg/L Spectinomycin and 100 mg/L Ampicillin, and cultured at 32°C to obtain single colonies.
(3) Taking the single colonies obtained in step (2) as a template respectively, a PCR amplification product was obtained by performing PCR amplification using a primer pair composed of primers P19 and P22. If the PCR amplification product obtained from a single colony contained a DNA fragment with a size of 1253 bp, this colony was preliminarily identified as a positive transformant.
(4) The positive transformant obtained in step (3) was inoculated on a 2-YT agar plate containing 100 mg/L Spectinomycin and 0.2% (m/v) arabinose and cultured at 32°C (for eliminating pGRB-mdh sgRNA plasmids); then, colonies that grew on a 2-YT agar plate containing 100 mg/L Spectinomycin but did not grow on a 2-YT agar plate containing 100 mg/L Ampicillin were picked out, then transferred to a 2-YT agar plate and cultured at 42°C (for eliminating plasmids pREDCas9); and finally, colonies which did not grow on a 2-YT agar plate containing 100 mg/L Spectinomycin but on an antibody-free 2-YT agar plate were picked out.
(5) Taking the single colonies obtained in step (4) as a template respectively, a PCR amplification product was obtained by performing PCR amplification using a primer pair composed of primers P19 and P22. If the PCR amplification product obtained from a single colony contained a DNA fragment with a size of 1253 bp, this colony was identified as a positive transformant in which an mdh gene was deleted from a genome of the valine-producing bacterium CGMCC 22721. This positive transformant was named as the engineered bacterium YPVal-mdh01.

### III. Obtainment of engineering bacterium YPVal-thiE02

Taking the engineered bacterium YPVal-thiE01 obtained in step I as an originating bacterium, according to a genome sequence of *Escherichia coli* W3110 published by NCBI, an mdh gene was knocked out from a genome of the engineered bacterium YPVal-thiE01 using a CRISPR/Cas9 gene editing technology.

The mdh gene encodes malate dehydrogenase with a Gene ID of 947854 and an amino acid sequence as shown in SEQ ID No. 18.

The specific steps were as follows:

### 1. Construction of pGRB-mdh sgRNA plasmid

The same as 1 in step II.

### 2. Obtainment of Δmdh-Up-Down fragment

The same as 2 in step II.

### 3. Obtainment of YPVal- thiE01-Cas9 strain

(1) A plasmid pREDCas9 was transformed into competent cells of an engineered bacterium YPVal-thiE01, then coated on a 2-YT agar plate containing 100 mg/L Spectinomycin and cultured at 32°C to obtain Spectinomycin-resistant single colonies.
(2) Taking the single colonies obtained in step (1) as a template respectively, a PCR amplification product was obtained by performing PCR amplification using a primer pair composed of primers pRedCas9-PF and pRedCas9-PR. If the PCR amplification product obtained from a single colony contained a DNA fragment with a nucleotide sequence as shown in SEQ ID No. 3, this colony contained the plasmid pREDCas9. The strain of this colony was named as the YPVal-thiE01-Cas9 strain.

### 4. Obtainment of engineering bacterium YPVal-thiE02

(1) Culture of YPVal-thiE01-Cas9 strain; when the YPVal-thiE01-Cas9 strain grew to OD₆₀₀ₙₘ of 0.1, IPTG was added such that the concentration of IPTG in the system was 0.1 mM, and continued to be cultured to induce λ-Red-mediated homologous recombination; and when theYPVal-thiE01-Cas9 strain grew to OD₆₀₀ₙₘ of 0.6, bacterial cells were collected and competent cells of the YPVal-thiE01-Cas9 strain were prepared.
(2) The pGRB-mdh sgRNA plasmid obtained in step 1 and the Δmdh-Up-Down fragment obtained in step 2 were transformed into competent cells of the YPVal-thiE01-Cas9 strain, coated on a 2-YT agar plate containing 100 mg/L Spectinomycin and 100 mg/L Ampicillin, and cultured at 32°C to obtain single colonies.
(3) Taking the single colonies obtained in step (2) as a template respectively, a PCR amplification product was obtained by performing PCR amplification using a primer pair composed of primers P19 and P22. If the PCR amplification product obtained from a single colony contained a DNA fragment with a size of 1253 bp, this colony was preliminarily identified as a positive transformant.
(4) The positive transformant obtained in step (3) was inoculated on a 2-YT agar plate containing 100 mg/L Spectinomycin and 0.2% (m/v) arabinose and cultured at 32°C (for eliminating pGRB-mdh sgRNA plasmids); then, colonies that grew on a 2-YT agar plate containing 100 mg/L Spectinomycin but did not grow on a 2-YT agar plate containing 100 mg/L Ampicillin were picked out, then transferred to a 2-YT agar plate and cultured at 42°C (for eliminating plasmids pREDCas9); and finally, colonies which did not grow on a 2-YT agar plate containing 100 mg/L Spectinomycin but on an antibody-free 2-YT agar plate were picked out.
(5) Taking the single colonies obtained in step (4) as a template respectively, a PCR amplification product was obtained by performing PCR amplification using a primer pair composed of primers P19 and P22. If the PCR amplification product obtained from a single colony contained a DNA fragment with a size of 1253 bp, this colony was identified as a positive transformant in which an mdh gene was deleted from a genome of an engineered bacterium YPVal-thiE01. This positive transformant was named as the engineered bacterium YPVal-thiE02.

### Example 11: Modification IV of engineered bacteria originating from Escherichia coli W3110

After a large number of experiments, the inventors of the present invention obtained engineered bacteria YPVal-thiE03, YPVal-thiE04 and YPVal-mdh02 by means of modification originating from *Escherichia coli* W3110. Genotypes of the engineered bacteria YPVal-thiE03, YPVal-thiE04 and YPVal-mdh02 were shown in Table 16.

**Table 16**

| Engineered bacteria | Genotypes |
|---|---|
| YPVal-thiE03 | W3110-ΔthiE |
| YPVal-thiE04 | W3110-ΔthiE-Δmdh |
| YPVal-mdh02 | W3110-Δmdh |

### I. Obtainment of engineering bacterium YPVal-thiE03

### 1. Construction of pGRB-thiE sgRNA plasmid

The same as 1 in step I in Example 10.

### 2. Obtainment of ΔthiE-Up-Down fragment

The same as 2 in step I in Example 10.

### 3. Obtainment of W3110-Cas9 strain

According to 3 in step I in Example 10, competent cells of a valine-producing bacterium CGMCC 22721 were replaced with competent cells of *Escherichia coli* W3110, and the other steps were unchanged, obtaining the W3110-Cas9 strain.

### 4. Obtainment of engineering bacterium YPVal-thiE03

According to 4 in step I in Example 10, a CGMCC 22721-Cas9 strain was replaced with a W3110-Cas9 strain, and the other steps were unchanged, obtaining the engineered bacterium YPVal-thiE03.

### II. Obtainment of engineering bacterium YPVal-mdh02

### 1. Construction of pGRB-mdh sgRNA plasmid

The same as 1 in step II in Example 10.

### 2. Obtainment of Δmdh-Up-Down fragment

The same as 2 in step II in Example 10.

### 3. Obtainment of W3110-Cas9 strain

According to 3 in step I in Example 10, competent cells of a valine-producing bacterium CGMCC 22721 were replaced with competent cells of *Escherichia coli* W3110, and the other steps were unchanged, obtaining the W3110-Cas9 strain.

### 4. Obtainment of engineering bacterium YPVal-mdh02

According to 4 in step II in Example 10, a CGMCC 22721-Cas9 strain was replaced with a W3110-Cas9 strain, and the other steps were unchanged, obtaining the engineered bacterium YPVal-mdh02.

### III. Obtainment of engineering bacterium YPVal-thiE04

### 1. Construction of pGRB-mdh sgRNA plasmid

The same as 1 in step II in Example 10.

### 2. Obtainment of Δmdh-Up-Down fragment

The same as 2 in step II in Example 10.

### 3. Obtainment of YPVal- thiE03-Cas9 strain

According to 3 in step III in Example 10, competent cells of an engineered bacterium YPVal-thiE01 were replaced with competent cells of an engineered bacterium YPVal-thiE03, and the other steps were unchanged, obtaining the YPVal-thiE03-Cas9 strain.

### 4. Obtainment of engineering bacterium YPVal-thiE04

According to 4 in step III in Example 10, a YPVal-thiE01-Cas9 strain was replaced with a YPVal-thiE03-Cas9 strain, and the other steps were unchanged, obtaining the engineered bacterium YPVal-thiE04.

### Example 12: Production of L-valine from engineered bacteria modified in Example 10 and Example 11 by means of fermentation

1. The engineered bacteria YPVal-thiE01, YPVal-thiE02, YPVal-mdh01, YPVal-thiE03, YPVal-thiE04 and YPVal-mdh02 modified in Example 10 and Example 11, as well as a valine-producing bacterium CGMCC 22721 and *Escherichia coli* W3110 as originating bacteria were respectively fermented in a fermentation tank (Shanghai Bailun Biotechnology Co., Ltd., a model of BLBIO-5GC-4-H) to obtain fermentation broth.

### Each strain was repeated three times

The composition of a fermentation medium during fermentation was shown in Table 3.

The fermentation control process was shown in Table 4.

2. The yield of L-valine in the fermentation broth was detected by high-performance liquid chromatography.

The detection results of the valine-producing bacterium CGMCC 22721 and the engineered bacteria YPVal-thiE01, YPVal-thiE02 and YPVal-mdh01 obtained from the valine-producing bacterium CGMCC 22721 by means of three fermentations were shown in Table 17 (P-value<0.01 indicated a very significant difference). The results showed that compared with the valine-producing bacterium CGMCC 22721, YPVal-thiE01, YPVal-thiE02 and YPVal-mdh01 could significantly increase the yield of L-valine. That is, the yield of L-valine in the valine-producing bacterium CGMCC 22721 could be increased by knocking out a thiE gene or knocking out an mdh gene or knocking out an mdh gene while knocking out a thiE gene.

**Table 17**

| Strains | L-valine concentration-1 (g/L) | L-valine concentration-2 (g/L) | L-valine concentration-3 (g/L) | Mean (g/L) | P-value |
|---|---|---|---|---|---|
| Valine-producing bacterium CGMCC 22721 | 78.10 | 77.88 | 78.23 | 78.07±0.18 | |
| YPVal-thiE01 | 80.13 | 80.26 | 80.29 | 80.23±0.09 | P<0.01 |
| YPVal-thiE02 | 82.57 | 82.33 | 82.43 | 82.44±0.12 | P<0.01 |
| YPVal-mdh01 | 80.11 | 80.32 | 80.37 | 80.27±0.14 | P<0.01 |

The detection results of *Escherichia coli* W3110 and engineered bacteria YPVal-thiE03, YPVal-thiE04 and YPVal-mdh02 obtained from *Escherichia coli* W3110 by means of three fermentations were shown in Table 18 (P-value<0.01 indicated a very significant difference). The results showed that compared with *Escherichia coli* W3110, YPVal-thiE03, YPVal-thiE04 and YPVal-mdh02 could significantly increase the yield of L-valine. That is, the yield of L-valine in the *Escherichia coli* W3110 could be increased by knocking out a thiE gene or knocking out an mdh gene or knocking out an mdh gene while knocking out a thiE gene.

**Table 18**

| Strains | L-valine concentration-1 (g/L) | L-valine concentration-2 (g/L) | L-valine concentration-3 (g/L) | Mean (g/L) | P-value |
|---|---|---|---|---|---|
| *Escherichia coli* W3110 | 0.009 | 0.005 | 0.007 | 0.007±0.002 | |
| YPVal-thiE03 | 0.153 | 0.164 | 0.157 | 0.158±0.006 | P<0.01 |
| YPVal-thiE04 | 0.301 | 0.287 | 0.294 | 0.294±0.007 | P<0.01 |
| YPVal-mdh02 | 0.177 | 0.154 | 0.159 | 0.163±0.012 | P<0.01 |

The present application is detailed above. For a person skilled in the art, the present application may be implemented within a wide range under the same parameters, concentrations and conditions without departing from the purpose and scope of the present application and without unnecessary experiments. Although special examples are given in the present application, it should be understood that further improvements may be made to the present application. In summary, according to the principles of the present application, the present application is intended to include any change, use or improvement of the present application, including changes that deviate from the scope disclosed in the present application and are made by conventional technologies known in the art.

### Industrial Applicability

The present application provides use a pflB gene, an adhE gene, an ilvE(E) gene, an ilvE(B) gene, a thiE gene and an mdh gene in increasing the yield of L-amino in *Escherichia coli.* The experiments showed that "inserting an ilvE(B) gene from *Bacillus subtilis* initiated by ptrc and/or knocking out or weakening one of a pflB gene, an adhE gene, a thiE gene, an mdh gene and an ilvE(E) gene (e.g., "knocking out a pflB gene", "knocking out a pflB gene and an adhE gene", knocking out a pflB gene and an adhE gene and inserting an ilvE(B) gene from *Bacillus subtilis* initiated by ptrc while knocking out an ilvE(E) gene", "knocking out a pflB gene, an adhE gene and a thiE gene and inserting an ilvE(B) gene from *Bacillus subtilis* initiated by ptrc while knocking out an ilvE(E) gene", "knocking out a pflB gene, an adhE gene, a thiE gene and an mdh gene and inserting an ilvE(B) gene from *Bacillus subtilis* initiated by ptrc while knocking out an ilvE(E) gene", "knocking out an adhE gene", "knocking out an adhE gene and inserting an ilvE(B) gene from *Bacillus subtilis* initiated by ptrc while knocking out an ilvE(E) gene", "knocking out an adhE gene and a thiE gene and inserting an ilvE(B) gene from *Bacillus subtilis* initiated by ptrc while knocking out an ilvE(E) gene", "knocking out an adhE gene, a thiE gene and a mdh gene and inserting an ilvE(B) gene from *Bacillus subtilis* initiated by ptrc while knocking out an ilvE(E) gene", "inserting an ilvE(B) gene from *Bacillus subtilis* initiated by ptrc while knocking out an ilvE(E) gene", "knocking out a thiE gene and inserting an ilvE(B) gene from *Bacillus subtilis* initiated by ptrc while knocking out a thiE gene", "knocking out a thiE gene and an mdh gene and inserting an ilvE(B) gene from *Bacillus subtilis* initiated by ptrc while knocking out an ilvE(E) gene", or "knocking out a thiE gene and an mdh gene or knocking out an mid gene while knocking out a thiE gene" was conducive to the accumulation of L-amino acid in *Escherichia coli* that can produce valine. Therefore, the pflB gene, the adhE gene, the ilvE(E) gene, the ilvE(B) gene, the thiE gene and the mdh gene may be used to construct a genetically engineered strain for producing L-amino acid so as to greatly increase the yield of L-amino acid, and reduce the cost, which was of great significance to accelerate the process of L-amino acid industrialization.

## Claims

1. Use of a protein combination, which is at least one of A1)-A5):
A1) the construction of genetically engineered bacteria that produce L-amino acid;
A2) the production of L-amino acid;
A3) the regulation of the yield of L-amino acid;
A4) the preparation of a product for producing L-amino acid; and
A5) the preparation of food, feed or drug containing L-amino acid,
the protein combination comprises at least one of pyruvate formate lyase, alcohol dehydrogenase,
branched-chain amino acid transaminase derived from *Escherichia coli,* branched-chain amino acid transaminase derived from *Bacillus subtilis,* thiamine phosphate synthase and malate dehydrogenase;
the pyruvate formate lyase is B1) or B2) or B3):
B1) a protein comprising an amino acid sequence as shown in SEQ ID No. 13;
B2) a protein which is obtained by substitution and/or deletion and/or addition of amino acid residue(s) in the amino acid sequence as shown in SEQ ID No. 13, has 90% or more identity with the protein shown in B1) and has the same function as the protein shown in B1);
B3) a fusion protein which is obtained by linking a tag to an N-terminal or/and a C-terminal of B1) or B2) and has the same function as that of B1) or B2);
the alcohol dehydrogenase is C1) or C2) or C3):
C1) a protein comprising an amino acid sequence as shown in SEQ ID No. 14;
C2) a protein which is obtained by substitution and/or deletion and/or addition of amino acid residue(s) in the amino acid sequence as shown in SEQ ID No. 14, has 90% or more identity with the protein shown in C1) and has the same function as the protein shown in C1);
C3) a fusion protein which is obtained by linking a tag to an N-terminal or/and a C-terminal of C1) or C2) and has the same function as that of C1) or C2);
the branched-chain amino acid transaminase derived from *Escherichia coli* is D1) or D2) or D3):
D1) a protein comprising an amino acid sequence as shown in SEQ ID No. 15;
D2) a protein which is obtained by substitution and/or deletion and/or addition of amino acid residue(s) in the amino acid sequence as shown in SEQ ID No. 15, has 90% or more identity with the protein shown in D1) and has the same function as the protein shown in D1);
D3) a fusion protein which is obtained by linking a tag to an N-terminal or/and a C-terminal of D1) or D2) and has the same function as that of D1) or D2);
the branched-chain amino acid transaminase derived from *Bacillus subtilis* is E1) or E2) or E3):
E1) a protein comprising an amino acid sequence as shown in SEQ ID No. 16;
E2) a protein which is obtained by substitution and/or deletion and/or addition of amino acid residue(s) in the amino acid sequence as shown in SEQ ID No. 16, has 90% or more identity with the protein shown in E1) and has the same function as the protein shown in E1);
E3) a fusion protein which is obtained by linking a tag to an N-terminal or/and a C-terminal of E1) or E2) and has the same function as that of E1) or E2);
the thiamine phosphate synthase is F1) or F2) or F3):
F1) a protein comprising an amino acid sequence as shown in SEQ ID No. 17;
F2) a protein which is obtained by substitution and/or deletion and/or addition of amino acid residue(s) in the amino acid sequence as shown in SEQ ID No. 17, has 90% or more identity with the protein shown in F1) and has the same function as the protein shown in F1);
F3) a fusion protein which is obtained by linking a tag to an N-terminal or/and a C-terminal of F1) or F2) and has the same function as that of F1) or F2);
the malate dehydrogenase is G1) or G2) or G3):
G1) a protein comprising an amino acid sequence as shown in SEQ ID No. 18;
G2) a protein which is obtained by substitution and/or deletion and/or addition of amino acid residue(s) in the amino acid sequence as shown in SEQ ID No. 18, has 90% or more identity with the protein shown in G1) and has the same function as the protein shown in G1); and
G3) a fusion protein which is obtained by linking a tag to an N-terminal or/and a C-terminal of G1) or G2) and has the same function as that of G1) or G2).

2. Use of a nucleic acid molecule that encodes the protein combination of claim 1, which is at least one A1)-A5):
A1) the construction of genetically engineered bacteria that produce L-amino acid;
A2) the production of L-amino acid;
A3) the regulation of the yield of L-amino acid;
A4) the preparation of a product for producing L-amino acid; and
A5) the preparation of food, feed or drug containing L-amino acid.

3. Use of a substance that inhibits or down-regulates the expression amount and/or activity of the pyruvate formate lyase according to claim 1; and/or a substance that inhibits or down-regulates the expression amount and/or activity of the alcohol dehydrogenase according to claim 1; and/or a substance that inhibits or down-regulates the expression amount and/or activity of the branched-chain amino acid transaminase derived from *Escherichia coli* according to claim 1; and/or a substance that inhibits or down-regulates the expression amount and/or activity of the thiamine phosphate synthase according to claim 1; and/or or a substance that inhibits or down-regulates the expression amount and/or activity of the malate dehydrogenase according to claim 1; and/or a substance that increases or up-regulates the expression amount and/or activity of the branched-chain amino acid transaminase derived from *Bacillus subtilis* according to claim 1, which is at least one of A1)-A5):
A1) the construction of genetically engineered bacteria that produce L-amino acid;
A2) the production of L-amino acid;
A3) the regulation of the yield of L-amino acid;
A4) the preparation of a product for producing L-amino acid; and
A5) the preparation of food, feed or drug containing L-amino acid.

4. Use of an expression cassette, a recombinant vector, a recombinant bacterium or a recombinant host cell, which contains the nucleic acid molecule according to claim 2, the substance that inhibits or down-regulates the expression amount and/or activity of the pyruvate formate lyase according to claim 1, the substance that inhibits or down-regulates the expression amount and/or activity of the alcohol dehydrogenase according to claim 1, the substance that inhibits or down-regulates the expression amount and/or activity of the branched-chain amino acid transaminase derived from *Escherichia coli* according to claim 1, the substance that inhibits or down-regulates the expression amount and/or activity of the thiamine phosphate synthase according to claim 1, the substance that inhibits or down-regulates the expression amount and/or activity of the malate dehydrogenase according to claim 1, and/or the substance that increases or up-regulates the expression amount and/or activity of the branched-chain amino acid transaminase derived from *Bacillus subtilis* according to claim 1, which is at least one of A1)-A5):
A1) the construction of genetically engineered bacteria that produces L-amino acid;
A2) the production of L-amino acid;
A3) the regulation of the yield of L-amino acid;
A4) the preparation of a product for producing L-amino acid; and
A5) the preparation of food, feed or drug containing L-amino acid.

5. A recombinant bacterium, **characterized in that**, the recombinant bacterium is obtained by weak expression or no expression of at least one of the pyruvate formate lyase, the alcohol dehydrogenase, the branched-chain amino acid transaminase derived from *Escherichia coli,* the thiamine phosphate synthase and the malate dehydrogenase of claim 1 in originating bacteria; and/or expression or over-expression of the branched-chain amino acid transaminase derived from *Bacillus subtilis* of claim 1.

6. The recombinant bacterium according to claim 5, **characterized in that**,
the weak expression or no expression is implemented by reducing the expression amount and/or activity of the pyruvate formate lyase, the alcohol dehydrogenase, the branched-chain amino acid transaminase derived from *Escherichia coli,* the thiamine phosphate synthase and/or the malate dehydrogenase in the originating bacteria; and
the expression or over-expression is implemented by introducing a coding gene of the branched-chain amino acid transaminase derived from *Bacillus subtilis* into the originating bacteria.

7. The recombinant bacterium according to claim 6, **characterized in that**,
the reducing the expression amount and/or activity of the pyruvate formate lyase, the alcohol dehydrogenase, the branched-chain amino acid transaminase derived from *Escherichia coli,* the thiamine phosphate synthase and/or the malate dehydrogenase in the originating bacteria are implemented by a gene editing, gene knockout, gene mutation or gene attenuation technology, causing a decrease in the expression amount, activity reduction or inactivation of the pyruvate formate lyase, the alcohol dehydrogenase, the branched-chain amino acid transaminase derived from *Escherichia coli,* the thiamine phosphate synthase and/or the malate dehydrogenase in the originating bacteria.

8. A method for increasing the yield of L-amino acid, comprising the following steps: reducing the expression amount and/or activity of at least one of the pyruvate formate lyase, the alcohol dehydrogenase, the branched-chain amino acid transaminase derived from *Escherichia coli,* the thiamine phosphate synthase and the malate dehydrogenase of claim 1 in originating bacteria; and/or increasing the expression amount and/or activity of the branched-chain amino acid transaminase derived from *Bacillus subtilis* of claim 1 to obtain a recombinant bacterium, the yield of L-amino acid in the recombinant bacterium is higher than that of the originating bacteria.

9. The method according to claim 8, **characterized in that**,
the reducing the expression amount and/or activity of at least one of the pyruvate formate lyase, the alcohol dehydrogenase, the branched-chain amino acid transaminase derived from *Escherichia coli,* the thiamine phosphate synthase and the malate dehydrogenase of claim 1 in the originating bacteria is achieved by a gene editing, gene knockout, gene mutation or gene attenuation technology, causing a decrease in the expression amount, activity reduction or inactivation of the pyruvate formate lyase, the alcohol dehydrogenase, the branched-chain amino acid transaminase derived from *Escherichia coli,* the thiamine phosphate synthase and/or the malate dehydrogenase in the originating bacteria.
the increasing the expression amount and/or activity of the branched-chain amino acid transaminase derived from *Bacillus subtilis* of claim 1 is implemented by introducing a coding gene of the branched-chain amino acid transaminase derived from *Bacillus subtilis* into the originating bacteria.

10. A method for producing L-amino acid, comprising the following steps: fermenting and culturing the recombinant bacterium of any one of claims 5 to 7, and collecting a fermentation product to obtain L-amino acid therefrom.

11. The use according to any one of claims 1 to 4 or the method according to claim 8 or 10, **characterized in that**, the L-amino acid is L-valine.

12. The recombinant bacterium according to any one of claims 5 to 7 or the method according to claim 8 or 9, **characterized in that**, the originating bacterium is *Escherichia coli.*

13. Use of the recombinant bacterium of any one of claims 5 to 7, which is at least one of A2)-A5):
A2) the production of L-amino acid;
A3) the regulation of the yield of L-amino acid;
A4) the preparation of a product for producing L-amino acid; and
A5) the preparation of food, feed or drug containing L-amino acid.
